(19) Europäisches Patentamt
European Patent Office
Office européen des brevets

(11) Numéro de publication : **0 007 097**
**B1**

(12) **FASCICULE DE BREVET EUROPÉEN**

(45) Date de publication du fascicule du brevet : **28.04.82**

(51) Int. Cl.³ : **C 07 D323/00**, A 61 K 7/06, C 11 D 1/00// C07C153/07

(21) Numéro de dépôt : **79102399.7**

(22) Date de dépôt : **12.07.79**

(54) **Nouveaux composés polypodes tensio-actifs, procédé pour les préparer et compositions les contenant.**

(30) Priorité : **13.07.78 FR 7821081**

(43) Date de publication de la demande : **23.01.80 (Bulletin 80/02)**

(45) Mention de la délivrance du brevet : **28.04.82 Bulletin 82/17**

(84) Etats contractants désignés : **AT BE CH DE FR GB IT LU NL SE**

(56) Documents cités : **Néant**

(73) Titulaire : **L'OREAL Société anonyme dite:**
**14, Rue Royale**
**F-75008 Paris (FR)**

(72) Inventeur : **Vanlerberghe, Guy**
**rue du Général de Gaulle**
**Villevaudé, F-77410 Claye-Souilly (FR)**
Inventeur : **Sebag, Henri**
**26 rue Erlanger**
**F-75016 Paris (FR)**

(74) Mandataire : **Casalonga, Alain et al**
**BUREAU D.A. CASALONGA OFFICE JOSSE & PETIT**
**Baaderstrasse 12-14**
**D-8000 München 5 (DE)**

**EP 0 007 097 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

Nouveaux composés polypodes tensio-actifs, procédé pour les préparer et compositions les contenant

L'invention a pour objet de nouveaux polyéthers cycliques tensio-actifs, appelés « Polypodes », les procédés pour les préparer et compositions les contenant.

Les nouveaux polyéthers cycliques tensio-actifs présentent des propriétés remarquables qui les différencient des composés analogues décrits jusqu'à présent. Par leur structure chimique ils sont un peu apparentés aux éthers-couronnes (Crown ethers) qui ont été très étudiés au cours des dernières années comme complexants des cations de métaux alcalins ou alcalino-terreux.

Ils s'en différencient par le caractère amphiphile c'est-à-dire une affinité à la fois vis-à-vis de l'eau et des milieux organiques, ce qui leur confère une forte activité interfaciale.

Ils se distinguent également des agents de surface conventionnels qui comportent une seule chaîne lipophile par molécule.

Comme cela est bien connu, ces derniers, lorsqu'ils sont dissous dans l'eau, manifestent au-delà d'un seuil de concentration appelé concentration micellaire critique, un ensemble de propriétés très avantageuses en vue d'un grand nombre d'applications. En particulier, à des concentrations au moins égales à ce seuil, ils solubilisent dans l'eau des substances organiques telles que des colorants liposolubles et des hydrocarbures.

Les composés selon l'invention possèdent des propriétés solubilisantes à des concentrations très faibles, souvent bien inférieures à la concentration micellaire critique des agents de surface qui comportent une chaîne lipophile de longueur comparable.

Ceci constitue un avantage important pour certaines utilisations de tensio-actifs telles que par exemple dans des compositions pharmaceutiques ou cosmétiques où il y a intérêt à réduire le plus possible la quantité de composé tensio-actif utilisé afin de ne pas interférer avec le principe actif de ces compositions.

En outre, les composés de l'invention sont moins agressifs vis-à-vis de la peau et des muqueuses, en particulier les muqueuses oculaires, ou moins « dénaturants » vis-à-vis des protéines, que les agents de surface comportant une seule chaîne lipophile par molécule et des groupements fonctionnels comparables.

Les nouveaux polyéthers cycliques tensio-actifs dits « Polypodes » peuvent être représentés par la formule générale :

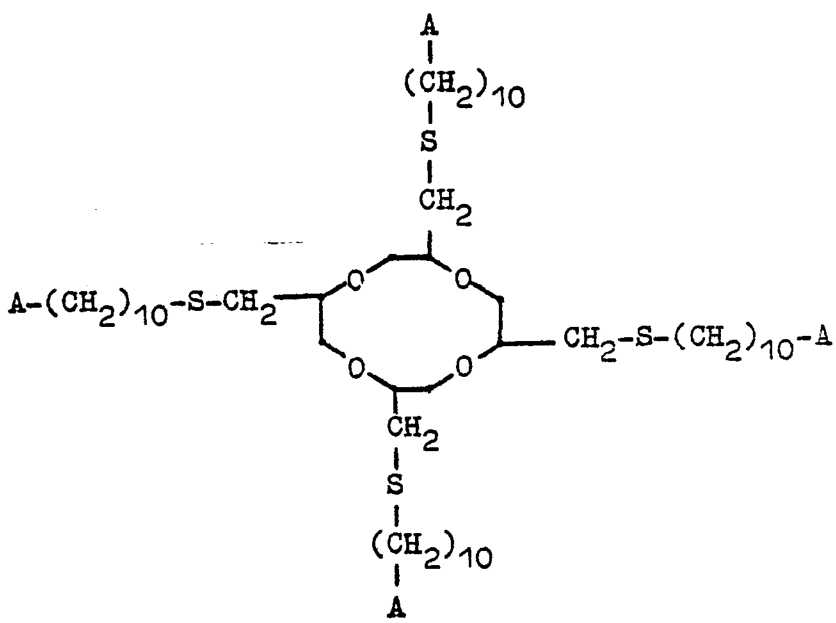

(I)

Cette formule générale peut également s'écrire sous la forme simplifiée :

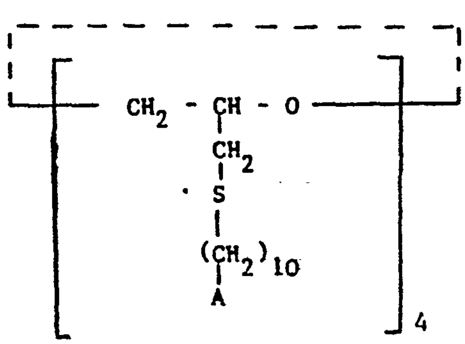

(Ia)

Dans les formules (I) et (Ia), A désigne un ensemble hydrophile qui est relié au cycle par le radical thia-11 dodécaméthylène qui constitue l'ensemble lipophile.

La configuration moléculaire des composés de l'invention rappelle la forme de certains invertébrés possédant plusieurs tentacules d'où le nom de « polypodes » utilisé ici pour les désigner et qui a été proposé pour des composés analogues par R. Fornasier & F. Montanarí « Tetrahydronc Letters » n° 17, p. 1381-1384 (1976).

L'ensemble hydrophile A peut être cationique, zwitterionique, anionique ou non ionique. Il comporte au moins un groupement choisi parmi les groupements amine, ammonium, ammonio alcoyl carboxylate, ammonio alcoyl sulfonate et/ou parmi les groupements spécifiques (g), (h), (i), (j) ci-après définis.

Comme exemples de groupements amine, ammonium, ammonio alcoyl carboxylate et ammonio alcoyl sulfonate on peut citer les groupements suivants :

— le groupement :

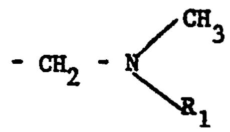

(a)

où $R_1$ désigne $CH_3$ ou $—[CH_2—CH_2—O]_{\overline{n}}—H$

*n* désigne un nombre quelconque de 1 à 10 ;

— le groupement :

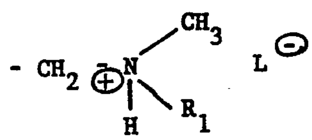

(b)

où $L^{\ominus}$ désigne l'anion d'un acide organique ou minéral et de préférence un anion chlorure, bromure, sulfate, phosphate, acétate, glycolate, lactate, tartrate, méthane sulfonate, paratoluène sulfonate ;

$R_1$ a la signification indiquée pour (a) ;

— le groupement :

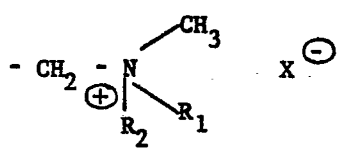

(c)

où $R_2$ désigne un radical alkyle ou hydroxyalkyle ayant de 1 à 3 atomes de carbone et de préférence un radical méthyle, hydroxyéthyle ou dihydroxypropyle ;

$X^{\ominus}$ désigne un anion et de préférence un anion chlorure, bromure, iodure, méthyle sulfate, mésylate ou tosylate ;

$R_1$ a la signification indiquée pour (a) ;

— le groupement :

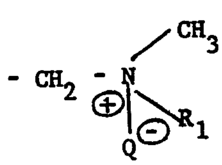

(d)

où $Q^{\ominus}$ désigne l'un des groupements :

$$—CH_2COO^{\ominus}$$

$$—CH_2—CH_2COO^{\ominus}$$

$$—CH_2—CH_2—CH_2—SO_3^{\ominus} ;$$

$R_1$ a la signification indiquée pour (a) ;

— le groupement :

$$-(CH_2-S-CH_2)_w-CONH-(CH_2)_m-B \qquad (e)$$

où *m* désigne le nombre 2 ou 3 ;
*w* désigne le nombre 0 ou 1 ;
B désigne l'un des groupements :

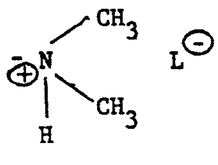 , 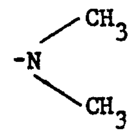 , 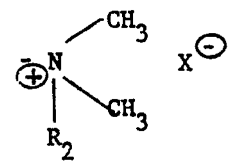 , 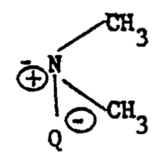 ;

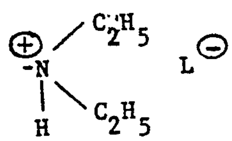 , 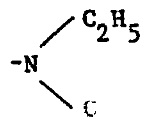 , 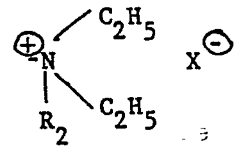 ; 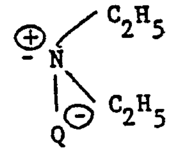

$Q^{\ominus}$ a la signification indiquée pour (d),
$R_2$ et $X^{\ominus}$ ont la signification indiquée po... (c),
$L^{\ominus}$ a la signification indiquée pour (b),
— le groupement :

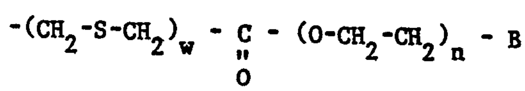 (f)

*n* ayant la signification indiquée pour (a), *w* désigne le nombre 0 ou 1,
B ayant les significations indiquées pour (e) ;
— le groupement :

$$-[CH_2-S-CH_2]_w-COOM_1 \qquad (g')$$

où $M_1$ désigne un groupement ammonium ;
*w* désigne ... nombre 0 ou 1 ;
— le grou...ment :

$$-CH_2-O-SO_3M \qquad (h')$$

où M désigne un groupement ammonium ;
— le groupement :

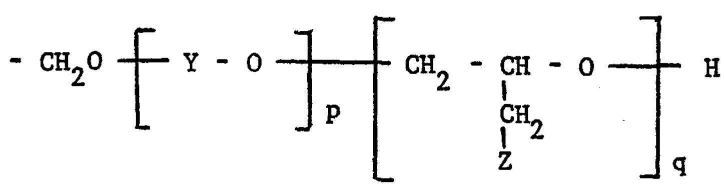 (j')

où p et q, identiques ou différents, désignent un nombre quelconque de 0 à 10, cependant p et q ne désignent pas simultanément zéro,

Y désigne le radical éthylène ou hydroxypropylène,
Z désigne l'un des groupements suivants :

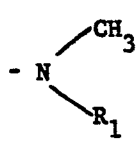 ; 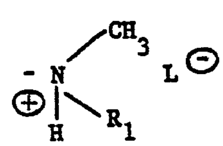 ; 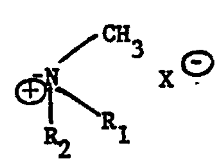 ; 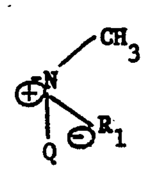

$-S-CH_2-COOM$ ; $-SO_3M$ ;
où $R_1$ a la signification indiquée pour (a) ;
$L^{\ominus}$ a la signification indiquée pour (b) ;
$R_2$ et $X^{\ominus}$ ont la signification indiquée pour (c) ;
$Q^{\ominus}$ a la signification indiquée pour (d) ;
M désigne un groupement ammonium ;

de plus, quand p désigne zéro ou lorsque Y désigne le groupement éthylène, Z en outre désigne l'un des groupements :

$$-OSO_3M \quad ou \quad -OCO-CH_2-SO_3M ;$$

où M désigne un groupement ammonium ;

Les groupements spécifiques (g), (h), (i) et (j) sont les suivants :

— le groupement :

$$-[CH_2-S-CH_2]_w-COOM_1 \qquad (g)$$

où *w* désigne le nombre 0 ou 1 ;
$M_1$ désigne un métal alcalin ;

— le groupement :

$$-CH_2-O-SO_3M \qquad (h)$$

où M désigne un atome d'hydrogène ou un métal alcalin ;

— le groupement :

$$-CH_2-S-[Y-O]_n-H \qquad (i)$$

où *n* désigne un nombre quelconque de 1 à 10,
Y désigne le radical éthylène ou hydroxypropylène ;

— le groupement :

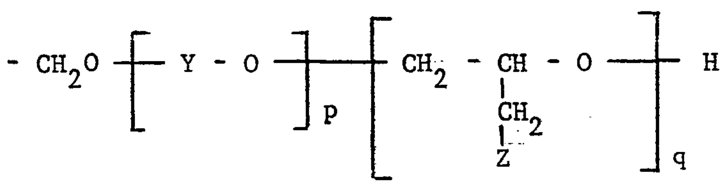 (j)

où p et q, identiques ou différents, désignent un nombre quelconque de 0 à 10, cependant p et q ne désignent pas simultanément zéro,

Y a la signification indiquée sous (i),
Z désigne l'un des groupements suivants :

$-OH$ ; $-S-CH_2-CH_2OH$ ; $-S-CH_2-CHOH-CH_2OH$ ; $-S-CH_2-COOM$ ; $-SO_3M$ ;

de plus, quand p désigne zéro ou quand Y désigne éthylène, Z désigne également $-C-SO_3-M$ ou $-OCO-CH_2-SO_3M$ ;

où M désigne un atome d'hydrogène ou un métal alcalin.

La répartition des motifs :

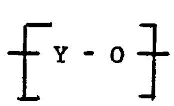 et 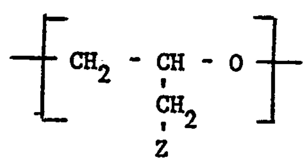

est le plus généralement une répartition en blocs comme indiqué par les formules (j) et (j').

Cependant quand Y désigne le groupement hydroxypropylène, la répartition peut également être statistique.

Les composés de formule (I) peuvent être préparés à partir de l'alcool undécylénique ou de l'acide undécylénique.

Par réaction de ces composés avec l'acide thioacétique suivie de saponification avec une base alcaline et de préférence avec l'hydroxyde de sodium ou de potassium, on obtient respectivement :

le mercapto-11 undécanol-1 de formule

$$HS{-}(CH_2)_{10}{-}CH_2OH \qquad (II)$$

ou l'acide mercapto-11-undécanoïque-1 de formule

$$HS{-}(CH_2)_{10}{-}COOH \qquad (III)$$

La préparation de ces composés intermédiaires de formules (II) et (III) est décrite plus en détail dans les exemples 1 et 2.

Par réaction du mercapto-11 undécanol-1 avec le tétramère de l'épichlorhydrine ou de l'épibromhydrine de formule (IV)

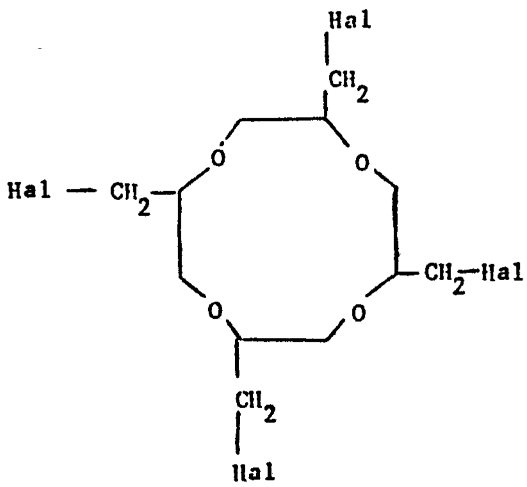

(IV)

qui sous la forme simplifiée s'écrit :

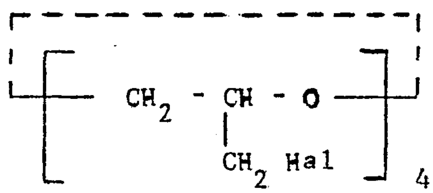

(IVa)

où Hal désigne le chlore ou le brome,

on obtient le composé intermédiaire de formule (V)

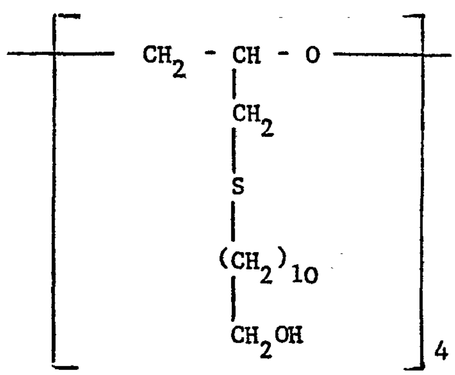

(V)

dont la préparation est décrite plus en détail dans l'exemple 4 ci-après.

Par réaction, soit de l'acide mercapto-11 undécanoïque-1, ou de son ester méthylique ou éthylique avec le tétramère de l'épichlorhydrine ou de l'épibromhydrine de formule (IV), soit du thioglycolate de méthyle ou d'éthyle avec le dérivé halogéné, le mésylate ou tosylate du composé de formule (V), on obtient le composé intermédiaire (IV) :

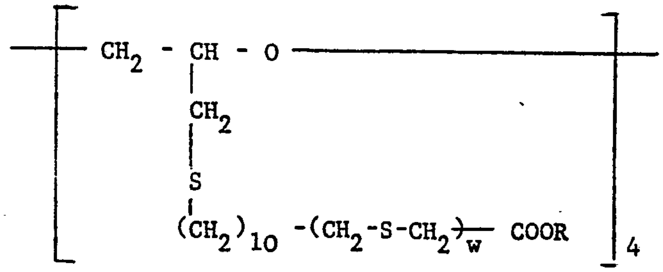

(VI)

où R désigne hydrogène, $CH_3$ ou $C_2H_5$, *w* désigne le nombre 0 ou 1.

L'invention a également pour objet les composés intermédiaires de formule (VII) :

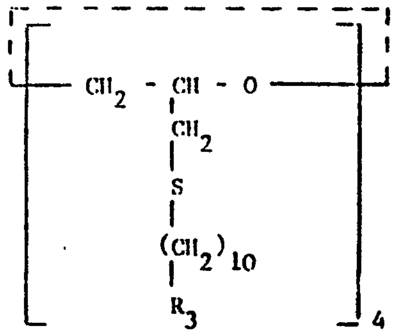

(VII)

où $R_3$ désigne $-CH_2OH$ ; $-CH_2-O-SO_2-CH_3$ ; $-CH_2-O-SO_2-C_6H_4-CH_3$ ; $-COOH$ ; $-COOCH_3$ ; $COOC_2H_5$ ; $-CH_2-S-CH_2-COOH$ ; $-CH_2-S-CH_2-COOCH_3$ ou $-CH_2-S-CH_2-COOC_2H_5$.

Le tétramère de l'épichlorhydrine ou de l'épibromhydrine de formule (IV) est préparé d'une manière connue par polymérisation de l'épichlorhydrine ou de l'épibromhydrine en présence d'un catalyseur acide de Lewis, tel que $BF_3$, $SnCl_4$, $SbCl_5$, ou leur mélange et purifié par fractionnement sous pression réduite. Ce procédé est décrit dans l'exemple 3.

Les composés du type I(a), répondant à la formule I dans laquelle A désigne le groupement (a), sont préparés par réaction de la diméthylamine ou de la méthyléthanolamine,

— soit avec le dérivé chloré ou bromé du composé intermédiaire de formule (V) (obtenu par réaction du composé de formule (V) avec respectivement le chlorure de thionyle ou l'acide bromhydrique),

— soit avec le mésylate ou tosylate de ce composé de formule (V).

La condensation de l'amine peut être réalisée à la pression atmosphérique ou en autoclave, à une température comprise entre 20 et 160 °C. Lorsqu'on utilise la méthyléthanolamine, le composé obtenu peut éventuellement être amené à réagir avec l'oxyde d'éthylène.

Les composés du type I(b) répondant à la formule (I) dans laquelle A désigne le groupement (b), dérivent des composés I(a) par salicification avec un acide minéral ou organique, et avantageusement avec un des acides suivants : chlorhydrique, bromhydrique, sulfurique, phosphorique, acétique, glycolique, lactique, tartrique, méthane sulfonique, paratoluène sulfonique.

Les composés du type I(c), répondant à la formule (I) dans laquelle A désigne le groupement (c), peuvent être préparés par alcoylation des composés I(a) avec un agent alcoylant tel que le chlorure, bromure, iodure, mésylate ou tosylate de méthyle, le sulfate de diméthyle, la chlorhydrine du glycol ou la chlorhydrine du glycérol.

Les composés du type I(c) dans lesquels $X^{\ominus}$ désigne un anion mésylate (méthane sulfonate) ou tosylate (p-toluène sulfonate) peuvent également être obtenus par réaction des mésylate ou tosylate du composé intermédiaire de formule (V) avec une amine de formule :

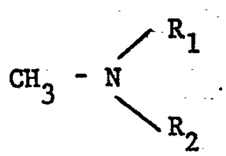

où $R_1$ désigne $CH_3$ ou $-[CH_2-CH_2-O]_n-H$,

*n* désigne un nombre quelconque de 1 à 10,

$R_2$ désigne un radical méthyle, hydroxyéthyle ou dihydroxypropyle, à une température de 20 à 100 °C.

Les composés du type I(d), répondant à la formule (I) dans laquelle A désigne le groupement (d),

peuvent être préparés par alcoylation des composés I(a) avec le chloracétate ou chloropropionate de sodium ou la propane sultone.

Les composés du type I(e), répondant à la formule (I) dans laquelle A désigne le groupement (e), peuvent être préparés par condensation du composé intermédiaire de formule (VI) avec la diméthylamino éthylamine, la diméthylamino propylamine, la diéthylamino éthylamine ou la diéthylamino propylamine, à une température de 20 à 60 °C.

Les groupements amine des amino amides ainsi obtenus peuvent être ensuite salifiés avec un acide minéral ou organique comme indiqué pour les composés du type I(b). Ils peuvent également être alcoylés avec des agents alcoylants comme ceux indiqués pour les composés du type I(c) et I(d), à une température de 10 à 100 °C.

Les composés du type I(f) répondant à la formule (I) dans laquelle A désigne le groupement (f) sont obtenus par condensation du composé intermédiaire de formule (VI) avec une diméthylamine oxyéthylénée de formule :

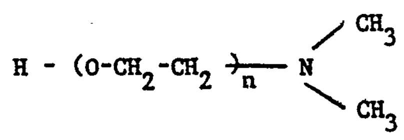

où *n* désigne un nombre quelconque de 1 à 10 ; la condensation s'effectuant à une température de 20 à 160 °C, les groupements amine peuvent ensuite être salifiés avec un acide minéral ou organique ou alcoylés avec un agent alcoylant.

Les composés du type I(g) et I(g'), répondant à la formule (I) dans laquelle A désigne le groupement (g) ou (g') sont obtenus par saponification de l'ester méthylique ou éthylique du composé intermédiaire de formule (VI), suivie de neutralisation avec un hydroxyde alcalin ou l'ammoniaque ou bien, lorsque *w* désigne zéro, directement par réaction du tétramère d'épichlorhydrine ou d'épibromhydrine avec l'acide mercapto-11 undécanoïque-1 en présence de méthylate ou d'éthylate de sodium ou de potassium, à une température de 60 à 130 °C suivie de neutralisation avec un hydroxyde alcalin ou l'ammoniaque.

Les composés du type I(h) et I(h'), répondant à la formule (I) dans laquelle A désigne le groupement (h) ou (h') sont obtenus par sulfatation du composé intermédiaire de formule (V) avec la chlorhydrine sulfurique, à une température de 0 à 80 °C et neutralisation éventuelle avec un hydroxyde alcalin ou l'ammoniaque.

Ces réactions peuvent être réalisées sans solvant ou en présence d'un solvant comme le chloroforme, le benzène, le toluène, l'éther, les éthers de glycol.

Les composés du type I(i), répondant à la formule (I) dans laquelle A désigne le groupement (i), sont obtenus par réaction du mercaptoéthanol ou du mercaptoglycérol avec le mésylate ou tosylate du composé intermédiaire de formule (V) ou avec un composé halogéné dérivé de celui-ci. Ces réactions sont généralement réalisées dans un solvant (de préférence éthanol, propanol, isopropanol, t-butanol, butanol-1, les glycols ou monoéthers de glycol, avec éventuellement de l'eau) en présence de méthylate ou éthylate de sodium ou de potassium, ou d'hydroxyde de sodium ou de potassium, à des températures comprises entre 60 et 160 °C.

Les dérivés ainsi obtenus peuvent ensuite initier des réactions de polyaddition d'oxyde d'éthylène et/ou de glycidol à 120-180 °C en présence d'un catalyseur alcalin, avantageusement en présence de méthylate ou d'éthylate de sodium ou de potassium. Dans le cas d'addition à la fois d'oxyde d'éthylène et de glycidol, ces additions sont réalisées généralement en deux étapes successives, l'ordre des additions étant indifférent. Ces additions peuvent être réalisées avec ou sans solvant. Les solvants préférés sont : l'eau, l'isopropanol, le tertiobutanol, la méthyl éthyl cétone, la méthyl isobutyl cétone.

Les composés du type I(j) et I(j'), répondant à la formule (I) dans laquelle A désigne le groupement (j) ou (j') sont obtenus, en un ou plusieurs stades, par des réactions de polyaddition de réactifs à fonction époxyde avec le composé intermédiaire de formule (V).

Les époxydes utilisables sont l'oxyde d'éthylène, le tertio-butyl glycidyléther (TBGE), une épihalohydrine comme l'épichlorhydrine ou l'épibromhydrine et leurs mélanges.

Les réactions de polyaddition sont généralement des opérations séquentielles mais dans le cas où l'on utilise le TBGE et une épihalohydrine on peut également additionner les deux réactifs simultanément ou en mélange.

Dans le cas d'addition de TBGE au composé intermédiaire de formule (V), les groupements protecteurs tertio-butoxy peuvent être remplacés par des groupements hydroxyle par chauffage à 50-120 °C en présence d'un acide sulfocarboxylique ou sulfonique. Cette réaction est décrite plus en détail dans le brevet français n° 2 027 585 ou les brevets 3 840 606 et 3 959 390 de la demanderesse.

Les atomes d'halogène des oligomères obtenus par réaction avec une épihalohydrine peuvent être remplacés par un groupement hydroxyle, thiohydroxyéthyle, thiodihydroxypropyle, thioglycolate, amine, ammonium, ammonio acétate, ammonio propionate, ammonio propane sulfonate ou sulfonate.

Le remplacement des atomes d'halogène par des groupements hydroxyle s'effectue par réaction avec un sel alcalin d'acide carboxylique et de préférence avec l'acétate de sodium ou de potassium à une température de 150 à 200 °C dans un solvant approprié choisi avantageusement parmi les glycols et les dérivés de glycol ; l'ester acétique formé est ensuite coupé par saponification au moyen d'hydroxyde de sodium ou de potassium ou par alcoolyse au moyen d'un alcool inférieur, et de préférence au moyen de méthanol ou d'éthanol en présence d'un catalyseur basique choisi de préférence parmi le méthylate ou l'éthylate de sodium ou de potassium.

Le remplacement des atomes d'halogène par des groupements thiohydroxyéthyle, thiodihydroxypropyle ou thioglycolate s'effectue par réaction avec le thioéthanol, le thioglycérol ou le thioglycolate de méthyle ou d'éthyle ; à une température de 20-150 °C, en présence éventuellement d'un solvant et d'un composé alcalin choisi avantageusement parmi les hydroxyde, méthylate et éthylate de sodium ou de potassium.

Le remplacement des atomes d'halogène par des groupements amine s'effectue comme pour les composés du type I(a).

Les fonctions amine ainsi obtenues peuvent être salifiées ou alcoylées comme indiqué pour les composés I(b), I(c) et I(d) et transformés en fonction ammonium, ammonio acétate, ammonio propionate ou ammonio propane sulfonate.

Les groupements hydroxyles obtenus peuvent ensuite éventuellement être estérifiés et remplacés par des groupements sulfate ou sulfoacétate respectivement par réaction avec la chlorhydrine sulfurique ou l'acide sulfoacétique.

Dans ce dernier cas, la réaction peut être réalisée directement avec les groupements t-butoxy.

Les polyéthers cycliques tensio-actifs de formule (I) dits «polypodes» selon l'invention, se présentent sous la forme d'huiles, de pâtes, de cires ou de poudres généralement solubles ou dispersibles dans l'eau.

Ils abaissent la tension superficielle de l'eau et permettent à de très faibles concentrations, la solubilisation de produits non hydrosolubles.

Parmi les composés non hydrosolubles pouvant être solubilisés par les polyéthers cycliques tensio-actifs de formule (I) il faut citer les colorants, les parfums, certains produits pharmaceutiques.

Outre la solubilisation de ces produits, les composés tensio-actifs cycliques de formule (I) peuvent permettre de solubiliser ou de disperser des composés minéraux ou polaires en milieu organique ou des composés hydrophobes en milieu aqueux.

Les composés de l'invention peuvent être utilisés dans l'industrie, notamment dans des compositions cosmétiques, pharmaceutiques, dans les industries du textile, des teintures, des insecticides et des industries similaires.

L'invention a également pour objet les compositions renfermant au moins un polyéther cyclique tensio-actif de formule (I).

Parmi ces compositions, il faut citer plus particulièrement les compositions cosmétiques et pharmaceutiques renfermant au moins $0,5.10^{-2}$ gramme par litre ou $0,5.10^{-3}$ % en poids de polyéther cyclique tensio-actif de formule (I).

Les compositions cosmétiques englobent notamment les compositions destinées aux soins de la peau, des ongles, des cheveux.

Les compositions pour les soins des cheveux visent notamment les shampooings et les compositions de conditionnement des cheveux, ainsi que les compositions tinctoriales et les shampooings colorants.

Les compositions cosmétiques peuvent se présenter sous la forme d'une solution aqueuse ou hydroalcoolique, ou sous la forme d'une crème, d'un gel, d'une émulsion ou d'un aérosol.

Les solutions hydroalcooliques renferment généralement un alcool ou un polyol ayant de 1 à 4 atomes de carbone et de préférence l'éthanol ou l'isopropanol, avantageusement en une proportion de 5 à 70 % du poids total de la composition.

Les compositions cosmétiques pour le traitement des cheveux et en particulier les shampooings, les shampooings colorants et les compositions tinctoriales peuvent également contenir en plus d'un polypode tensio-actif de formule (I), également un adjuvant choisi dans le groupe formé par les tensio-actifs anioniques, cationiques, amphotères, zwitterioniques ou non ioniques et leurs mélanges, les parfums, les colorants, les conservateurs.

Les stabilisateurs de mousse, les agents adoucissants, les agents de restructuration des cheveux, les agents antipelliculaires, les résines cosmétiques, les séquestrants, les épaississants, les synergistes de mousse, les électrolytes.

Les shampooings colorants et les compositions tinctoriales peuvent en outre contenir un ou plusieurs colorants directs et en particulier des colorants anthraquinoniques, des colorants azoïques, des colorants nitrés de la série benzénique, des indophénols, des indoanilines, des indamines.

Le pH des compositions est généralement compris entre 3 et 10.

L'invention a également pour objet un procédé de traitement des cheveux consistant à appliquer sur les cheveux une quantité efficace d'une composition aqueuse ou hydroalcoolique renfermant un ou plusieurs polypodes tensio-actifs de formule (I) et éventuellement un ou plusieurs adjuvants ci-dessus définis.

L'invention sera mieux comprise à l'aide des exemples non limitatifs ci-après.

## Exemple 1

Préparation du mercapto-11 *undécanol-1 (composé intermédiaire de formule* (II))

A 256 g (1,5 mole) d'alcool undécylénique on ajoute 3,7 g d'azodiisobutyronitrile, puis en l'espace d'une heure, 105,5 g (1,5 mole) d'acide thioacétique, en maintenant la température entre 25 et 50 °C. On chauffe ensuite à 70 °C pendant 4 heures, on obtient le composé :

$$CH_3—CO—S—(CH_2)_{10}—CH_2OH$$

Le taux de réaction déterminé par dosage du groupement SH est de 95 %.

On reprend la masse réactionnelle avec 200 ml d'éthanol à 96° et 40 ml d'eau. On saponifie en ajoutant 165 g de solution aqueuse d'hydroxyde de sodium à 10 meq/g (milliéquivalent par gramme) et en chauffant pendant 1 heure à la température de reflux. On acidifie avec de l'acide chlorhydrique et on sépare par décantation la phase organique que l'on lave ensuite trois fois avec 300 ml d'eau à 80-90 °C.

Après déshydratation, le mercapto-11 undécanol-1 distille à 126-130 °C sous une pression réduite de 0,4 mm de Hg.

Le produit obtenu se présente sous la forme d'un solide blanc ayant un indice SH de 4,8 meq/g.

## Exemple 2

Préparation de l'acide mercapto-11 undécanoïque-1 (composé intermédiaire de formule (III))

A 184 g (1 mole) d'acide undécylénique, on ajoute 2,5 g d'azo-diisobutyronitrile, puis en 40 minutes 76 g (1 mole) d'acide thioacétique. La réaction est exothermique et la température s'élève de 24 °C à 65 °C. On chauffe ensuite à 70 °C pendant 3 heures. On obtient le composé de formule :

$$CH_3—CO—S(CH_2)_{10}—COOH$$

Le taux de réaction d'après le dosage du groupement —SH est de 98-99 %.

On dilue la masse réactionnelle avec 100 g d'éthanol à 96° puis on ajoute en 30 minutes 220 g (2,2 moles) de solution aqueuse d'hydroxyde de sodium à 10 meq/g. Après avoir chauffé le mélange 1 heure à 70 °C, on acidifie avec 190 ml d'acide chlorhydrique concentré.

L'acide mercapto-11 undécanoïque-1 est décanté puis lavé quatre fois avec 100 ml d'eau à 65-70 °C. Il est ensuite séché par chauffage sous pression réduite puis distille à 145 °C sous une pression de 0,07 mm de Hg.

Indice d'acide = 4,38 meq/g

Indice SH = 4,38 meq/g

## Exemple 3

Préparation du tétramère de l'épichlorhydrine (composé intermédiaire de formule (IV)) par polymérisation de l'épichlorhydrine.

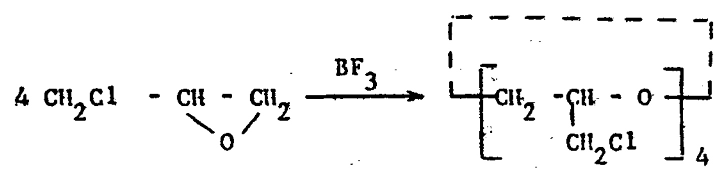


Dans un réacteur de 6 l on mélange 1 180 g d'épichlorhydrine et 500 ml de tétrachlorure de carbone. On refroidit le mélange à 10 °C par immersion dans un bain de glace. Sous vive agitation, on ajoute, en 6 heures, 16 g d'éthérate de trifluorure de bore en solution dans 1 litre de tétrachlorure de carbone. On maintient pendant ce temps la température entre 10 et 13 °C.

On laisse ensuite monter la température et le mélange est porté, progressivement à la température de reflux pendant 1 heure. On ajoute 50 g de carbonate de sodium sec, finement pulvérisé et le mélange est agité 2 heures au reflux. Les sels minéraux sont ensuite séparés par filtration. Le tétrachlorure de carbone est évaporé sous pression réduite, puis on fractionne par distillation.

On recueille ainsi, à 185 °C et sous une pression de 0,2 mm de Hg, 210 g de produit qui se présente sous la forme d'une masse vitreuse incolore cristallisant au bout de quelques jours.

Dosage de chlore organique : 10,6 meq/g.

## Exemple 4

Préparation du composé intermédiaire de formule (V).

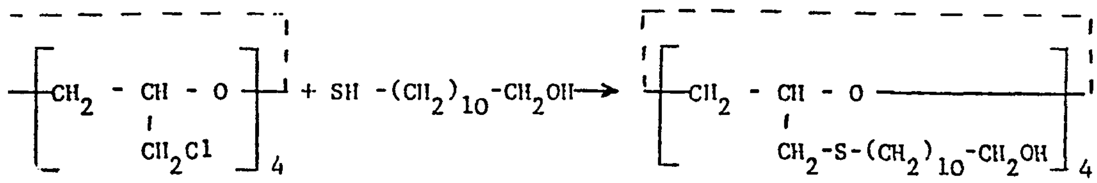

(IV) (II) (V)

On dissout dans 600 ml d'éthanol absolu, 74 g (0,8 équivalent en chlore de tétramère d'épichlorhydrine et 167 g (0,8 mole) de composé de formule (II).

On coule ensuite, goutte à goutte, à 40 °C, en 30 minutes, 152 g (0,82 mole) de liqueur méthanolique de méthylate de sodium à 5,4 meq/g.

Après 7 heures de chauffage à reflux, le taux de réaction est de 96 %.

On neutralise éventuellement avec HCl concentré l'alcalinité résiduelle puis on sépare par filtration le chlorure de sodium formé.

Après élimination de l'éthanol par distillation on obtient un composé solide dont l'indice de thioéther est de 3,75 meq/g et l'indice d'hydroxyle de 4,1 meq/g.

Exemple 5

Préparation du composé intermédiaire

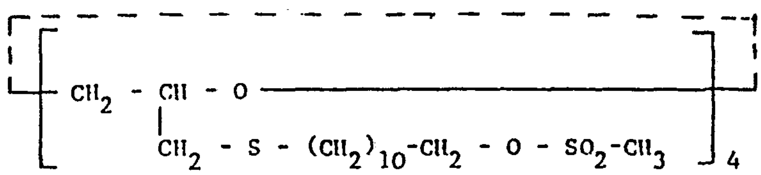

(VIII)

(ester méthane sulfonique du composé (V) préparé dans l'exemple 4).

On dissout 180 g de composé (V) (0,7 équivalent théorique en groupement hydroxyle) dans 300 ml de benzène anhydre. On ajoute 71,5 g de triéthylamine (0,7 mole) puis, à la température de 15 °C en 30 minutes, 80 g (0,7 mole) de méthane sulfochlorure. On maintient ensuite sous agitation pendant 2 heures entre 15 et 20 °C.

On filtre le chlorhydrate de triéthylamine que l'on rince deux fois avec 200 ml de benzène.

La phase benzénique est extraite à l'eau puis séchée sur sulfate de sodium.

Après élimination du solvant, on obtient le composé de formule (VIII) sous forme d'une huile jaune clair dont l'indice de saponification est de 3,08 meq/g.

Exemple 6

Préparation du composé intermédiaire :

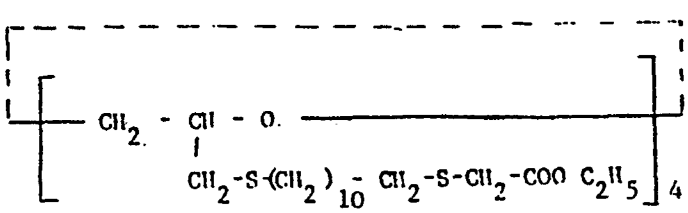

(IX)

A 34 g (100 meq) de composé intermédiaire de formule (VIII), préparé selon l'exemple 5, dispersé dans 100 g d'éthanol absolu, on ajoute, à la température ambiante, 12,3 g (0,1 mole) de thioglycolate d'éthyle et 18,6 g (0,1 mole) de méthylate de sodium dans le méthanol, à 5,4 meq/g.

On chauffe 7 heures au reflux. On filtre le méthane sulfonate de sodium formé et on élimine le solvant sous pression réduite.

On obtient le composé de formule (IX) sous forme d'une huile de couleur brun clair qui a les caractéristiques suivantes :

Indice de saponification : 2,7 meq/g

S % = 17,7

Exemple 7

Préparation d'un composé de formule I dans laquelle A désigne le groupement :

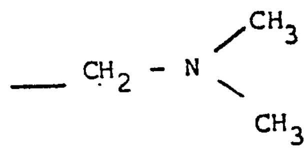

(a)

On dissout 43 g (0,13 équivalent) de composé intermédiaire de formule (VIII), préparé dans l'exemple 5, dans 150 g de benzène, puis on ajoute en 20 minutes, à la température ordinaire, 120 ml de solution benzénique de diméthylamine à 33 %.

Après 5 heures d'agitation à 40 °C le rendement calculé d'après l'indice d'acide apparu est pratiquement quantitatif.

L'amine en excès et le solvant sont éliminés sous pression réduite, après neutralisation de l'acide méthane sulfonique formé, avec du méthylate de sodium.

La masse réactionnelle est reprise avec 250 ml de chloroforme et extraite trois fois avec 60 ml d'eau.

Après séchage on obtient une huile brun clair contenant 11,5 % de S et 4,74 % d'azote.

Cette huile est soluble dans l'eau après neutralisation avec un acide, par exemple avec l'acide lactique ou l'acide chlorhydrique.

Exemple 8

Préparation d'un composé de formule (I) dans laquelle A désigne le groupement :

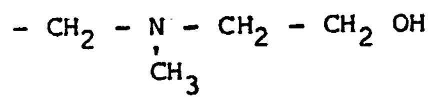

(a)

A 33,8 g (100 meq) de composé intermédiaire de formule (VIII) préparé selon l'exemple 5 on ajoute à 45 °C, en 10 minutes, 16 g (0,2 mole) de méthyléthanolamine. Après 8-9 heures à 80 °C la réaction est pratiquement quantitative.

On reprend la masse réactionnelle avec 100 ml d'isopropanol et on ajoute 16,3 g de liqueur méthanolique de méthylate de sodium à 5,4 meq/g. On filtre le méthane sulfonate de sodium et on élimine le solvant et l'amine en excès par distillation sous pression réduite.

On reprend le résidu au chloroforme et on lave la phase organique à l'eau.

On obtient ainsi une huile épaisse brun clair soluble en milieu acide dilué et ayant les caractéristiques suivantes :

S % = 10,1
N % = 4,42

Exemple 9

Préparation d'un composé de formule (I) dans laquelle A désigne le groupement :

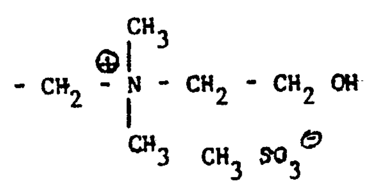

(c)

A 13,5 g (40 meq) de composé intermédiaire de formule (VIII) préparé selon l'exemple 5, on ajoute 3,63 g (0,04 mole) de diméthyl éthanolamine et on chauffe 3 heures à 60 °C.

On obtient ainsi une pâte jaune clair soluble dans l'eau.

Dosage du soufre S % = 15,63-15,89.

Exemple 10

Préparation d'un composé de formule (I) dans laquelle A désigne le groupement :

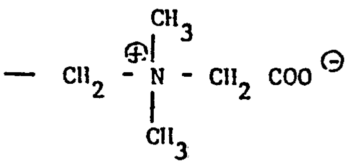

(d)

A 5,75 g (0,020 équivalent d'amine) de composé préparé selon l'exemple 7, on ajoute, à température ordinaire, 2,45 g (0,020 mole) de chloracétate d'éthyle.

On chauffe ensuite 1 heure à 50 °C.

Le taux de réaction, calculé d'après l'indice de basicité restant, est pratiquement quantitatif.

On ajoute 20 ml d'éthanol au milieu réactionnel, puis 20 meq de NaOH en solution dans 10 ml d'eau. Après 35 minutes de chauffage au reflux, on neutralise par addition de 2 ml d'acide chlorhydrique concentré.

Après élimination du chlorure de sodium et des solvants on reprend avec 20 ml de diméthyléther du diéthylèneglycol.

On obtient ainsi une poudre blanche soluble dans l'eau ayant un point de fusion de 150 °C et contenant 3,2 % d'azote et 8,6 % de soufre.

## Exemple 11

Préparation d'un composé de formule (I) dans laquelle A désigne le groupement :

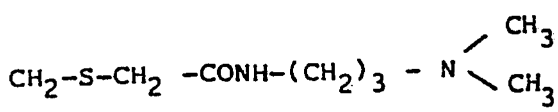

A 5,5 g (15,2 meq) de composé intermédiaire de formule (IX), préparé selon l'exemple 6, on ajoute 0,1 g (0,5 meq) de méthylate de sodium dans le méthanol et 3,5 g (30 meq) de diméthyl aminopropylamine.

On chauffe ensuite le milieu réactionnel pendant 15 heures à 110 °C.

La masse réactionnelle est reprise avec 20 ml de chloroforme et lavée avec trois fois 10 ml d'eau. La phase organique est ensuite séchée sur sulfate de sodium puis chauffée sous pression réduite pour éliminer l'éthanol formé et le méthanol utilisé comme solvant.

On obtient le composé recherché sous forme d'une pâte de couleur brun clair, soluble dans l'eau en présence d'un acide, par exemple l'acide chlorhydrique ou l'acide lactique.

Le composé obtenu présente les caractéristiques suivantes :

Indice de basicité : 2,5 meq/g

N % = 15,14

S % = 6,57

## Exemple 12

Préparation d'un composé de formule (I) dans laquelle A désigne le groupement :

$$—COO\ H \qquad (g)$$

On dissout dans 600 g d'éthanol absolu 22,3 g (0,24 équivalent en chlore) de tétramère d'épichlorhydrine et 55,2 g (0,24 mole) d'acide mercapto-11 undécanoïque-1, puis on ajoute en 45 minutes 96 g (0,48 mole) de méthylate de sodium dans le méthanol. On chauffe 5 heures à 50 °C, puis 3 heures au reflux.

On acidifie par addition d'acide chlorhydrique concentré. On dilue à l'eau chaude et on sépare, par décantation à 80 °C, la phase organique que l'on lave et sèche sous pression réduite.

On obtient ainsi le composé recherché sous forme d'un solide blanc de point de fusion 60 °C, d'indice d'acide 3,49 meq/g, soluble dans l'eau, sous forme de sel alcalin ou de sel d'amine, par exemple de sel de sodium, de potassium ou de triéthanolamine.

## Exemple 13

Préparation d'un composé de formule I dans laquelle A désigne le groupement :

$$—CH_2—S—CH_2—COOH \qquad (g)$$

A 18,1 g (0,05 équivalent) de composé de formule (IX) préparé dans l'exemple 6, dissous dans 200 ml d'éthanol à 96°, on ajoute 6 g de solution aqueuse de NaOH à 10 meq/g et 20 ml d'eau et on chauffe 1 heure au reflux.

On ajoute 200 ml d'eau puis après acidification avec HCl on extrait au chloroforme l'acide correspondant.

Après séchage sous pression réduite, on obtient le composé recherché sous forme d'un solide jaune clair, soluble dans l'eau, sous forme de sel alcalin ou de sel d'amine, par exemple sous forme de sel de sodium, de potassium, de triéthanolamine.

Les caractéristiques de ce composé sont les suivantes :

Indice d'acide : 2,7 meq/g

S % = 19,1

Exemple 14

Préparation d'un composé de formule I dans laquelle A désigne le groupement :

$$—CH_2—S—CH_2—CHOH—CH_2OH \quad (i)$$

A 17 g (50 meq) de composé intermédiaire de formule (VIII) préparé selon l'exemple 5, dispersé dans 20 g d'éthanol absolu, on ajoute 6 g (0,050 mole) de thioglycérol dans 10 g d'éthanol absolu, puis 13,5 g de liqueur méthanolique de méthylate de sodium à 3,7 meq/g.

On chauffe à reflux pendant 7 heures.

On sépare par filtration le méthane sulfonate de sodium formé et on élimine le solvant sous pression réduite.

On obtient le composé recherché qui présente les caractéristiques suivantes :

Indice OH = 5,8 meq/g

S % = 17,5

Exemple 15

Préparation d'un composé de formule I dans laquelle A désigne le groupement :

$$—CH_2—S—[CH_2—CHOH—CH_2—O]_6—H \quad (i)$$

A 7 g de composé préparé selon l'exemple 14 (0,02 équivalent en groupement hydroxyle), on ajoute 0,35 g de méthylate de sodium, puis en l'espace d'une heure, à la température de 150 °C, 7,65 g (0,1 mole) de glycidol.

On obtient ainsi le composé recherché sous la forme d'une pâte de couleur brun clair soluble dans l'eau.

Le point de trouble dans une solution salée contenant 25 % de chlorure de sodium est de 88 °C.

Exemple 16

Préparation d'un composé de formule I dans laquelle A désigne le groupement :

$$CH_2—O\,SO_3M \quad (h)$$

où M désigne l'hydrogène ou $NH_4$ ou $HN(CH_2—CH_2OH)_3$

a) Préparation de l'acide (M = H)

5,2 g de composé intermédiaire de formule (V), soit 0,02 équivalent en groupement hydroxyle, sont mis en solution dans 10 ml de chloroforme. On ajoute en l'espace de 10 minutes, 2,33 g de chlorhydrine sulfurique en solution dans 5 ml de chloroforme. La température s'élève de 20 à 30 °C ; l'agitation est maintenue 20 minutes.

Après élimination du chloroforme sous pression réduite, on obtient une huile jaune clair.

b) Préparation du sel d'ammonium ($M = NH_4$)

L'acide ainsi obtenu est mis en solution dans 10 ml d'éthanol à 90°. On ajoute sous agitation 6 ml d'ammoniaque 6 N. Après concentration on reprend avec 2 ml d'éthanol absolu et 10 ml d'éther ; ensuite le sel d'ammonium est essoré puis lavé par 5 ml d'éther. Il se présente sous forme d'une poudre blanche soluble dans l'eau à chaud. Par refroidissement la solution aqueuse gélifie en donnant une masse translucide.

c) Préparation du sel de triéthanolamine $M = HN(CH_2—CH_2OH)_3$

A l'acide mis en solution dans 10 ml d'éthanol à 90°, on ajoute 3 g de triéthanolamine en solution dans 10 ml d'éthanol à 90 °C. Le solvant est éliminé par distillation sous pression réduite. Le produit se présente sous la forme d'une pâte jaune clair, soluble dans l'eau à chaud, donnant une opalescence à froid.

Exemple 17

Préparation d'un mélange de composés de formule I dans laquelle A désigne le groupement :

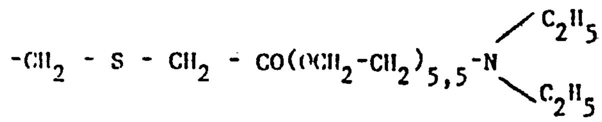

(f)

(groupement du type (f) dans lequel w = 1 et n = 5,5)

18,07 g de produit intermédiaire de formule (IX), (soit 0,05 équivalent en groupement ester) sont mélangés avec 17,3 g de N,N-diéthylaminoéthanol polyoxyéthyléné (0,05 mole).

Sous agitation on ajoute 1 g de méthylate de sodium en solution méthanolique à 30 %. On porte à 110 °C en éliminant l'éthanol à pression ordinaire, puis sous pression réduite pendant 1 h 30.

Le produit ainsi obtenu se présente sous la forme d'une huile brune, très dispersible dans l'eau, soluble dans l'acide acétique ou dans l'acide lactique dilué.

Indice de basicité = 1,6 meq/g.

Exemple 18

Préparation du mélange de composés de formule I dans laquelle A désigne le groupement :

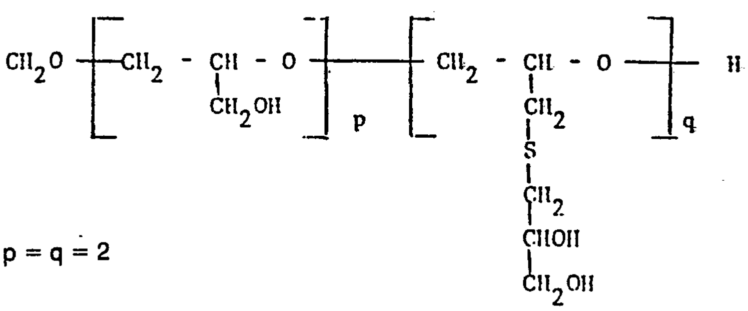

(j)

p = q = 2

A 13 g de composé intermédiaire de formule (V) (0,05 équivalent en groupement hydroxyle) fondu, on ajoute 0,1 ml de complexe éthéré de $BF_3$ et 1 g de chlorure de zinc sec. On introduit sous agitation en 20 minutes, le mélange de 13 g de tertiobutylglycidyléther (0,1 mole) et 9,25 g d'épichlorhydrine (0,1 mole). On chauffe 8 heures à 105 °C sous agitation. On ajoute ensuite 0,25 g d'acide sulfo-acétique et on chauffe le mélange à 100 °C sous agitation jusqu'à la fin du dégagement gazeux. Durée de la réaction : environ 8 heures.

La masse réactionnelle est reprise avec 40 ml de dichloréthane et la solution lavée avec 40 ml d'eau saturée en bicarbonate de sodium, puis avec 40 ml d'eau. La phase organique est séchée sur sulfate de sodium anhydre et concentrée à sec.

15,1 g de produit ainsi obtenu sont mis en solution dans 50 ml d'éthanol absolu. On ajoute 6,95 g de thioglycérol, et on chauffe le mélange à 45 °C.

On ajoute sous agitation 9,5 g de solution méthanolique de méthylate de sodium à 6,1 meq/g.

Le milieu réactionnel est maintenu pendant 8 heures au reflux. La réaction est suivie par dosage de la fonction mercaptan. Le chlorure de sodium formé est séparé par filtration et le filtrat concentré à sec sous pression réduite.

On obtient une huile épaisse, brune, dispersible dans l'eau. Le point de trouble mesuré à 5 % dans une solution aqueuse à 25 % en poids de butyldiglycol, est de 79 °C.

Exemple 19

On prépare la lotion colorante suivante :

| | |
|---|---|
| — Composé de l'exemple 14 | 1 g |
| — N-(amino-3-propyl) amino-1-anthraquinone | 0,05 g |
| — Eau q.s.p. | 100 g |

Cette solution, appliquée pendant 30 minutes sur des cheveux décolorés leur confère, après rinçage, shampooing et rinçage, une nuance rose saumon clair.

Exemple 20

On prépare la lotion colorante suivante :

| | |
|---|---|
| — Composé de l'exemple 9 | 1,2 g |
| — N-(β-hydroxy éthyl) amino-4 phényl azo nitro-4-phényl | 0,1 g |
| — Eau q.s.p. | 100 g |

Cette solution, appliquée pendant 30 minutes sur des cheveux décolorés, leur confère, après rinçage, passage au shampooing et rinçage, une nuance sable doré.

Exemple 21

On prépare la lotion suivante :

| | |
|---|---|
| — Sel de triéthanolamine du composé de l'exemple 13 | 1 g |
| — bis-(méthylamino)-1,4 nitro-2 phényl | 0,08 g |
| — Eau q.s.p. | 100 g |

Cette lotion, appliquée pendant 30 minutes sur des cheveux décolorés, leur confère, après rinçage, shampooing et rinçage, une nuance rose cyclamen clair.

Exemple 22

On prépare le shampooing anionique de composition suivante :

| | |
|---|---|
| — Composé de l'exemple 7 | 0,6 g |
| — Lauryl sulfate de triéthanolamine | 6 g |
| — Diéthanolamide laurique | 3 g |
| — Acide lactique q.s.p. pH 6 | |
| — Eau q.s.p. | 100 g |

Exemple 23

On prépare le shampooing non ionique de composition suivante :

| | |
|---|---|
| — Composé de l'exemple 8 | 0,8 g |
| — Hydroxy alcoyl éther de polyglycérol de formule : | |

$$R—CH\,OH—CH_2\,O—[CH_2—CH\,OH—CH_2\,O]_{3,5}—H$$

| | |
|---|---|
| où R représente un mélange de radicaux alkyle ayant de 9 à 12 atomes de carbone | 9,2 g |
| — Acide lactique q.s.p. pH 6,7 | |
| — Eau q.s.p. | 100 g |

On applique les solutions des exemples 22 ou 23 sur une chevelure préalablement mouillée de façon à émulsionner toutes les salissures. On rince et on effectue une deuxième application ; on obtient une mousse abondante et on rince. Les cheveux séchés se démêlent facilement et se mettent en forme aisément.

**Revendications** (pour les Etats contractants : BE, CH, DE, FR, GB, IT, LU, NL, SE)

1. Polyéthers cycliques tensio-actifs de formule générale :

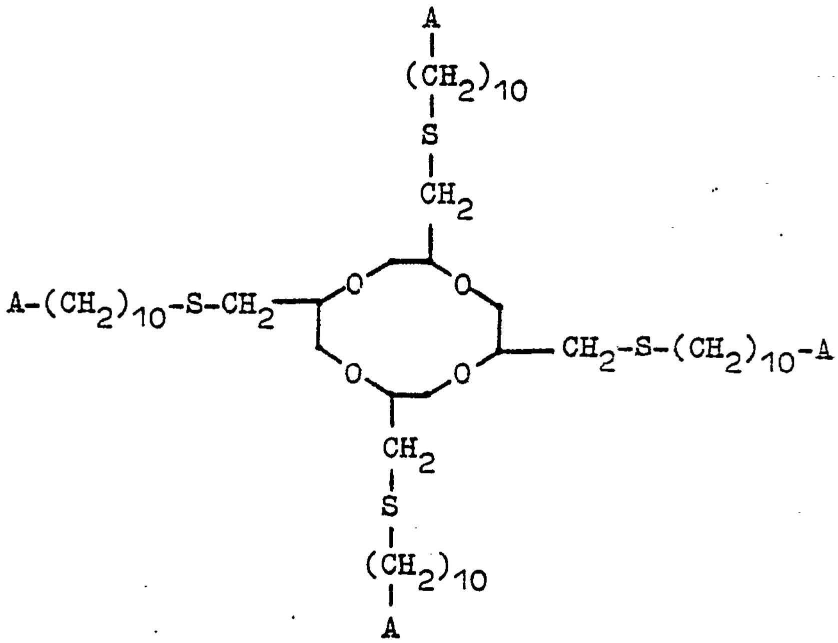

(I)

dans laquelle A désigne :

— un ensemble hydrophile cationique, anionique, zwitterionique ou non ionique, comportant au moins un groupement choisi parmi les groupements amine, ammonium, ammonio alcoyl carboxylate, ammonio alcoyl sulfonate ;

— le groupement

$$-[CH_2-S-CH_2]_w-COO\ M_1 \quad (g)$$

où *w* désigne le nombre 0 ou 1 ;

$M_1$ désigne un métal alcalin ;

— le groupement

$$-CH_2-O-SO_3\ M \quad (h)$$

où M désigne un atome d'hydrogène, ou un métal alcalin ;

— le groupement

$$-CH_2-S-[Y-O]_n-H \quad (i)$$

où *n* désigne un nombre quelconque de 1 à 10,

Y désigne le radical éthylène ou hydroxypropylène ;

— le groupement

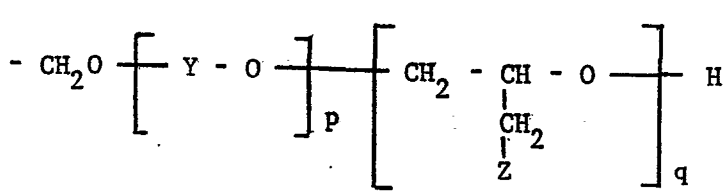

(j)

où p et q, identiques ou différents, désignent un nombre quelconque de 0 à 10, cependant p et q ne désignent pas simultanément zéro,

Y a la signification indiquée sous (i) ;

Z désigne l'un des groupements suivants :

$-OH$ ; $-S-CH_2-CH_2OH$ ; $-S-CH_2-CHOH-CH_2OH$ ; $-S-CH_2-COO\ M$ ; $-SO_3M$ ;

de plus, quand p désigne zéro ou quand Y désigne éthylène, Z désigne également $-O-SO_3-M$ ou $-OCO-CH_2-SO_3M$ ;

M désigne un atome d'hydrogène ou un métal alcalin;

2. Polyéther cyclique tensio-actif selon la revendication 1, caractérisé par le fait que le groupement hydrophile A contenant des groupements amine, ammonium, ammonio alcoyl carboxylate et/ou ammonio alcoyl sulfonate est choisi parmi les suivants :

— le groupement

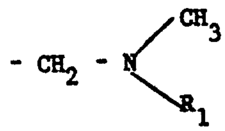

(a)

où $R_1$ désigne $CH_3$ ou $—[CH_2—CH_2—O]_n—H$

où *n* désigne un nombre quelconque de 1 à 10

— le groupement

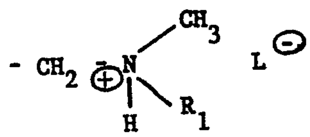

(b)

où $L^{\ominus}$ désigne un anion d'acide organique ou minéral

$R_1$ a la signification indiquée pour (a)

— le groupement

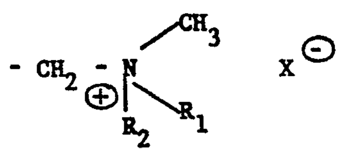

(c)

où $R_2$ désigne un radical alkyle ou hydroxyalkyle ayant de 1 à 3 atomes de carbone ;

$X^{\ominus}$ désigne un anion et de préférence un anion chlorure, bromure, iodure, méthyl sulfate, mésylate ou tosylate

$R_1$ a la signification indiquée sous (a)

— le groupement

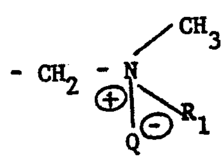

(d)

où $Q^{\ominus}$ désigne l'un des groupements

$—CH_2—COO^{\ominus}$, $—CH_2—CH_2—COO^{\ominus}$ ou $—CH_2—CH_2—CH_2—SO_3^{\ominus}$ ;

$R_1$ a la signification indiquée pour (a)

— le groupement

$$—(CH_2—S—CH_2)_w—CONH—(CH_2)_m—B \quad (e)$$

où *m* désigne 2 ou 3,

*w* désigne le nombre 0 ou 1,

B désigne l'un des groupements

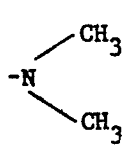 ; 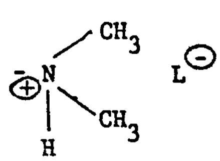 ; 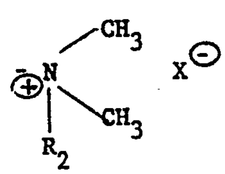 ; 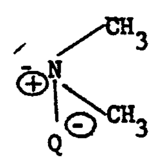

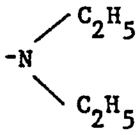 , 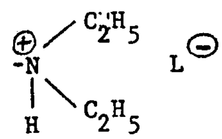 , 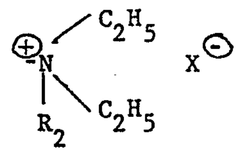 et 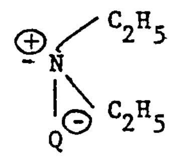

dans lesquels

$L^{\ominus}$ a la signification indiquée pour (b),

$R_2$ et $X^{\ominus}$ ont la signification indiquée pour (c),

$Q^{\ominus}$ a la signification indiquée pour (d) ;

— le groupement

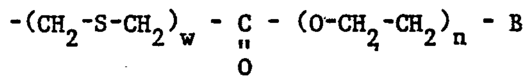 (f)

où *w* désigne le nombre 0 ou 1,

*n* désigne un nombre quelconque de 1 à 10,

B a la signification indiquée pour (e) ;

— le groupement

$$—[CH_2—S—CH_2]_{\overline{w}}—COOM_1 \qquad (g')$$

où *w* désigne le nombre 0 ou 1,

$M_1$ désigne un groupement ammonium

— le groupement

$$—CH_2—O—SO_3M \qquad (h')$$

où M désigne un groupement ammonium ;

— le groupement

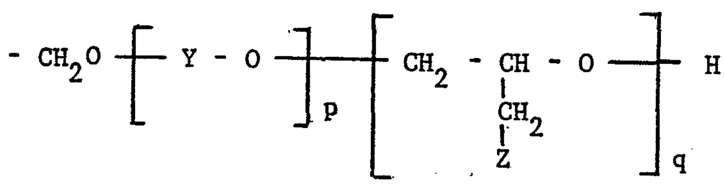 (j')

où p et q, identiques ou différents, désignent un nombre quelconque de 0 à 10, cependant p et q ne désignent pas simultanément zéro,

Y désigne le radical éthylène ou hydroxypropylène,

Z désigne l'un des groupements suivants :

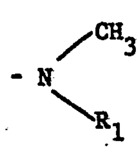 ; 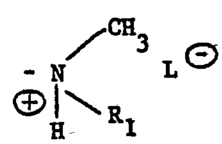 ; 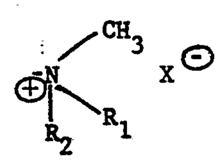 ; 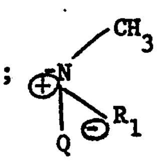

$—S—CH_2—COO\ M$ ; $—SO_3\ M$ ;

de plus, quand p désigne zéro ou quand Y désigne éthylène, Z désigne également $—O—SO_3—M$ ou $—OCO—CH_2—SO_3\ M$.

dans ces groupements :

$R_1$ désigne $CH_3$ ou $[CH_2—CH_2—O]_n—H$, où *n* désigne un nombre quelconque de 1 à 10, $L^{\ominus}$ désigne un anion d'un acide organique ou minéral,

$R_2$ désigne un radical méthyle, hydroxyéthyle ou dihydroxypropyle,

$X^{\ominus}$ désigne un anion,

$Q^{\ominus}$ désigne $—CH_2—COO^-$, $—CH_2—CH_2—CH_2—COO^-$ ou $CH_2—CH_2—CH_2—SO_3^-$ ;

M désigne un groupement ammonium.

3. Composés intermédiaires pour la préparation des composés de formule (I) selon la revendication 1, répondant à la formule :

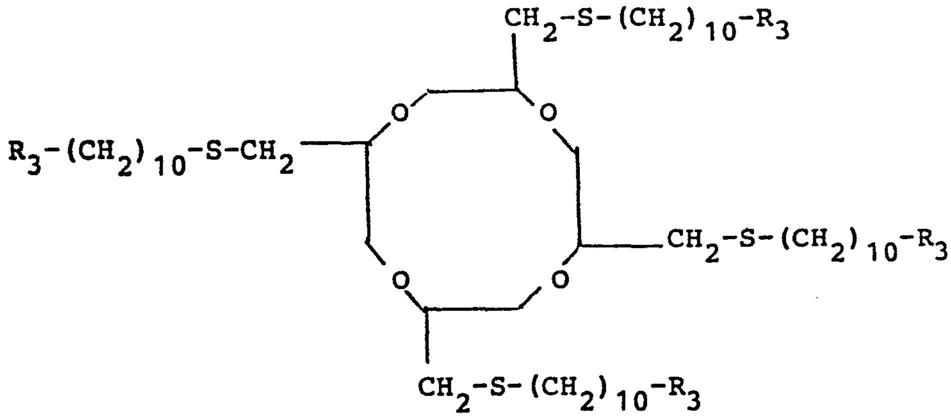

(VII)

où $R^3$ désigne $-CH_2OH$ ; $-CH_2-O-SO_2-CH_3$ ; $-CH_2-O-SO_2-C_6H_4-CH_3$ ; $-COOH$ ; $-COOCH_3$ ; $-COOC_2H_5$ ; $CH_2-S-CH_2-COOH$ ; $-CH_2-S-CH_2-COOCH_3$ ou $-CH_2-S-CH_2-COOC_2H_5$.

4. Procédé de préparation des composés intermédiaires de formule (VII), selon la revendication 3, caractérisé par le fait que (1) on additionne l'acide thioacétique sur l'alcool undécylénique ou l'acide undécylénique ; (2) on saponifie le produit de réaction préparé en (1) pour obtenir respectivement le mercapto-11 undécanol ou l'acide mercapto-11-undécanoïque-1 ; (3) on estérifie éventuellement l'acide mercapto-11 undécanoïque-1 en ester méthylique ou éthylique ; (4) on condense le composé obtenu en (2) ou (3) sur le tétramère de l'épichlorhydrine ou de l'épibromhydrine, ou bien on condense le thioglycolate de méthyle ou d'éthyle sur le dérivé halogéné, le mésylate ou le tosylate du composé obtenu en faisant réagir le mercapto-11 undécanol-1 sur le tétramère de l'épichlorhydrine ou de l'épibromhydrine.

5. Procédé de préparation d'un polyéther cyclique polypode tensio-actif de formule (I) dans laquelle A désigne le groupement :

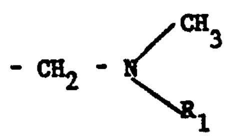

(a)

où $R_1$ désigne $CH_3$ ou $-[CH_2-CH_2-O]_n-H$

*n* désignant un nombre quelconque de 1 à 10, par réact. de la diméthylamine ou de la méthyléthanolamine avec le dérivé chloré ou bromé du composé intermédiaire de formule (V)

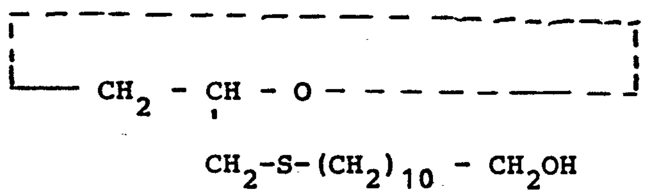

(V)

obtenu par réaction du chlorure de thionyle ou de l'acide bromhydrique avec ce composé intermédiaire (V), ou bien avec le mésylate ou tosylate du composé intermédiaire de formule (V), à une température de 20 à 160 °C, à la pression ordinaire ou en autoclave ; lorsque l'amine utilisée est la méthyléthanolamine, le composé obtenu est éventuellement oxyéthyléné avec 1 à 10 moles d'oxyde d'éthylène.

6. Procédé de préparation d'un polyéther cyclique tensio-actif polypode de formule (I) dans laqu. A désigne le groupement :

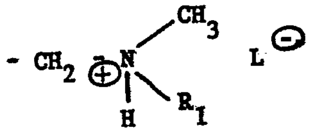

(b)

où $R_1$ désigne $CH_3$ ou $—[CH_2—CH_2—O]_n—H$ où *n* désigne un nombre quelconque de 1 à 10, et $L^{\ominus}$ désigne un anion organique ou minéral et de préférence un chlorure, bromure, sulfate, phosphate, acétate, glycolate, lactate, tartrate, méthane sulfonate para-toluène sulfonate, par salification avec un acide de formule HL, où L a la signification ci-dessus indiquée.

7. Procédé de préparation d'un polyéther cyclique tensio-actif polypode de formule (I) dans laquelle A désigne le groupement :

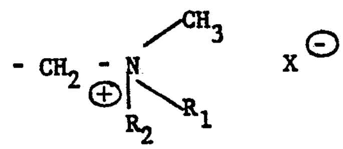

(c)

où $R_2$ désigne un radical méthyle, hydroxyéthyle ou dihydroxypropyle,

$X^{\ominus}$ désigne un anion et de préférence un anion chlorure, bromure, iodure, méthyl sulfate, mésylate ou tosylate ; $R_1$ désigne $CH_3$ ou $—[CH_2—CH_2—O]_n—H$ où *n* désigne un nombre quelconque de 1 à 10, par alcoylation d'un composé 1 (a), selon la revendication 2 avec un agent alcoylant choisi de préférence parmi le chlorure, bromure, iodure, sulfate, mésylate ou tosylate de méthyle, la chlorhydrine du glycol ou la chlorhydrine du glycérol.

8. Procédé selon la revendication 7, caractérisé par le fait que $X^-$ désigne un anion mésylate ou tosylate et qu'on fait réagir les mésylate ou tosylate du composé intermédiaire de formule (V)

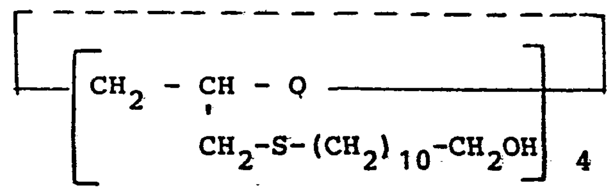

(V)

avec une amine de formule :

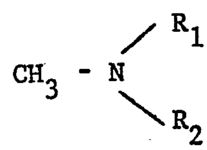

où $R^1$ désigne $CH_3$ ou $—[CH_2—CH_2—O]_n—H$ ; *n* désignant un nombre quelconque de 1 à 10 ;

$R_2$ désigne un radical méthyle, hydroxyéthyle ou dihydroxypropyle, à une température comprise entre 20 et 100 °C.

9. Procédé de préparation d'un polyéther cyclique tensio-actif polypode de formule (I) dans laquelle A désigne le groupement :

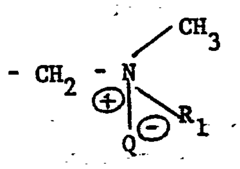

(d)

où $Q^{\ominus}$ désigne l'un des groupements :

$—CH_2COO^{\ominus}$, $—CH_2—CH_2—COO^{\ominus}$, $—CH_2—CH_2—CH_2—SO_3^{\ominus}$

$R_1$ désigne $CH_3$ ou $[CH_2—CH_2—O]_n—H$ ou *n* désigne un nombre quelconque de 1 à 10 par alcoylation des composés de formule (I) dans laquelle A désigne le groupement (a), avec le chloroacétate ou chloropropionate de sodium ou la propane sultone.

10. Procédé de préparation d'un polyéther cyclique tensio-actif polypode de formule (I) dans laquelle A désigne le groupement :

$$—(CH_2—S—CH_2)_w—CONH—(CH_2)_m—B \qquad (e)$$

où *m* désigne le nombre 2 ou 3 ; *w* désigne le nombre 0 ou 1 ;

B désigne l'un des groupements :

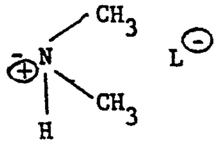 , 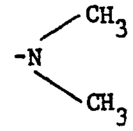 , 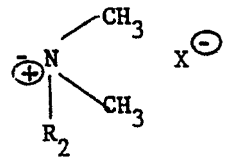 , 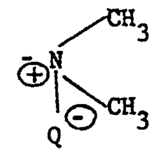

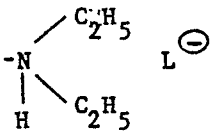 , 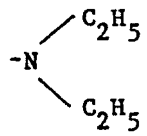 , 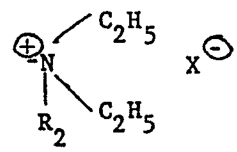 et 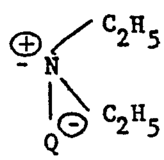

où $R_2$ et $X^{\ominus}$ ont la signification indiquée dans la revendication 7,

$Q^{\ominus}$ a la signification indiquée dans la revendication 9, et

$L^{\ominus}$ a la signification indiquée dans la revendication 6 ; par condensation du composé intermédiaire de formule (VI) :

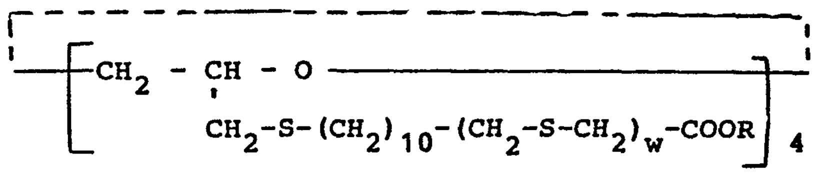 (VI)

où *w* désigne le nombre 0 ou 1 ;

R désigne $CH_3$ ou $C_2H_5$ avec la diméthylamino éthylamine, la diméthylaminopropylamine, la diéthylamino éthylamine, la diéthylamino propylamine, les fonctions amines ainsi obtenues étant éventuellement salifiées ou alcoylées et/ou transformées en groupes ammonium, ammonio acétate, ammonio propionate ou ammonio propane sulfonate.

11. Procédé de préparation d'un polyéther cyclique tensio-actif polypode de formule (I) dans laquelle A désigne le groupement :

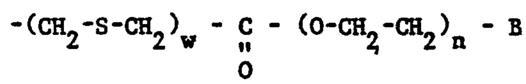 (f)

où *w* désigne le nombre 0 ou 1,

*n* désigne un nombre quelconque de 1 à 10,

B a la signification indiquée dans la revendication 10, par condensation du composé intermédiaire de formule (VI) figurant dans la revendication 10, avec une diméthylamine oxyéthylénée de formule :

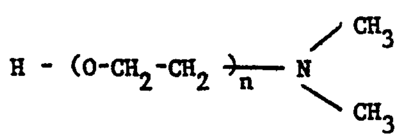

où *n* désigne un nombre quelconque de 1 à 10 ;

la condensation s'effectuant à une température de 20 à 160 °C ;

le groupement amine étant éventuellement salifié ou alcoylé et/ou transformé en groupe ammonium, ammonio acétate, ammonio propionate ou ammonio propane sulfonate.

12. Procédé de préparation d'un polyéther cyclique tensio-actif polypode de formule (I) dans laquelle A désigne le groupement :

$$-[CH_2-S-CH_2]_w-COOM_1 \qquad (g)$$

où *w* désigne le nombre 0 ou 1,

$M_1$ désigne un groupement ammonium ou un métal alcalin et de préférence le lithium, le sodium ou le potassium, par saponification de l'ester méthylique ou éthylique du composé intermédiaire de formule (VI) figurant dans la revendication 10, suivie de neutralisation avec un hydroxyde alcalin ou l'ammoniaque, ou lorsque *w* désigne zéro, directement par réaction du tétramère de l'épichlorhydrine ou de l'épibromhydrine avec l'acide mercapto-11 undécanoïque-1, en présence de méthylate ou d'éthylate de sodium ou de potassium, suivie de neutralisation avec un hydroxyde alcalin ou l'ammoniaque.

13. Procédé de préparation d'un polyéther cyclique tensio-actif polypode de formule (I) dans laquelle A désigne le groupement :

$$—CH_2—O—SO_3M \quad (h)$$

où M désigne un atome d'hydrogène, un groupement ammonium ou un métal alcalin, par sulfatation du composé intermédiaire de formule (V) figurant dans la revendication 5, avec la chlorhydrine sulfurique et neutralisation éventuelle avec un hydroxyde alcalin ou l'ammoniaque.

14. Procédé de préparation d'un polyéther cyclique tensio-actif polypode de formule (I) dans laquelle A désigne le groupement :

$$—CH_2—S—[Y—O]_n—H \quad (i)$$

où *n* désigne un nombre quelconque de 1 à 10,

Y désigne le radical éthylène ou hydroxypropylène, par réaction du mercapto-éthanol ou mercapto-glycérol avec le mésylate ou tosylate du composé intermédiaire de formule (V) ou avec un composé halogéné du composé intermédiaire de formule (V), figurant dans la revendication 5, le composé résultant pouvant être condensé avec 1 à 10 moles d'oxyde d'éthylène et/ou de glycérol.

15. Procédé de préparation d'un polyéther cyclique tensio-actif polypode de formule (I) dans laquelle A désigne le groupement :

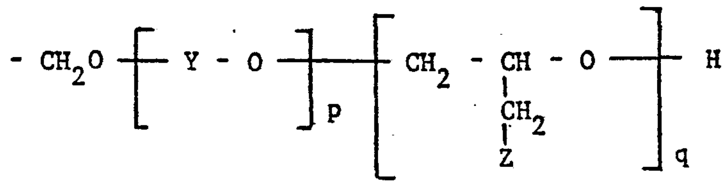 (j)

où p et q, identiques ou différents, désignent un nombre quelconque de 0 à 10, p et q ne désignant pas simultanément zéro,

Y désigne le radical éthylène ou hydroxypropylène,

Z désigne l'un des groupements :

$—OH$ ; $—S—CH_2—CH_2OH$ ; $—S—CH_2—CHOH—CH_2OH$ ;

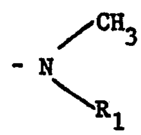 ; 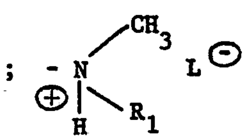 ; 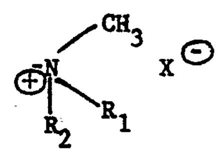 ; 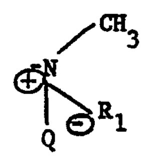

$—S—CH_2—COOM$ ; $—SO_3M$ ;

de plus, quand p = 0 ou quand Y désigne le groupement éthylène, Z en outre désigne l'un des groupements :

$—OSO_3M$ ou $—OCO—CH_2—SO_3M$

où $R_1$ désigne $CH_3$ ou $—[CH_2—CH_2—O]_n—H$

*n* désigne un nombre quelconque de 1 à 10,

$L^\ominus$ désigne un anion d'acide organique ou minéral,

$X^\ominus$ désigne un anion chlorure, bromure, iodure, méthylsulfate, mésylate ou tosylate,

M désigne un atome d'hydrogène, un groupement ammonium ou un métal alcalin, par réaction de polyaddition sur le composé intermédiaire de formule (V) figurant dans la revendication 5, de réactifs à fonction époxyde choisis parmi l'oxyde d'éthylène, le tertiobutylglycidyléther (TBGE), l'épichlorhydrine, l'épibromhydrine et leurs mélanges, les groupements protecteurs tertiobutoxy étant remplacés par des groupements hydroxyles, les atomes d'halogène provenant d'une épihalohydrine étant remplacés par un groupement hydroxyle (par réaction avec l'acétate de sodium ou de potassium, suivie de saponification ou alcoolyse de l'ester acétique formé) ou par un groupement thio-hydroxyéthyle, thio-dihydroxypropyle ou thioglycolate, par réaction avec le thioéthanol, le thioglycérol ou le thioglycolate de méthyle ou d'éthyle ou par un groupement amine par réaction avec la diméthylamine ou la méthyléthanolamine ; lorsqu'on utilise la méthyléthanolamine le composé obtenu peut éventuellement être amené à réagir avec l'oxyde d'éthylène, les fonctions amine ainsi obtenues pouvant être salifiées et transformées en fonction ammonium, ammonio-acétate, ammonio-propionate ou ammonio-propane sulfonate ;

les groupements hydroxyles obtenus pouvant éventuellement être estérifiés et remplacés par des groupements sulfate ou sulfoacétate, respectivement par réaction avec la chlorhydrine sulfurique ou l'acide sulfoacétique.

16. Composition renfermant au moins $0{,}5.10^{-2}$ gramme/litre d'un composé tensio-actif cyclique de formule (I).

17. Composition cosmétique ou pharmaceutique, caractérisée par le fait qu'elle renferme au moins $0{,}5.10^{-2}$ gramme/litre d'un composé de formule (I) en présence d'un véhicule aqueux ou hydroalcoolique.

18. Composition selon la revendication 17, caractérisée par le fait que la solution hydroalcoolique renferme un alcool ou un polyol ayant de 1 à 4 atomes de carbone.

19. Composition selon les revendications 16 ou 17, caractérisée par le fait qu'elle se présente sous la forme d'une solution, d'une crème, d'un gel, d'une émulsion ou d'un aérosol.

20. Composition cosmétique pour le traitement des cheveux contenant en solution dans un solvant choisi dans un groupe formé par l'eau et une solution hydroalcoolique, une quantité cosmétiquement efficace de un ou plusieurs composés de formule (I).

21. Composition de shampooing pour le traitement des cheveux contenant en solution dans un solvant choisi dans un groupe formé par l'eau et une solution hydroalcoolique, une quantité cosmétiquement efficace d'un ou plusieurs composés de formule (I).

22. Composition de shampooing pour cheveux, contenant en solution d n solvant choisi dans le groupe formé par l'eau et un solvant hydroalcoolique, une quantité cosmé ment efficace d'un ou plusieurs adjuvants choisis dans le groupe formé par les tensio-actif oniques, cationiques, amphotères, zwitterioniques, non ioniques, les parfums, les colorants, les c rvateurs, les épaississants, les stabilisateurs de mousse, les agents adoucissants, les agents de re cturation des cheveux, les agents antipelliculaires, les résines cosmétiques, les synergistes de mousse, les électrolytes.

23. Procédé de traitement des cheveux consistant à appliquer sur les cheveux humains une quantité efficace d'une composition comprenant en solution dans un solvant choisi dans un groupe formé par l'eau et une solution hydroalcoolique, une quantité cosmétiquement efficace d'un ou plusieurs polyéthers cycliques tensio-actifs de formule (I).

24. Composition tinctoriale pour cheveux contenant en solution dans un solvant choisi dans un groupe formé par l'eau et une solution hydroalcoolique une quantité cosmétiquement efficace d'un ou plusieurs composés de formule (I), ainsi qu'un colorant pour cheveux et éventuellement un ou plusieurs adjuvants choisis dans le groupe formé par les tensio-actifs anioniques, cationiques, non ioniques, zwitterioniques, amphotères et leurs mélanges, les parfums, les conservateurs, les épaississants, les adoucissants, les résines cosmétiques, les séquestrants.

25. Composition selon la revendication 24, caractérisée par le fait que le colorant est choisi parmi les colorants anthraquinoniques, les colorants azoïques, les colorants nitrés de la série benzénique, les indophénols, les indoanilines, les indamines.

**Revendications** (pour l'Etat contractant AT)

1. Procédé de préparation de polyéthers cycliques tensio-actifs de formule générale :

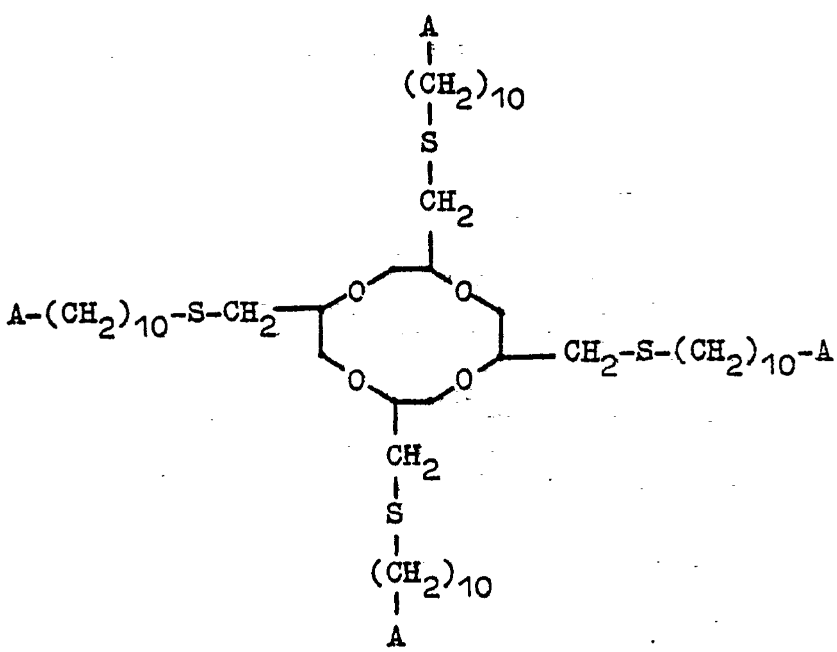

(I)

dans laquelle

A désigne un groupement cationique, anionique, zwitterionique ou non-ionique, choisi parmi les groupements suivants :

a) le groupement

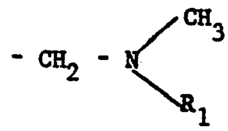

où $R_1$ désigne $CH_3$ ou $-[CH_2-CH_2-O]_n-H$

où *n* désigne un nombre quelconque de 1 à 10

b) le groupement

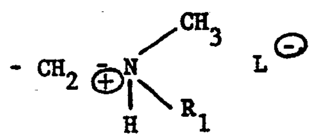

où $L^{\ominus}$ désigne un anion d'acide organique ou minéral

$R_1$ a la signification indiquée pour (a)

c) le groupement

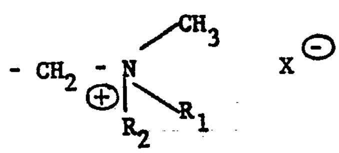

où $R_2$ désigne un radical alkyle ou hydroxyalkyle ayant de 1 à 3 atomes de carbone ;

$X^{\ominus}$ désigne un anion et de préférence un anion chlorure, bromure, iodure, méthyl sulfate, mésylate ou tosylate

$R_1$ a la signification indiquée sous (a)

d) le groupement

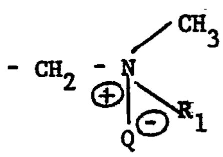

où $Q^{\ominus}$ désigne l'un des groupements $-CH_2-COO^{\ominus}$, $-CH_2-CH_2-COO^{\ominus}$ ou $CH_2-CH_2-CH_2-SO_3^{\ominus}$ ;

$R_1$ a la signification indiquée pour (a)

e) le groupement

$-(CH_2-S-CH_2)_w-CONH-(CH_2)_m-B$

où *m* désigne 2 ou 3,

*w* désigne le nombre 0 ou 1,

B désigne l'un des groupements

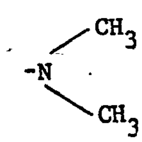 , 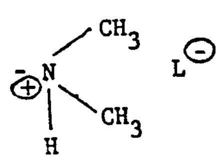 , 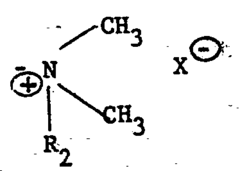 , 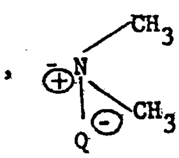

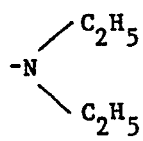 , 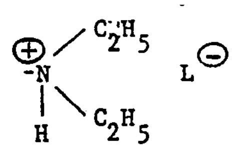 , 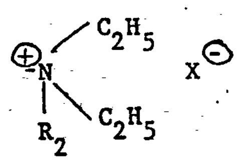 et 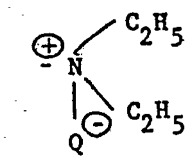

dans lesquels

$L^{\ominus}$ a la signification indiquée pour (b),

$R_2$ et $X^{\ominus}$ ont la signification indiquée pour (c),

$Q^{\ominus}$ a la signification indiquée pour (d) ;

f) le groupement

$$-(CH_2-S-CH_2)_w-\underset{\underset{O}{\|}}{C}-(O-CH_2-CH_2)_n-B$$

où *w* désigne le nombre 0 ou 1,

*n* désigne un nombre quelconque de 1 à 10,

B a la signification indiquée pour (e) ;

g) le groupement

$$-[CH_2-S-CH_2]_w-COOM_1$$

où *w* désigne le nombre 0 ou 1,

$M_1$ désigne un groupement ammonium ou un métal alcalin

h) le groupement

$$-CH_2-O-SO_3M$$

où M désigne un atome d'hydrogène, un groupement ammonium ou un métal alcalin

i) le groupement

$$-CH_2-S-[Y-O]_n-H$$

où *n* désigne un nombre quelconque de 1 à 10,

Y désigne le radical éthylène ou hydroxypropylène ;

j) le groupement

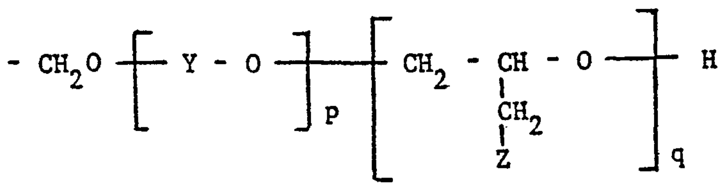

où p et q, identiques ou différents, désignent un nombre quelconque de 0 à 10, cependant p et q ne désignent pas simultanément zéro,

Y a la signification indiquée sous (i),

Z désigne l'un des groupements suivants :

$-OH$ ; $-S-CH_2-CH_2OH$ ; $-S-CH_2-CHOH-CH_2OH$ ;

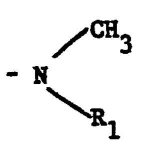 ; 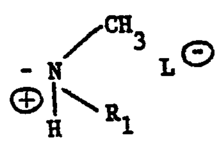 ; 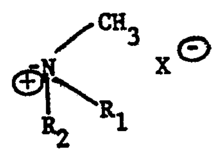 ; 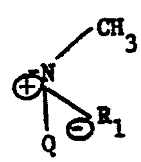

$-S-CH_2-COOM$ ; $-SO_3M$ ;

de plus, quand p désigne zéro ou quand Y désigne éthylène, Z désigne également $-O-SO_3-M$ ou $-OCO-CH_2-SO_3M$.

Dans ces groupements :

$R_1$ désigne $CH_3$ ou $[CH_2-CH_2-O]_n-H$, où *n* désigne un nombre quelconque de 1 à 10, $L^{\ominus}$ désigne un anion d'un acide organique ou minéral,

$R_2$ désigne un radical méthyle, hydroxyéthyle ou dihydroxypropyle,

$X^{\ominus}$ désigne un anion,

$Q^{\ominus}$ désigne $-CH_2-COO^{\ominus}$, $-CH_2-CH_2-COO^{\ominus}$ ou $CH_2-CH_2-CH_2-SO_3^{\ominus}$,

M désigne un atome d'hydrogène, un groupement ammonium, ou un métal alcalin ;

caractérisé par le fait que dans un premier stade on prépare le mercapto-11 undécanol-1 de formule

$$HS-(CH_2)_{10}-CH_2OH \quad (II)$$

ou l'acide mercapto-11-undécanoïque-1 de formule

$$HS-(CH_2)_{10}-COOH \quad (III)$$

que dans un deuxième stade on prépare le composé intermédiaire de formule (V)

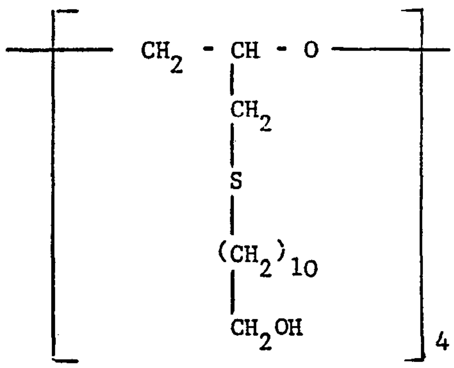

(V)

par réaction du mercapto-11 undécanol-1 avec le tétramère de l'épichlorhydrine ou de l'épibromhydrine de formule (IVa)

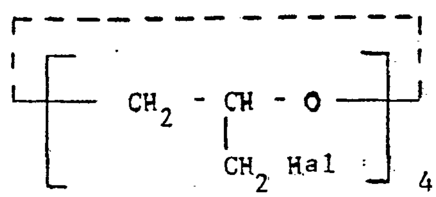

(IVa)

où Hal désigne le chlore ou le brome,
(le tétramère de l'épichlorhydrine ou de l'épibromhydrine étant préparé par polymérisation de l'épichlorhydrine ou de l'épibromhydrine en présence d'un catalyseur acide de Lewis) ;
que dans un troisième stade on prépare le composé intermédiaire de formule (VI)

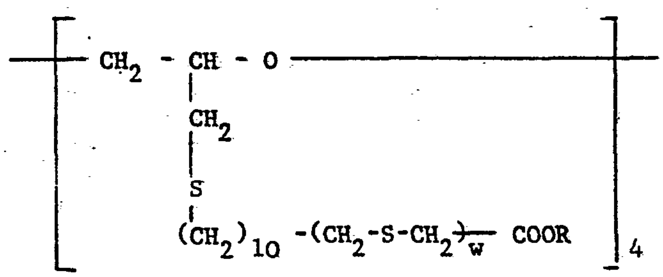

(VI)

où R désigne hydrogène, $CH_3$ ou $C_2H_5$, w désigne le nombre 0 ou 1
par réaction, soit de l'acide mercapto-11 undécanoïque-1, ou de son ester méthylique ou éthylique avec le tétramère de l'épichlorhydrine ou de l'épibromhydrine, soit du thioglycolate de méthyle ou d'éthyle avec le dérivé halogéné, le mésylate ou tosylate du composé intermédiaire de formule (V) ; qu'on prépare ensuite :

a) les composés Ia répondant à la formule (I) dans laquelle A désigne le groupement (a), par réaction de la diméthylamine ou de la méthyléthanolamine, avec le dérivé chloré ou bromé ou avec le mésylate ou tosylate du composé intermédiaire de formule (V) ;

b) les composés Ib répondant à la formule (I) dans laquelle A désigne le groupement (b), par salification des composés Ia correspondants, avec un acide minéral ou organique ;

(c) les composés Ic répondant à la formule (I) dans laquelle A désigne le groupement(c), par alcoylation des composés Ia correspondants, ou directement par réaction du mésylate ou tosylate du composé intermédiaire de formule (V) avec une amine de formule :

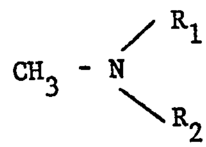

d) les composés Id répondant à la formule (i) dans laquelle A désigne le groupement (d) par alcoylation des composés Ia avec le chloroacétate ou chloropropionate de sodium ou la propane sultone ;

e) les composés Ie répondant à la formule (I) dans laquelle A désigne le groupement (e) par condensation du composé intermédiaire de formule (VI) avec la diméthylamino éthylamine, la diméthylamino propylamine, la diéthylamino éthylamine ou la diéthylamino propylamine ;

f) les composés If répondant à la formule (I) dans laquelle A désigne le groupement (f) par condensation du composé intermédiaire de formule (VI) avec une diméthylamine oxyéthylénée de formule :

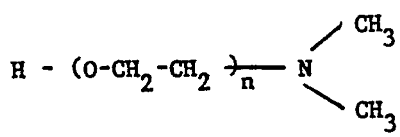

où *n* désigne un nombre quelconque de 1 à 10, les groupements amine pouvant ensuite être salifiés avec un acide minéral ou organique ou alcoylés avec un agent alcoylant ;

g) les composés Ig répondant à la formule (I) dans laquelle A désigne le groupement (g) par saponification de l'ester méthylique ou éthylique du composé intermédiaire de formule (VI), suivie de neutralisation avec un hydroxyde alcalin ou l'ammoniaque ou bien, lorsque *w* désigne zéro, directement par réaction du tétramère d'épichlorhydrine ou d'épibromhydrine avec l'acide mercapto-11 undécanoïque-1 en présence de méthylate ou d'éthylate de sodium ou de potassium, suivie de neutralisation avec un hydroxyde alcalin ou l'ammoniaque ;

h) les composés Ih répondant à la formule (I) dans laquelle A désigne le groupement (h) par sulfatation du composé intermédiaire de formule (V) avec la chlorhydrine sulfurique et neutralisation éventuelle avec un hydroxyde alcalin ou l'ammoniaque ;

i) les composés Ii répondant à la formule (I) dans laquelle A désigne le groupement (i) par réaction du mercapto-éthanol ou du mercapto-glycérol avec le mésylate ou tosylate du composé intermédiaire de formule (V) ou avec un composé halogéné dérivé de celui-ci, éventuellement suivie de polyaddition d'oxyde d'éthylène et/ou de glycidol, en présence d'un catalyseur alcalin ;

j) les composés Ij répondant à la formule (I) dans laquelle A désigne le groupement (j) par des réactions de polyaddition sur le composé intermédiaire de formule (V) de réactifs à fonction époxyde choisis parmi l'oxyde d'éthylène, le tertiobutylglycidyléther (TBGE), l'épichlorhydrine, l'épibromhydrine et leurs mélanges, les groupements protecteurs tertiobutoxy étant remplacés par des groupements hydroxyles, les atomes d'halogène provenant d'une épihalohydrine étant remplacés par un groupement hydroxyle (par réaction avec l'acétate de sodium ou de potassium, suivie de saponification ou alcoolyse de l'ester acétique formé) ou par un groupement thio-hydroxyéthyle, thio-dihydroxypropyle ou thioglycolate, par réaction avec le thioéthanol, le thioglycérol ou le thioglycolate de méthyle ou d'éthyle ou par un groupement amine par réaction avec la diméthylamine ou la méthyléthanolamine ; lorsqu'on utilise la méthyléthanolamine le composé obtenu peut éventuellement être amené à réagir avec l'oxyde d'éthylène, les fonctions amine ainsi obtenues pouvant être salifiées et transformées en fonction ammonium, ammonio-acétate, ammonio-proprionate ou ammonio-propane sulfonate ;

les groupements hydroxyles obtenus pouvant éventuellement être estérifiés et remplacés par des groupements sulfate ou sulfoacétate, respectivement par réaction avec la chlorhydrine sulfurique ou l'acide sulfoacétique.

2. Procédé de préparation selon la revendication 1, caractérisé par le fait qu'on prépare un polyéther cyclique tensio-actif de formule (I) dans laquelle A désigne le groupement :

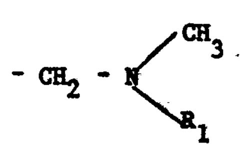

(a)

où $R_1$ désigne $CH_3$ ou $—[CH_2—CH_2—O]_n—H$

*n* désignant un nombre quelconque de 1 à 10,

par réaction de la diméthylamine ou de la méthyléthanolamine avec le dérivé chloré ou bromé du composé intermédiaire de formule (V), (la formule (V) figurant dans la revendication 1), ou bien avec le mésylate ou tosylate du composé intermédiaire de formule (V), à une température de 20 à 160 °C, à la pression ordinaire ou en autoclave ; lorsque l'amine utilisée est la méthyléthanolamine, le composé obtenu est éventuellement oxyéthyléné avec 1 à 10 moles d'oxyde d'éthylène.

3. Procédé selon la revendication 1, caractérisé par le fait qu'on prépare un polyéther cyclique tensio-actif de formule (I) dans laquelle A désigne le groupement :

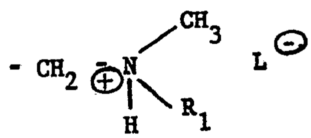

(b)

où $R_1$ a la signification indiquée dans la revendication 2, et $L^{\ominus}$ désigne un anion organique ou minéral et de préférence un chlorure, bromure, sulfate, phosphate, acétate, glycolate, lactate, tartrate, méthane sulfonate paratoluène sulfonate, par salification avec un acide de formule HL, où L a la signification ci-dessus indiquée.

4. Procédé selon la revendication 1, caractérisé par le fait qu'on prépare un polyéther cyclique tensio-actif de formule (I) dans laquelle A désigne le groupement :

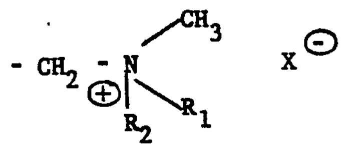

(c)

où $R_2$ désigne un radical méthyle, hydroxyéthyle ou dihydroxypropyle,

$X^{\ominus}$ désigne un anion et de préférence un anion chlorure, bromure, iodure, méthyl sulfate, mésylate ou tosylate ;

$R_1$ a la signification indiquée dans la revendication 2,

par alcoylation d'un composé Ia, selon la revendication 2, avec un agent alcoylant choisi de préférence parmi le chlorure, bromure, iodure, sulfate, mésylate ou tosylate de méthyle, la chlorhydrine du glycol ou la chlorhydrine du glycérol.

5. Procédé selon la revendication 4, caractérisé par le fait que $X^{\ominus}$ désigne un anion mésylate ou tosylate et qu'on fait réagir le mésylate ou tosylate du composé intermédiaire de formule (V)

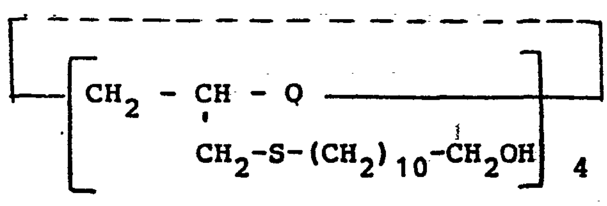

(V)

avec une amine de formule :

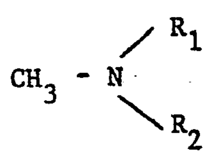

où $R_1$ désigne $CH_3$ ou $-[CH_2-CH_2-O]_n-H$ ; *n* désignant un nombre quelconque de 1 à 10 ;

$R_2$ désigne un radical méthyle, hydroxyéthyle ou dihydroxypropyle, à une température comprise entre 20 et 100 °C.

6. Procédé selon la revendication 1, caractérisé par le fait qu'on prépare un polyéther cyclique tensio-actif de formule (I) dans laquelle A désigne le groupement :

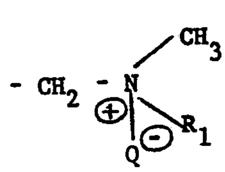

(d)

où $Q^{\ominus}$ désigne l'un des groupements :

$-CH_2COO^{\ominus}$, $-CH_2-CH_2-COO^{\ominus}$, $-CH_2-CH_2-CH_2-SO_3^{\ominus}$

$R_1$ a la signification indiquée dans la revendication 2, par alcoylation des composés de formule (I) dans laquelle A désigne le groupement (a), avec le chloracétate ou chloropropionate de sodium ou la propane sultone.

7. Procédé selon la revendication 1, caractérisé par le fait qu'on prépare un polyéther cyclique tensio-actif de formule (I) dans laquelle A désigne le groupement :

$$-(CH_2-S-CH_2)_w-CONH-(CH_2)_m-B \qquad (e)$$

où *m* désigne le nombre 2 ou 3 ; *w* désigne le nombre 0 ou 1 ;

B désigne l'un des groupements :

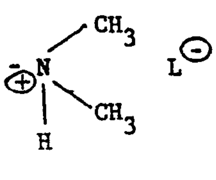 , 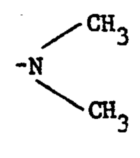 , 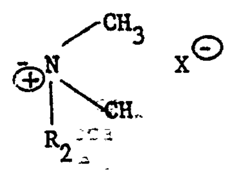 , 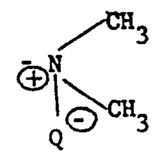

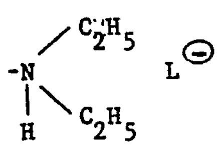 , 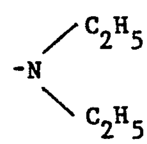 , 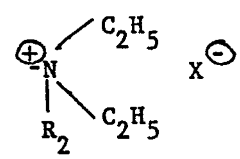 et 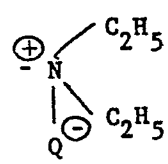

où $R_2$ et $X^{\ominus}$ ont la signification indiquée dans la revendication 4,

$Q^{\ominus}$ a la signification indiquée dans la revendication 6, et

$L^{\ominus}$ a la signification indiquée dans la revendication 3 ;

par condensation du composé intermédiaire de formule (VI) :

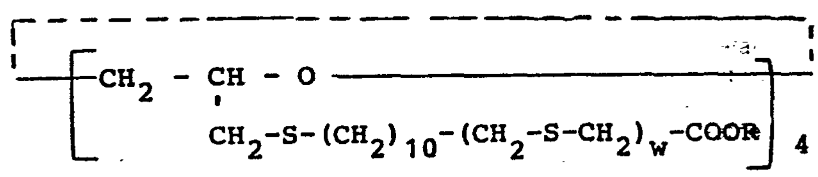

(VI)

où *w* désigne le nombre 0 ou 1 ;

R désigne $CH_3$ ou $C_2H_5$

avec une diamine primaire-tertiaire telle que la diméthylamino éthylamine, la diméthylamino propylamine, la diéthylamino éthylamine, la diéthylamino propylamine, les fonctions amines ainsi obtenues étant éventuellement salifiées, alcoylées et/ou transformées en groupe ammonium, ammonio acétate, ammonio propionate ou ammonio propane sulfonate.

8. Procédé selon la revendication 1, caractérisé par le fait qu'on prépare un polyéther cyclique tensio-actif de formule (I) dans laquelle A désigne le groupement :

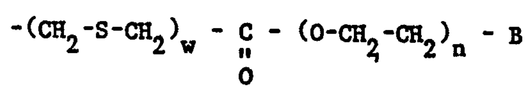

(f)

où *w* désigne le nombre 0 ou 1,

*n* désigne un nombre quelconque de 1 à 10,

B a la signification indiquée dans la revendication 7,

par condensation du composé intermédiaire de formule (VI) figurant dans la revendication 1, avec une diméthylamine oxyéthylénée de formule :

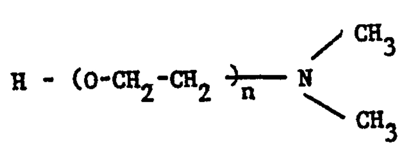

où *n* désigne un nombre quelconque de 1 à 10 ;

la condensation s'effectuant à une température de 20 à 160 °C ; les fonctions amines ainsi obtenues étant éventuellement salifiées, alcoylées et/ou transformées en groupe ammonium, ammonio acétate, ammonio propionate ou ammonio propane sulfonate.

9. Procédé selon la revendication 1, caractérisé par le fait qu'on prépare un polyéther cyclique tensio-actif de formule (I) dans laquelle A désigne le groupement :

$$-[CH_2\text{-}S-CH_2]_w-COOM_1 \qquad (g)$$

où *w* désigne le nombre 0 ou 1,

$M_1$ désigne un groupement ammonium ou un métal alcalin, et de préférence le lithium, le sodium ou le potassium, par saponification de l'ester méthylique ou éthylique du composé intermédiaire de formule (VI), figurant dans la revendication 1, suivie de neutralisation avec un hydroxyde alcalin ou l'ammoniaque, ou lorsque *w* désigne zéro, directement par réaction du tétramère de l'épichlorhydrine ou de l'épibromhydrine avec l'acide mercapto-11 undécanoïque-1, en présence de méthylate ou d'éthylate de sodium ou de potassium, suivie de neutralisation avec un hydroxyde alcalin ou l'ammoniaque.

10. Procédé selon la revendication 1, caractérisé par le fait qu'on prépare un polyéther cyclique tensio-actif de formule (I) dans laquelle A désigne le groupement :

$$-CH_2-O-SO_3M \qquad (h)$$

où M désigne un atome d'hydrogène, un groupement ammonium ou un métal alcalin, par sulfatation du composé intermédiaire de formule (V), figurant dans la revendication 1, avec la chlorhydrine sulfurique et neutralisation éventuelle avec un hydroxyde alcalin ou l'ammoniaque.

11. Procédé selon la revendication 1, caractérisé par le fait qu'on prépare un polyéther cyclique tensio-actif de formule (I) dans laquelle A désigne le groupement :

$$-CH_2-S-[Y-O]_n-H \qquad (i)$$

où *n* désigne un nombre quelconque de 1 à 10,

Y désigne le rédical éthylène ou hydroxypropylène, par réaction du mercapto-éthanol ou mercapto-glycérol avec le mésylate ou tosylate du composé intermédiaire de formule (V) (la formule (V) figurant dans la revendication 1), ou avec un composé halogéné du composé intermédiaire de formule (V), le composé résultant pouvant être condensé avec 1 à 10 moles d'oxyde d'éthylène et/ou de glycidol.

12. Procédé selon la revendication 1, caractérisé par le fait qu'on prépare un polyéther cyclique tensio-actif de formule (I) dans laquelle A désigne le groupement :

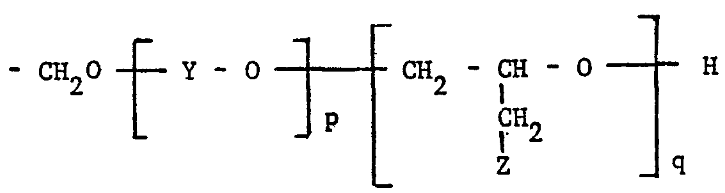 (j)

où p et q, identiques ou différents, désignent un nombre quelconque de 0 à 10, p et q ne désignant pas simultanément zéro,

Y désigne le radical éthylène ou hydroxypropylène,

Z désigne l'un des groupements :

— OH ; $-S-CH_2-CH_2OH$ ; $-S-CH_2-CHOH-CH_2OH$ ;

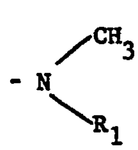 ; 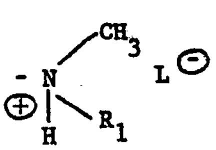 ; 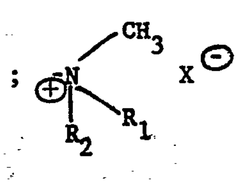 ; 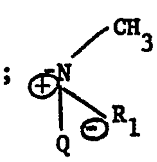

$-S-CH_2-COOM$ ; $-SO_3M$ ;

de plus, quand p = 0 ou quand Y désigne le groupement éthylène, Z en outre désigne l'un des groupements :

$-OSO_3M$ ou $-OCO-CH_2-SO_3M$

où $R_1$ désigne $CH_3$ ou $-[CH_2-CH_2-O]_n-H$

*n* désigne un nombre quelconque de 1 à 10,

$L^{\ominus}$ désigne un anion d'acide organique ou minéral,

$X^{\ominus}$ désigne un anion chlorure, bromure, iodure, méthylsulfate, mésylate ou tosylate,

M désigne un atome d'hydrogène, un groupement ammonium, ou un métal alcalin, par réaction de polyaddition sur le composé intermédiaire de formule (V), figurant dans la revendication 1, de réactifs à fonction époxyde choisis parmi l'oxyde d'éthylène, le tertiobutylglycidyléther (TBGE), l'apichlorhydrine, l'épibromhydrine et leurs mélanges, les groupements protecteurs tertiobutoxy étant remplacés par des groupements hydroxyles, les atomes d'halogène provenant d'une épihalohydrine étant remplacés par un groupement hydroxyle (par réaction avec l'acétate de sodium ou de potassium, suivie de saponification ou alcoolyse de l'ester acétique formé) ou par un groupement thio-hydroxyéthyle, thio-dihydroxypropyle ou thioglycolate, par réaction avec le thioéthanol, le tthioglycérol ou le thioglycolate de méthyle ou d'éthyle ou par un groupement amine par réaction avec la diméthylamine ou la méthyléthanolamine ; lorsqu'on utilise la méthyléthanolamine le composé obtenu peut éventuellement être amené à réagir avec l'oxyde d'éthylène, les fonctions amine ainsi obtenues pouvant être salifiées et transformées en fonction ammonium, ammonio acétate, ammonio propionate ou ammonio propane sulfonate ; les groupements hydroxyles obtenus pouvant éventuellement être estérifiés et remplacés par des groupements sulfate ou sulfoacétate, respectivement par réaction avec la chlorhydrine sulfurique ou l'acide sulfoacétique.

13. Composition cosmétique destinée aux soins des cheveux, sous forme de shampooing, de shampooing colorant, de composition de conditionnement des cheveux ou de composition tinctoriale, se présentant sous la forme d'une solution aqueuse ou hydroalcoolique ou sous la forme d'une crème, d'un gel, d'une émulsion ou d'un aérosol, pouvant également renfermer des adjuvants, notamment des tensio-actifs anioniques, cationiques, amphotères, zwitterioniques ou non-ioniques et leurs mélanges, les parfums, les colorants, les conservateurs, les stabilisateurs de mousse, les agents adoucissants, les agents de restructuration des cheveux, les agents antipelliculaires, les résines cosmétiques, les séquestrants, les épaississants, les synergistes de mousse, les électrolytes, caractérisée par le fait qu'elle renferme au moins $0{,}5.10^{-2}$ gramme/litre d'au moins un composé de formule (I)

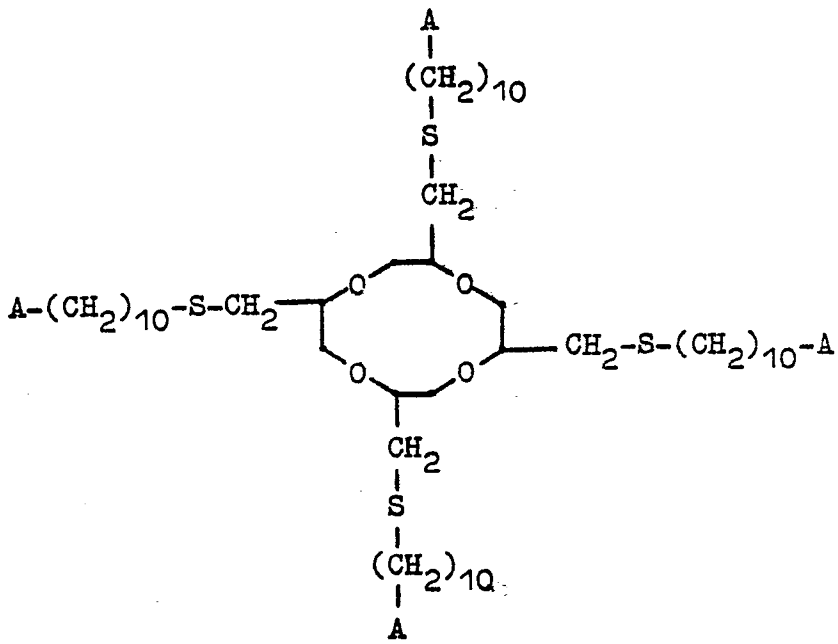

(I)

où A est choisi parmi les groupements suivants ;

a) le groupement

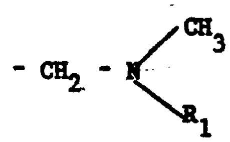

où $R_2$ désigne $CH_3$ ou $—[CH_2—CH_2—O]_n—H$

où *n* désigne un nombre quelconque de 1 à 10

b) le groupement :

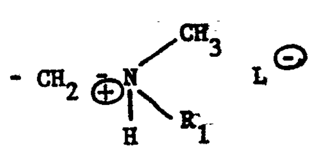

où $L^{\ominus}$ désigne un anion d'acide organique ou minéral

$R_1$ a la signification indiquée pour (a)

c) le groupement

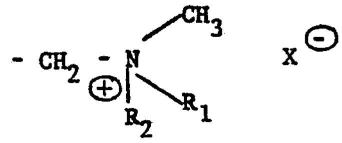

où $R_2$ désigne un radical alkyle ou hydroxyalkyle ayant de 1 à 3 atomes de carbone ;

$X^{\ominus}$ désigne un anion et de préférence un anion chlorure, bromure, iodure, méthyl sulfate, mésylate ou tosylate

$R_1$ a la signification indiquée sous (a)

d) le groupement

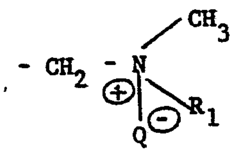

où $Q^{\ominus}$ désigne l'un des groupements $—CH_2—COO^{\ominus}$, $—CH_2—CH_2—COO^{\ominus}$ ou $CH_2—CH_2—CH_2—SO_3^{\ominus}$ ;

$R_1$ a la signification indiquée pour (a)

e) le groupement

$$—(CH_2—S—CH_2)_w—CONH—(CH_2)_m—B$$

où *m* désigne 2 ou 3,

*w* désigne le nombre 0 ou 1,

B désigne l'un des groupements

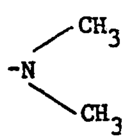 , 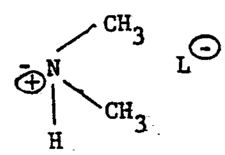 , 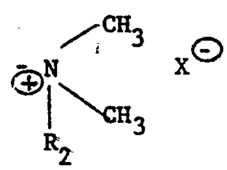 , 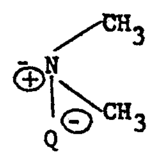

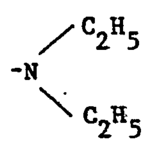 , 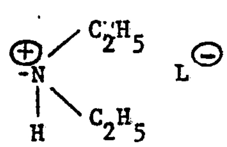 , 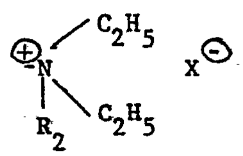 et 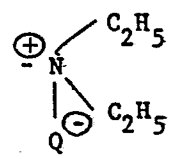

dans lesquels

$L^{\ominus}$ a la signification indiquée pour (b),

$R_2$ et $X^{\ominus}$ ont la signification indiquée pour (c),

$Q^{\ominus}$ a la signification indiquée pour (d) ;

f) le groupement

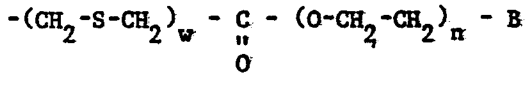

où *w* désigne le nombre 0 ou 1,

*n* désigne un nombre quelconque de 1 à 10,

B a la signification indiquée pour (e) ;

g) le groupement

$$—[CH_2—S—CH_2]_w—COOM_1$$

où *w* désigne le nombre 0 ou 1,

$M_1$ désigne un groupement ammonium ou un métal alcalin ;
h) le groupement

$$-CH_2-O-SO_3M$$

où M désigne un atome d'hydrogène, un groupement ammonium ou un métal alcalin ;
i) le groupement

$$-CH_2-S-[Y-O]_n-H$$

où $n$ désigne un nombre quelconque de 1 à 10,
Y désigne le radical éthylène ou hydroxypropylène ;
j) le groupement

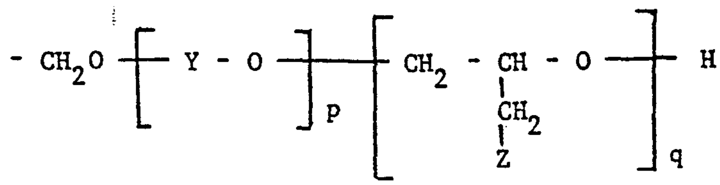

où p et q, identiques ou différents, désignent un nombre quelconque de 0 à 10, cependant p et q ne désignent pas simultanément zéro,
Y a la signification indiquée sous (i),
Z désigne l'un des groupements suivants :

$-OH$ ; $-S-CH_2-CH_2OH$ ; $-S-CH_2-CHOH-CH_2OH$ ;

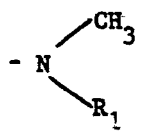 ; 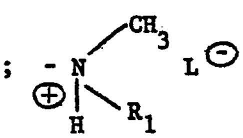 ; 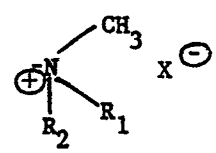 ; 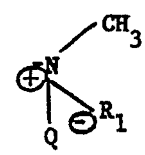

$-S-CH_2-COOM$ ; $-SO_3M$ ;

de plus, quand p désigne zéro ou quand Y désigne éthylène, Z désigne également $-O-SO_3-M$ ou $-OCO-CH_2-SO_3M$.

Dans ces groupements :

$R_1$ désigne $CH_3$ ou $[CH_2-CH_2-O]_n-H$, où $n$ désigne un nombre quelconque de 1 à 10, $L^\ominus$ désigne un anion d'un acide organique ou minéral,
$R_2$ désigne un radical méthyle, hydroxyéthyle ou dihydroxypropyle,
$X^\ominus$ désigne un anion,
$Q^\ominus$ désigne $-OH_2-COO^\ominus$, $-CH_2-CH_2-COO^\ominus$ ou $CH_2-CH_2-CH_2-SO_3^\ominus$,
M désigne un atome d'hydrogène, un groupement ammonium ou un métal alcalin.

14. Procédé de traitement des cheveux consistant à appliquer sur les cheveux humains une quantité efficace d'une composition selon la revendication 13, laisser au contact des cheveux pendant une durée suffisante, puis rincer, éventuellement appliquer un shampooing et rincer à nouveau.

15. Procédé de nettoyage de cheveux humides, consistant à appliquer sur une chevelure préalablement mouillée, une quantité suffisante de composition selon la revendication 13, masser pour émulsionner les salissures puis rincer.

**Claims** (for the contracting States : BE, CH, DE, FR, GB, IT, LU, NL, SE)

1. Surface-active cyclic polyethers of the general formula :

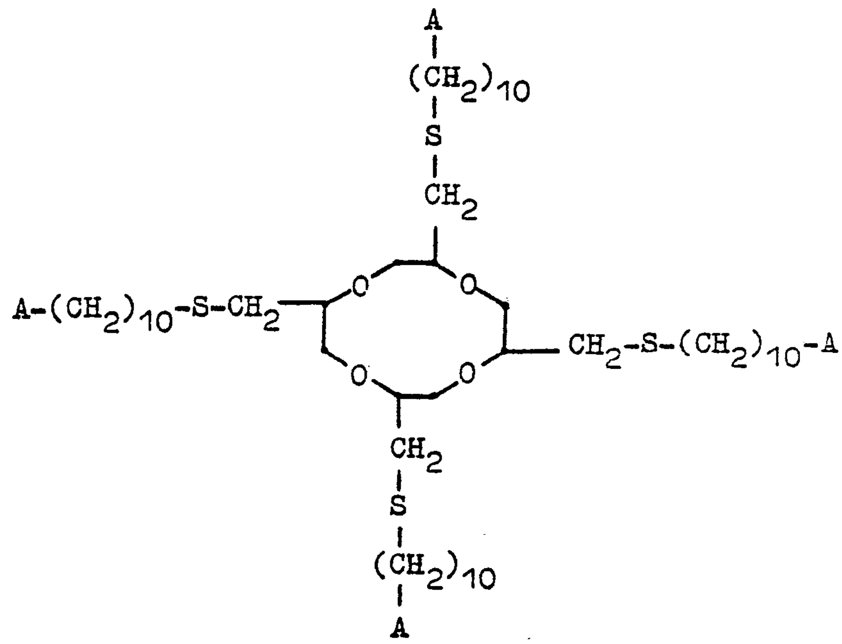

(I)

in which A denotes a cationic, anionic, zwitterionic or non-ionic hydrophilic unit containing at least one group chosen from amongst amine, ammonium, ammonioalkylcarboxylate and ammonioalkylsulphonate groups, the group

$$—[CH_2—S—CH_2]_w—COOM_1 \quad (g)$$

in which *w* denotes the number 0 or 1 and $M_1$ denotes an alkali metal, the group

$$—CH_2—O—SO_3M \quad (h)$$

in which M denotes a hydrogen atom or an alkali metal, the group

$$—CH_2—S—[Y—O]_n—H \quad (i)$$

in which *n* denotes any number from 1 to 0 and Y denotes the ethylene or hydroxypropylene radical, and the group

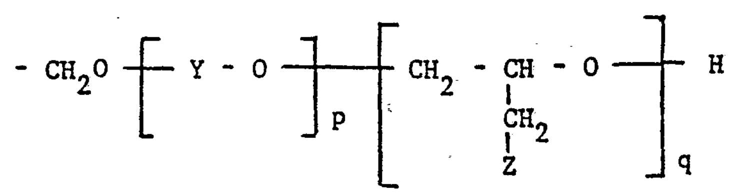

(j)

in which p and q, which are identical or different, denote any number from 0 to 10, but p and q do not simultaneously denote zero, Y has the meaning indicated under (i), Z denotes one of the following groups : —OH, $—S—CH_2—CH_2OH$, $—S—CH_2—CHOH—CH_2OH$, $—S—CH_2—COOM$ and $—SO_3M$, and, if p denotes zero or if Y denotes ethylene, Z also denotes $—O—SO_3—M$ or $—OCO—CH_2—SO_3M$, and M denotes a hydrogen atom or an alkali metal.

2. Surface-active cyclic polyether according to Claim 1, characterised in that the hydrophilic group A containing amine, ammonium, ammonioalkylcarboxylate and/or ammonioalkylsulphonate groups is chosen from amongst the following : the group

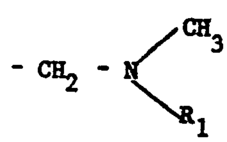

(a)

in which $R_1$ denotes $CH_3$ or $—[CH_2—CH_2—O]_n—H$, in which *n* denotes any number from 1 to 10, the group

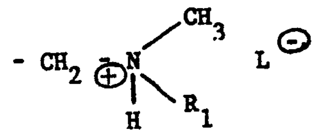

(b)

in which $L^{\ominus}$ denotes an organic or mineral acid anion and $R_1$ has the meaning indicated for (a), the group

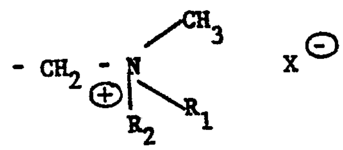

(c)

in which $R_2$ denotes an alkyl or hydroxyalkyl radical having from 1 to 3 carbon atoms, $X^{\ominus}$ denotes an anion and preferably a chloride, bromide, iodide, methyl-sulphate, mesylate or tosylate anion and $R_1$ has the meaning indicated under (a), the group

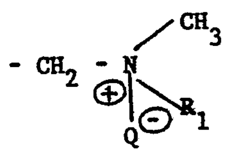

(d)

in which $Q^{\ominus}$ denotes one of the groups $—CH_2—COO^{\ominus}$, $—CH_2—CH_2—COO^{\ominus}$ or $—CH_2—CH_2—CH_2—SO_3^{\ominus}$, and $R_2$ has the meaning indicated for (a), the group

$$—(CH_2—S—CH_2)_w—CONH—(CH_2)_m—B \qquad (e)$$

in which *m* denotes 2 or 3, *w* denotes the number 0 or 1 and B denotes one of the groups

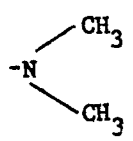 , 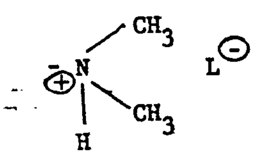 , 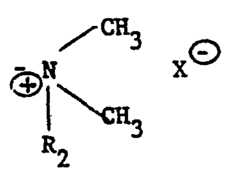 , 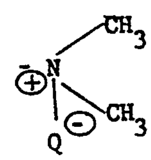

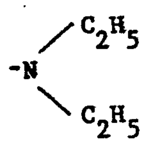 , 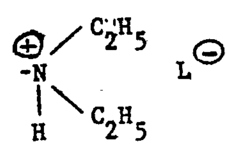 , 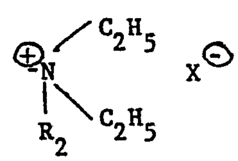 and 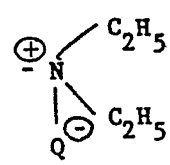

in which $L^{\ominus}$ has the meaning indicated for (b), $R_2$ and $X^{\ominus}$ have the meaning indicated for (c) and $Q^{\ominus}$ has the meaning indicated for (d), the group

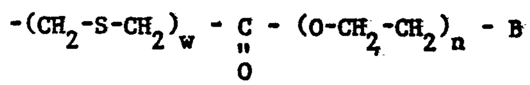

(f)

in which *w* denotes the number 0 or 1, *n* denotes any number from 1 to 10 and B has the meaning indicated for (e), the group

$$—[CH_2—S—CH_2]_w—COOM_1 \qquad (g)$$

in which *w* denotes the number 0 or 1 and $M_1$ denotes an ammonium group, the group

$$—CH_2—O—SO_3M \qquad (h')$$

in which M denotes an ammonium group, and the group

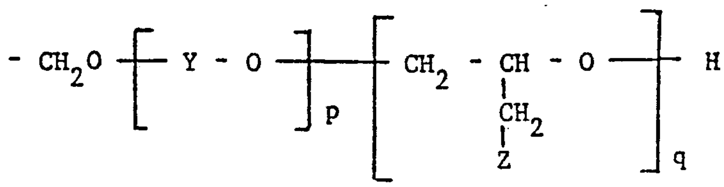

(j')

in which p and q, which are identical or different, denote any number from 0 to 10, but p and q do not simultaneously denote zero, Y denotes the ethylene or hydroxypropylene radical and Z denotes one of the following groups :

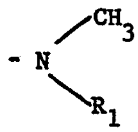 ; 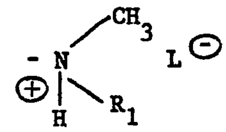 ; 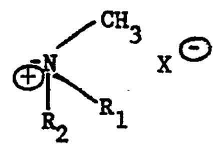 ; 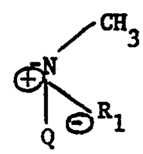

$—S—CH_2—COOM$ and $—SO_3M$,

and, if p denotes zero or if Y denotes ethylene, Z also denotes $—O—SO_3—M$ or $—OCO—CH_2—SO_3M$ ; in these groups : $R_1$ denotes $CH_3$ or $—[CH_2—CH_2—O]_n—H$, in which *n* denotes any number from 1 to 10, $L^{\ominus}$ denotes an organic or mineral acid anion, $R_2$ denotes a methyl, hydroxyethyl or dihydroxypropyl radical, $X^{\ominus}$ denotes an anion, $Q^{\ominus}$ denotes $—CH_2—COO^-$, $—CH_2—CH_2—CH_2—COO^-$ or $CH_2—CH_2—CH_2—SO_3^-$, and M denotes an ammonium group.

3. Intermediates for the preparation of the compounds of the formula (I) according to Claim 1, corresponding to the formula :

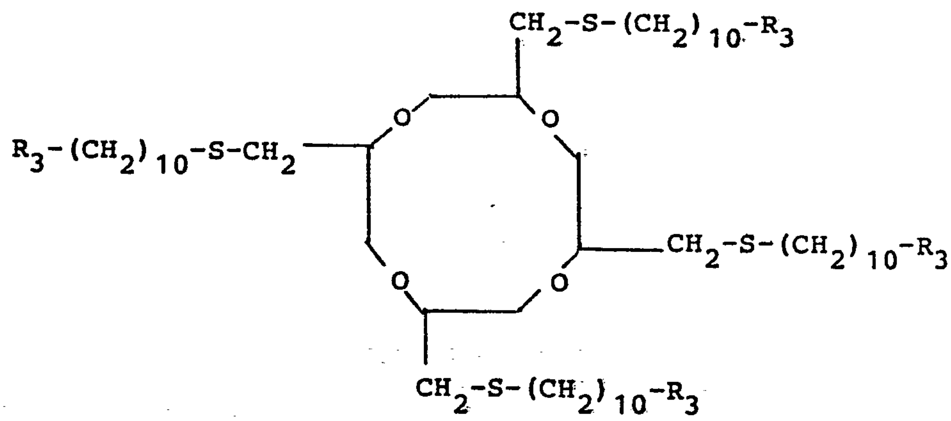

(VII)

in which $R_3$ denotes $—CH_2OH$, $—CH_2—O—SO_2—CH_3$, $—CH_2—O—SO_2—C_6H_4—CH_3$, $—COOH$, $—COOCH_3$, $F—COOC_2H_5$, $CH_2—S—CH_2—COOH$, $—CH_2—S—CH_2—COOCH_3$ or $—CH_2—S—CH_2—COOC_2H_5$.

4. Process for the preparation of the intermediates of the formula (VII), according to Claim 3, characterised in that (1) thioacetic acid is added to undecylenyl alcohol or undecylenic acid, (2) the reaction product prepared under (1) is saponified in order to obtain 11-mercaptoundecanol or 11-mercaptoundecan-1-oic acid, respectively, (3) the 11-mercaptoundecan-1-oic acid is esterified, if appropriate, to the methyl or ethyl ester, and (4) the compound obtained under (2) or (3) is condensed with the tetramer of epichlorohydrin or of epibromohydrin, or alternatively methyl or ethyl thioglycolate is condensed with the halogen derivative, the mesylate or the tosylate of the compound obtained by reacting 11-mercaptoundecan-1-ol with the tetramer of epichlorohydrin or of epibromohydrin.

5. Process for the preparation of a surface-active polypodous cyclic polyether of the formula (I) in which A denotes the group :

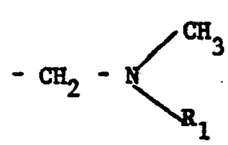

(a)

in which $R_1$ denotes $CH_3$ or $—[CH_2—CH_2—O]_n—H$, *n* denoting any number from 1 to 10, by reacting dimethylamine or methylethanolamine with the chlorine or bromine derivative of the intermediate of the formula (V)

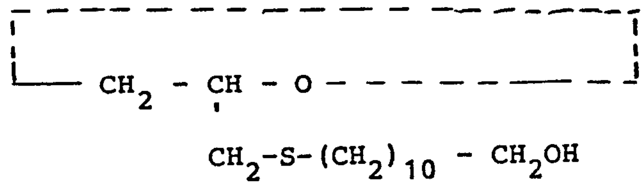

(V)

obtained by reacting thionyl chloride or hydrobromic acid with this intermediate (V), or with the mesylate or tosylate of the intermediate of the formula (V), at a temperature from 20 to 160 °C, at ordinary pressure or in an autoclave ; if the amine used is methylethanolamine, the compound obtained is oxyethyleneated, if appropriate, with 1 to 10 mols of ethylene oxide.

6. Process for the preparation of a polypodous surface-active cyclic polyether of the formula (I) in which A denotes the group :

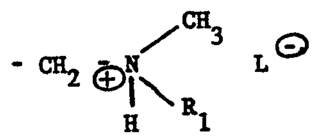

(b)

in which $R_1$ denotes $CH_3$ or $—[CH_2—CH_2—O]_n—H$, in which *n* denotes any number from 1 to 10, and $L^{\ominus}$ denotes an organic or inorganic anion and preferably a chloride, bromide, sulphate, phosphate, acetate, glycolate, lactate, tartrate, methanesulphonate or para-toluenesulphonate, by salification with an acid of the formula HL, in which L has the meaning indicated above.

7. Process for the preparation of a polypodous surface-active cyclic polyether of the formula (I) in which A denotes the group :

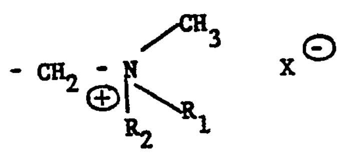

(c)

in which $R_2$ denotes a methyl, hydroxyethyl or dihydroxypropyl radical, $X^{\ominus}$ denotes an anion and preferably a chloride, bromide, iodide, methyl-sulphate, mesylate or tosylate anion and $R_1$ denotes $CH_3$ or $—[CH_2—CH_2—O]_n—H$, in which *n* denotes any number from 1 to 10, by alkylating a compound 1 (a), according to Claim 2, with an alkylating agent preferably chosen from amongst methyl chloride, bromide, iodide, sulphate, mesylate or tosylate, glycol chlorohydrin or glycerol chlorohydrin.

8. Process according to Claim 7, characterised in that $X^-$ denotes a mesylate or tosylate anion and that the mesylate or tosylate of the intermediate of the formula (V)

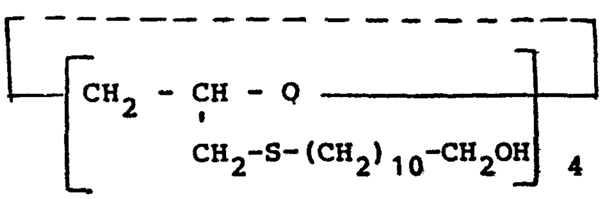

(V)

is reacted with an amine of the formula :

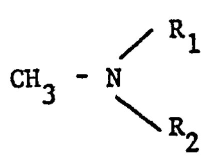

in which $R_1$ denotes $CH_3$ OR $—[CH_2—CH_2—O]_n—H$, *n* denoting any number from 1 to 10, and $R_2$ denotes a methyl, hydroxyethyl or dihydroxypropyl radical, at a temperature between 20 and 100 °C.

9. Process for the preparation of a polypodous surface-active cyclic polyether of the formula (I) in which A denotes the group :

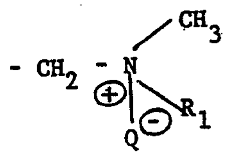

(d)

in which $Q^{\ominus}$ denotes one of the groups : $—CH_2COO^{\ominus}$, $—CH_2—CH_2—COO^{\ominus}$ and $—CH_2—CH_2—CH_2—SO_3^{\ominus}$, and $R_1$ denotes $CH_3$ or $—[CH_2—CH_2—O]_n—H$, in which *n* denotes any number from 1 to 10, by alkylating the compounds of the formula (I) in which A denotes the group (a), with sodium chloroacetate or chloropropionate or propanesultone.

10. Process for the preparation of a polypodous surface-active cyclic polyether of the formula (I) in which A denotes the group :

$$—(CH_2—S—CH_2)_w—CONH—(CH_2)_m—B \qquad (e)$$

in which *m* denotes the number 2 or 3, *w* denotes the number 0 or 1 and B denotes one of the groups :

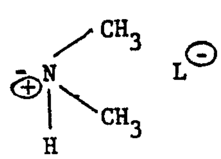 , 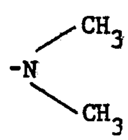 , 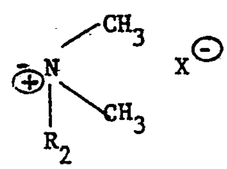 , 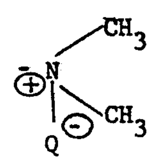

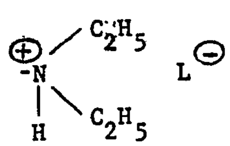 , 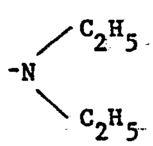 , 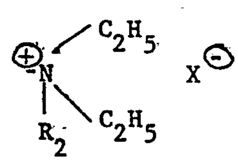 and 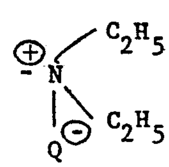

in which $R_2$ and $X^{\ominus}$ have the meaning indicated in Claim 7, $Q^{\ominus}$ has the meaning indicated in Claim 9 and $L^{\ominus}$ has the meaning indicated in Claim 6, by condensing the intermediate of the formula (VI) :

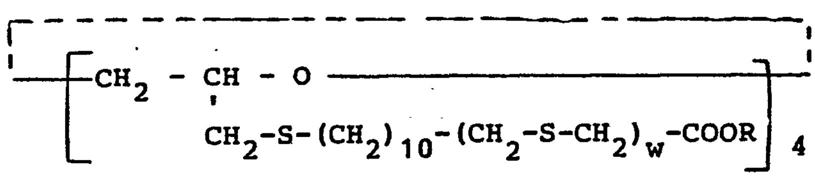

(VI)

in which *w* denotes the number 0 or 1 and R denotes $CH_3$ or $C_2H_5$, with dimethylaminoethylamine, dimethylaminopropylamine, diethylaminoethylamine or diethylaminopropylamine, the amine groups thus obtained optionally being salified or alkylated and/or converted to ammonium, ammonioacetate, ammoniopropionate or ammoniopropanesulphonate groups.

11. Process for the preparation of a polypodous surface-active cyclic polyether of the formula (I) in which A denotes the group :

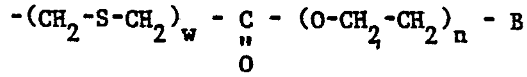 (f)

in which *w* denotes the number 0 or 1, *n* denotes any number from 1 to 10 and B has the meaning indicated in Claim 10, by condensing the intermediate of the formula (VI) shown in Claim 10, with an oxyethyleneated dimethylamine of the formula :

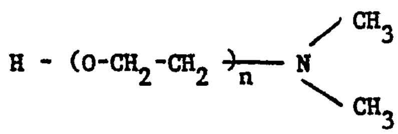

in which *n* denotes any number from 1 to 10, the condensation being carried out at a temperature from 20 to 160 °C and the amine group optionally being salified or alkylated and/or converted to an ammonium, ammonioacetate, ammoniopropionate or ammoniopropanesulfonate group.

12. Process for the preparation of a polypodous surface-active cyclic polyether of the formula (I) in which A denotes the group :

$$—[CH_2—S—CH_2]_w—COOM_1 \quad (g)$$

in which *w* denotes the number 0 or 1 and $M_1$ denotes an ammonium group or an alkali metal and preferably lithium, sodium or potassium, by saponification of the methyl or ethyl ester of the intermediate of the formula (VI) shown in Claim 10, followed by neutralisation with an alkali metal hydroxide or ammonia, or, if *w* denotes zero, directly by reaction of the tetramer of epichlorohydrin or of epibromohydrin with 11-mercaptoundecan-1-oic acid, in the presence of sodium methylate or ethylate or potassium methylate or ethylate, followed by neutralisation with an alkali metal hydroxide or ammonia.

13. Process for the preparation of a polypodous surface-active cyclic polyether of the formula (I) in which A denotes the group :

$$—CH_2—O—SO_3M \quad (h)$$

in which M denotes a hydrogen atom, an ammonium group or an alkali metal, by sulphation of the intermediate of the formula (V) shown in Claim 5, with chlorosulphonic acid and neutralisation, if appropriate, with an alkali metal hydroxide or ammonia.

14. Process for the preparation of a polypodous surface-active cyclic polyether of the formula (I) in which A denotes the group :

$$—CH_2—S—[Y—O]_n—H \quad (i)$$

in which *n* denotes any number from 1 to 10 and Y denotes the ethylene or hydroxypropylene radical, by reacting mercaptoethanol or mercaptoglycerol with the mesylate or tosylate of the intermediate of the formula (V), or with a halogen compound of the intermediate of the formula (V), shown in Claim 5, it being possible for the resulting compound to be condensed with 1 to 10 mols of ethylene oxide and/or of glycidol.

15. Process for the preparation of a polypodous surface-active cyclic polyether of the formula (I) in which A denotes the group :

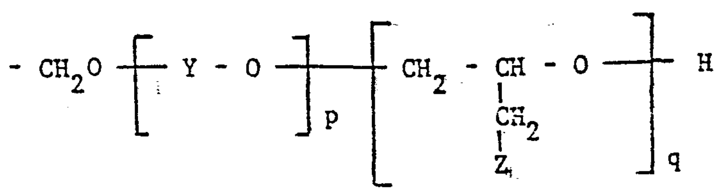 (j)

in which p and q, which are identical or different, denote any number from 0 to 10, p and q not simultaneously denoting zero, Y denotes the ethylene or hydroxypropylene radical and Z denotes one of the groups : —OH, $—S—CH_2—CH_2OH$, $—S—CH_2—CHOH—CH_2OH$,

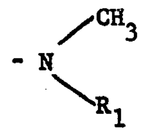 ; 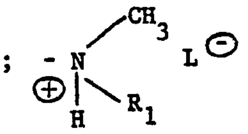 ; 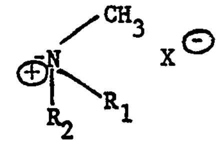 ; 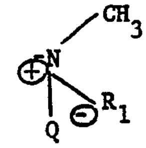

$-S-CH_2-COOM$ and $-SO_3M$,

and, if p = 0 or if Y denotes the ethylene group, Z also denotes one of the groups : $-OSO_3M$ or $-OCO-CH_2-SO_3M$, in which groups $R_1$ denotes $CH_3$ or $-[CH_2-CH_2-O]_n-H$, *n* denotes any number from 1 to 10, $L^{\ominus}$ denotes an organic or mineral acid anion, $X^{\ominus}$ denotes a chloride, bromide, iodide, methylsulphate, mesylate or tosylate anion and M denotes a hydrogen atom, an ammonium group or an alkali metal, by means of a polyaddition reaction, with the intermediate of the formula (V) shown in Claim 5, of reactants containing an epoxide group and chosen from amongst ethylene oxide, tert.-butyl glycidyl ether (TBGE), epichlorohydrin, epibromohydrin and mixtures thereof, the tert.-butoxy protective groups being replaced by hydroxyl groups, and the halogen atoms originating from an epihalogenohydrin being replaced by a hydroxyl group (by reaction with sodium acetate or potassium acetate, followed by saponification or alcoholysis of the acetic acid ester formed) or by a thiohydroxyethyl, thiodihydroxypropyl or thioglycolate group by reaction with thioethanol, thioglycerol or methyl or ethyl thioglycolate, or by an amine group by reaction with dimethylamine or methylethanolamine ; if methylethanolamine is used, the compound obtained can be reacted, if appropriate, with ethylene oxide, it being possible for the amine groups thus obtained to be salified and converted to ammonium, ammonioacetate, ammoniopropionate or ammoniopropanesulphonate groups, and it being possible, if appropriate, for the hydroxyl groups obtained to be esterified and replaced by sulphate or sulphoacetate groups, respectively by reaction with chlorosulphonic acid or sulphoacetic acid.

16. Composition containing at least $0.5.10^{-2}$ gram/litre of a cyclic surface-active compound of the formula (I).

17. Cosmetic or pharmaceutical composition, characterised in that it contains at least $0.5.10^{-2}$ gram/litre of a compound of the formula (I), in the presence of an aqueous or aqueous-alcoholic vehicle.

18. Composition according to Claim 17, characterised in that the aqueous-alcoholic solution contains an alcohol or a polyol having from 1 to 4 carbon atoms.

19. Composition according to Claims 16 or 17, characterised in that it is in the form of a solution, a cream, a gel, an emulsion or an aerosol.

20. Cosmetic composition for treating the hair, containing a cosmetically effective amount of one or more compounds of the formula (I), in solution in a solvent chosen from a group comprising water and an aqueous-alcoholic solution.

21. Shampoo composition for treating the hair, containing a cosmetically effective amount of one or more compounds of the formula (I), in solution in a solvent chosen from a group comprising water and an a aqueous-alcoholic solution.

22. Hair shampoo composition containing a cosmetically effective amount of one or more compounds of the formula (I), in solution in a solvent chosen from the group comprising water and an aqueous-alcoholic solvent, and also containing one or more adjuvants chosen from the group comprising anionic, cationic, amphoteric, zwitterionic and non-ionic surface-active agents, perfumes, dyestuffs, preservatives, thickeners, foam stabilisers, softeners, hair-restructing agents, anti-dandruff agents, cosmetic resins, foam synergistic agents and electrolytes.

23. Process for treating the hair, consisting in applying, to human hair, an effective amount of a composition comprising a cosmetically effective amount of one or more surface-active cyclic polyethers of the formula (I), in solution in a solvent chosen from a group comprising water and an aqueous-alcoholic solution.

24. Hair-dyeing composition containing a cosmetically effective amount of one or more compounds of the formula (I), in solution in a solvent chosen from a group comprising water and an aqueous-alcoholic solution, and also a dyestuff for hair and, if appropriate, one or more adjuvants chosen from the group comprising anionic, cationic, non-ionic, zwitterionic and amphoteric surface-active agents and mixtures thereof, perfumes, preservatives, thickeners, softeners, cosmetic resins and sequestering agents.

25. Composition according to Claim 24, characterised in that the dyestuff is chosen from amongst anthraquinone dyestuffs, azo dyestuffs, nitro dyestuffs of the benzene series, indophenols, indoanilines and indamines.

**Claims** (for the Contracting state AT)

1. Process for the preparation of surface active cyclic polyethers of the formula

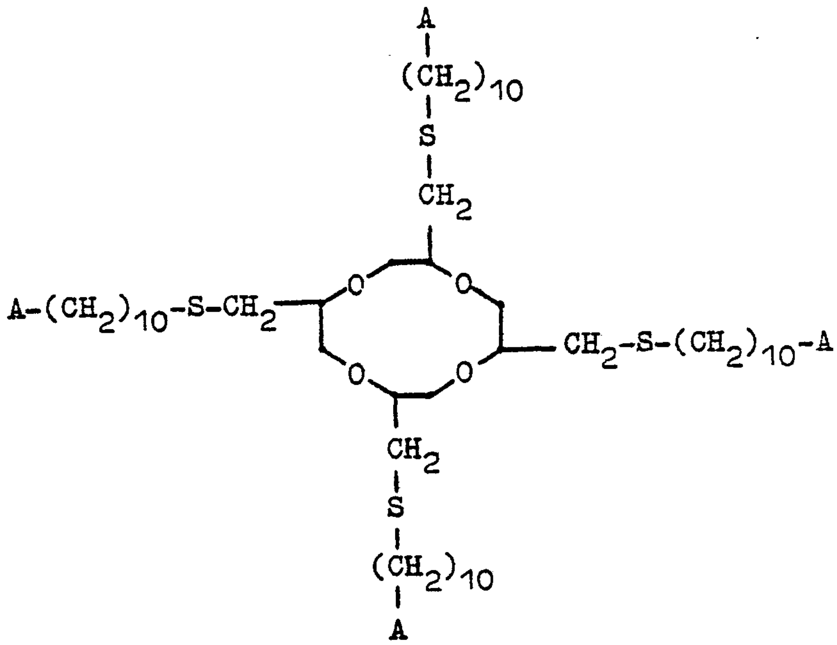

(I)

in which A denotes a cationic, anionic, zwitterionic or non-ionic group, selected from amongst the following :

a) the group

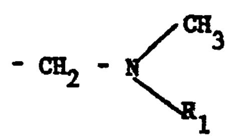

in which $R_1$ denotes $CH_3$ or $-[CH_2-CH_2-O]_n-H$
wherein *n* denotes any number from 1 to 10 ;

b) the group

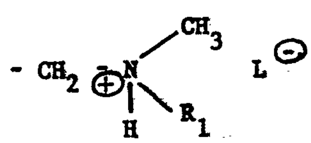

(b)

in which $L^\ominus$ denotes an organic or mineral acid anion and $R_1$ has the meaning indicated for (a),

c) the group

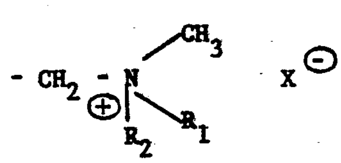

in which $R_2$ denotes an alkyl or hydroxyalkyl radical having from 1 to 3 carbon atoms ; $X^\ominus$ denotes an anion and preferably a chloride, bromide, iodide, methyl-sulphate, mesylate or tosylate anion and $R_1$ has the meaning indicated under (a),

d) the group

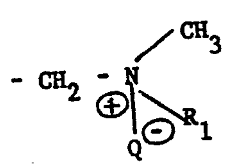

in which $Q^{\ominus}$ denotes one of the groups $—CH_2—COO^{\ominus}$, $—CH_2—CH_2—COO^{\ominus}$ or $—CH_2—CH_2—CH_2—SO_3^{\ominus}$, and $R_2$ has the meaning indicated for (a),

e) the group

$$—(CH_2—S—CH_2)_w—CONH—(CH_2)_m—B$$

in which *m* denotes 2 or 3, *w* denotes the number 0 or 1 and B denotes one of the groups :

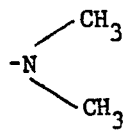 , 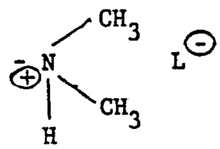 , 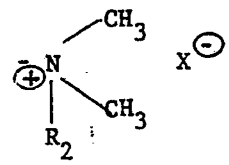 , 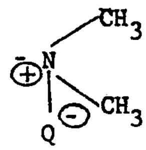

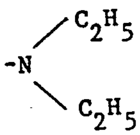 , 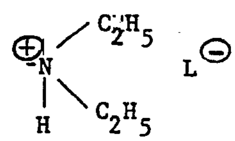 , 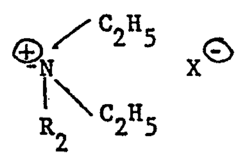 and 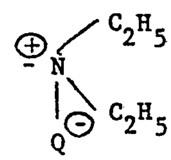

in which $L^{\ominus}$ has the meaning indicated for (b), $R_2$ and $X^{\ominus}$ have the meaning indicated for (c) and $Q^{\ominus}$ has the meaning indicated for (d),

f) the group

$$-(CH_2-S-CH_2)_w-\underset{\underset{O}{\|}}{C}-(O-CH_2-CH_2)_n-B$$

in which *w* denotes the number 0 or 1, *n* denotes any number from 1 to 10 and B has the meaning indicated for (e),

g) the group

$$—[CH_2—S—CH_2]_w—COOM_1$$

in which *w* denotes the number 0 or 1 and $M_1$ denotes an ammonium group or an alkali metal,

h) the group

$$—CH_2—O—SO_3M$$

in which M denotes a hydrogen atom, an ammonium group or an alkali metal,

i) the group

$$—CH_2—S—[Y—O]_n—H$$

in which *n* denotes any number from 1 to 10, Y denotes the ethylene or hydroxypropylene radical ;

j) the group

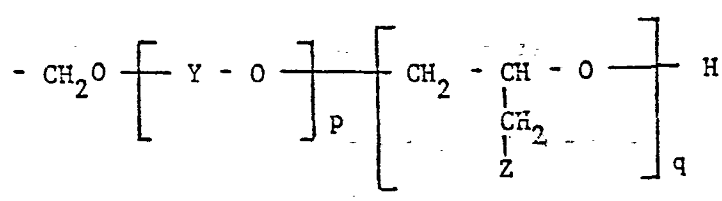

in which p and q, which are identical or different, denote any number from 0 to 10, but p and q do not simultaneously denote zero, Y denotes the ethylene or hydroxypropylene radical and Z denotes one of the following groups :

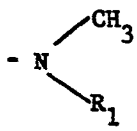 ; 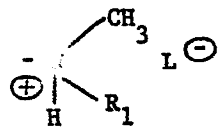 ; 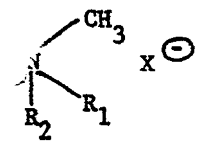 ; 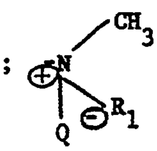

$-S-CH_2-COOM$ and $-SO_3M$,

and, if p denotes zero or if Y denotes ethylene, Z also denotes $-O-SO_3-M$ or $-OCO-CH_2-SO_3M$ ; in these groups : $R_1$ denotes $CH_3$ or $[CH_2-CH_2-O]_n-H$, in which *n* denotes any number from 1 to 10, $L^\ominus$ denotes an organic or mineral acid anion, $R_2$ denotes a methyl, hydroxyethyl or dihydroxypropyl radical, $X^\ominus$ denotes an anion, $Q^\ominus$ denotes $-CH_2-COO^-$, $-CH_2-CH_2-CH_2-COO^-$ or $CH_2-CH_2-CH_2-SO_3^-$, and M denotes a hydrogen atom, an ammonium group or an alkali metal, characterized in that in the first stage one prepares

11-mercapto undecan-1-ol of the formula :

$$HS-(CH_2)_{10}-CH_2OH \qquad (II),$$

or

11-mercapto undecan-1-oic acid of formula :

$$HS-(CH_2)_{10}-COOH \qquad (III),$$

that in the second stage one prepares the intermediate compound of formula :

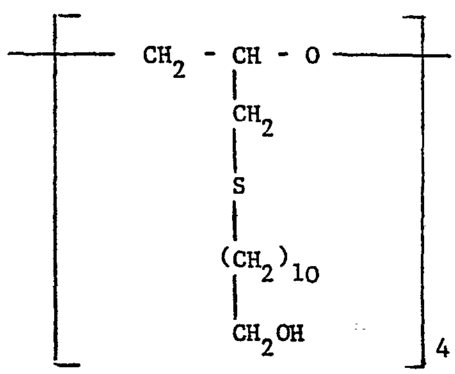 (V)

by reacting 11-mercapto undecan-1-ol with the tetramer of epichlorohydrin or of epibromohydrin of formula (IVa)

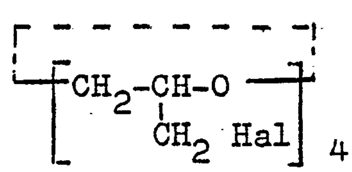 (IVa)

in which Hal denotes chlorine or bromine, (the tetramer of epichlorhydrin or of epibromhydrin being prepared by polymerisation of epichlorohydrin or of epibromohydrin in the presence of a Lewis acid catalyst) ;

that in a third stage one prepares the intermediate compound of formula (VI)

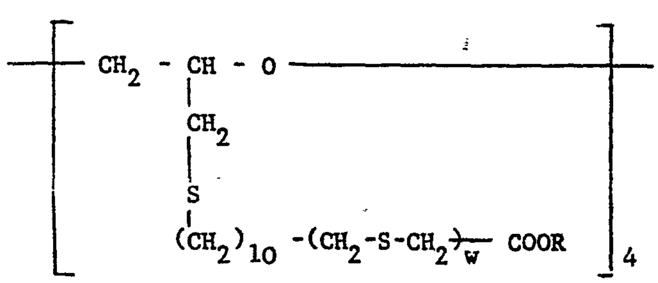 (VI)

in which R denotes hydrogen, $CH_3$ or $C_2H_5$, *w* denotes the number 0 or 1, by reacting either 11-mercapto undecan-1-oic acid, its methyl or ethyl ester with the tetramer of epichlorohydrin or of epibromohydrin or alternatively methyl or ethyl thioglycolate is reacted with the halogen derivative, the mesylate or the tosylate of the intermediate of the formula (V), further one prepares :

a) compounds Ia having formula (I) in which A denotes the group (a) by reacting dimethylamine or methylethanolamine with the chlorine or bromine derivative or with mesylate or tosylate of the intermedaite of the formula (V)

b) compounds Ib having formula (I) in which A denotes the group (b), by salification of the corresponding compounds with a mineral or organic acid ;

c) compounds Ic having formula (I) in which A denotes the group (c), by alkylating corresponding compounds Ia or directly by reacting the mesylate or tosylate of the intermediate of formula (V) with an amine of formula :

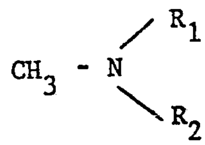

d) compounds Id, having formula (I) in which A denotes the group (d) by alkylating compounds Ia with sodium chloracetate or chloropropionate or propanesultone ;

e) compounds Ie, having formula (I) in which A denotes the group (e), by condensation of intermediate of the formula (VI) with dimethylamino ethylamine, dimethylamino propylamine, diethylamino ethylamine or diethylamino propylamine ;

f) compounds If, having formula (I) in which A denotes the group (f), by condensation of the intermediate of formula (VI) with an oxyethylenated dimethylamine of formula :

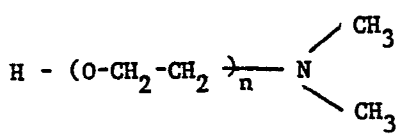

in which *n* denotes any number from 1 to 10, the amine group thus obtained optionally being salified with a mineral or organic acid or alkylated with an alkylating agent ;

g) compounds Ig having formula (I) in which A denotes the group (g), by saponification of the methyl or ethyl ester of the intermediate of the formule (VI), followed by neutralisation with an alkali metal hydroxide or ammonia, or, if *w* denotes zero, directly by reaction of the tetramer of epichlorhydrin or of epibromhydrin with 11-mercaptoundecan-1-oic acid, in the presence of sodium methylate or ethylate or potassium methylate or ethylate, followed by neutralisation with an alkali metal hydroxide or ammonia ;

h) compounds Ih, having formula (I) in which A denotes the group (h), by sulphation of the intermediate of the formula (V), with chlorosulphonic acid, an neutralisation, if appropriate, with an alkali metal hydroxide or ammonia ;

i) compounds Ii having formula (I) in which A denotes group (i), by reacting mercaptoethanol or mercaptoglycerol with the mesylate or tosylate of the intermediate of the formula (V), or with a halogen compound of the intermediate of the formula (V), it being possible for the resulting compound to be condensed with 1 to 10 mols of ethylene oxide and/or of glycerol, in the presence of an alkaline catalyst ;

j) compounds Ij having formula (I) in which A denotes (j) by means of a polyaddition reaction, with the intermediaite of the formula (V), of reactants containing an epoxide group and chosen from amongst ethylene oxide, tert.-butyl glycidylether (TBGE), epichlorohydrin, epibromohydrin and mixtures thereof, the tert.-butoxy protective groups being replaced by hydroxyl groups, and the halogen atoms originating from an epihalogenohydrin being replaced by a hydroxyl group (by reaction with sodium acetate or potassium acetate, followed by saponification or alcoholysis of the acetic acid ester formed) or by a thiohydroxyethyl, thiodihydroxypropyl or thioglycolate group by reaction with thioethanol, thioglycerol or methyl or ethyl thioglycolate, or by an amine group by reaction with dimethylamine or methylethanolamine ; if methylethanolamine is used, the compound obtained can be reacted, if appropriate, with ethylene oxide, it being possible for the amine groups thus obtained to be salified and converted to ammonium, ammonioacetate, ammoniopropionate or ammoniopropanesulphonate groups, and it being possible, if appropriate, for the hydroxyl groups obtained to be esterified and replaced by sulphate or sulphoacetate groups, respectively by reaction with chlorosulphonic acid or sulphoacetic acid.

2. Process of preparation according to claim 1, characterized that one prepares a surface-active cyclic polyether of the formula (I) in which A denotes the group :

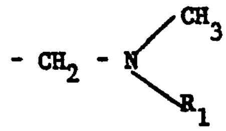

(a)

in which $R_1$ denotes $CH_3$ or $-[CH_2-CH_2-O]_n-H$, *n* denotes any number from 1 to 10, by reacting dimethylamine or methylethanolamine with the chlorine or bromine derivative of the intermediate of the formula (V) (which formula (V) is represented in claim 1, or with the mesylate or tosylate of the intermediate of the formula (V), at a temperature from 20 to 160 °C, at ordinary pressure or in an autoclave ; if the amine used is methylethanolamine, the compound obtained is oxyethylenated, if appropriate, with 1 to 10 mols of ethylene oxide.

3. Process according to claim 1, characterized that one prepares a surface-active cyclic polyether of the formula (I) in which A denotes the group :

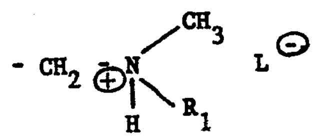

(b)

in which $R_1$ has the meaning indicated in claim 2 and $L^{\ominus}$ denotes an organic or inorganic anion and preferably a chloride, bromide, sulphate, phosphate, acetate, glycolate, lactate, tartrate, methanesulphonate or para-toluenesulphonate, by salification with an acid of the formula HL, in which L has the meaning indicated above.

4. Process according to claim 1, characterized that one prepares a surface-active cyclic polyether of the formula (I) in which A denotes the group :

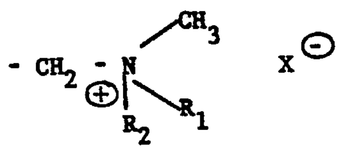

(c)

in which $R_2$ denotes a methyl, hydroxyethyl or dihydroxypropyl radical, $X^{\ominus}$ denotes an anion and preferably a chloride, bromide, iodide, methyl-sulphate, mesylate or tosylate anion and $R_1$ has the meaning indicated in claim 2, by alkylating a compound I (a), according to claim 2, with an alkylating agent preferably chosen from amongst methyl chloride, bromide, iodide, sulphate, mesylate or tosylate, glycol chlorohydrin or glycerol chlorohydrin.

5. Process according to claim 4, characterized in that $X^{\ominus}$ denotes a mesylate or tosylate anion and that the mesylate or tosylate of the intermediate of the formula (V)

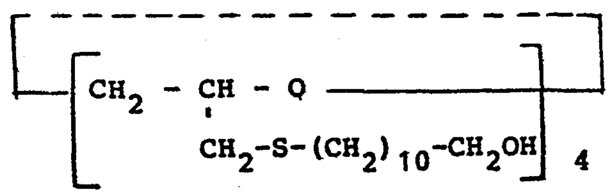

(V)

is reacted with an amine of the formula :

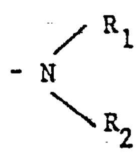

in which $R_1$ denotes $CH_3$ or $-[CH_2-CH_2-O]_n-H$, *n* denoting any number from 1 to 10, and $R_2$ denotes a methyl, hydroxyethyl or dihydroxypropyl radical, at a temperature between 20 and 100 °C.

6. Process according to claim 1, characterized in that one prepares a surface-active cyclic polyether of the formula (I) in which A denotes the group :

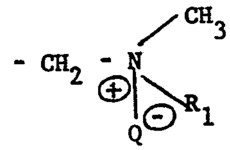

(d)

in which $Q^{\ominus}$ denotes one of the groups: $-CH_2-COO^{\ominus}$, $-CH_2-CH_2-COO^{\ominus}$ and $-CH_2-CH_2-CH_2-SO_3^{\ominus}$, and $R_1$ has the meaning indicated in claim 2, by alkylating the compounds of the formula (I) in which A denotes the group (a), with sodium chloroacetate or chloropropionate or propanesultone.

7. Process according to claim 1, characterized in that one prepares a surface-active cyclic polyether of the formula (I) in which A denotes the group:

$$-(CH_2-S-CH_2)_w-CONH-(CH_2)_m-B \qquad (e)$$

in which *m* denotes the number 2 or 3, *w* denotes the number 0 or 1 and B denotes one of the groups:

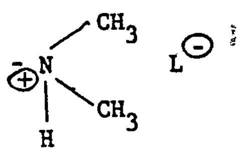 , 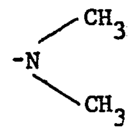 , 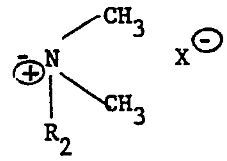 , 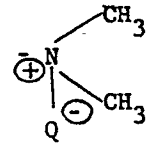

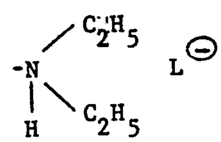 , 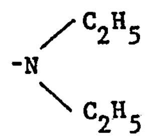 , 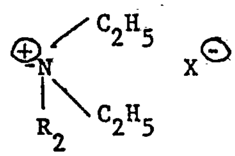 and 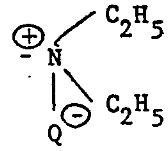

in which $R_2$ and $X^{\ominus}$ have the meaning indicated in claim 4, $Q^{\ominus}$ has the meaning indicated in claim 6 and $L^-$ has the meaning indicated in claim 3, by condensing the intermediate of the formula (VI):

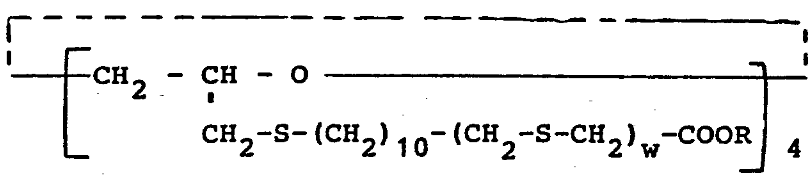

(VI)

in which *w* denotes the number 0 or 1 and R denotes $CH_3$ or $C_2H_5$ with a primary-tertiary diamine such as dimethylaminoethylamine, dimethylaminopropylamine, diethylaminoethylamine, diethylaminopropylamine, the amine groups thus obtained optionally being salified or alkyled and/or converted to ammonium, ammonioacetate, ammoniopropionate or ammoniopropanesulphonate groups.

8. Process according to claim 1, characterized in that one prepares a surface-active cyclic polyether of the formula (I) in which A denotes the group:

$$-(CH_2-S-CH_2)_w-\underset{\substack{\| \\ O}}{C}-(O-CH_2-CH_2)_n-B \qquad (f)$$

in which *w* denotes the number 0 or 1, *n* denotes any number from 1 to 10 and B has the meaning indicated in Claim 7, by condensing the intermediate of the formula (VI) shown in Claim 1, with an oxyethyleneated dimethylamine of the formula:

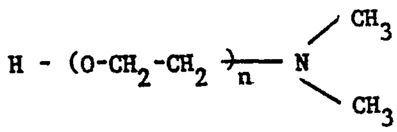

in which $n$ denotes any number from 1 to 10, the condensation being carried out at a temperature from 20 to 160 °C and the amine group optionally being salified or alkylated and/or converted to an ammonium, ammonioacetate, ammoniopropionate or ammoniopropanesulphonate group.

9. Process according to claim 1, characterized in that one prepares a surface-active cyclic polyether of the formula (I) in which A denotes the group :

$$—[CH_2—S—CH_2]_w—COOM_1 \qquad (g)$$

in which $w$ denotes the number 0 or 1 and $M_1$ denotes an ammonium group or an alkali metal and preferably lithium, sodium or potassium, by saponification of the methyl or ethyl ester of the intermediate of the formula (VI) shown in claim 1, followed by neutralisation which an alkali metal hydroxide or ammonia, or, if $w$ denotes zero, directly by reaction of the tetramer of epichlorohydrin or of epibromohydrin with 11-mercapto undecan-1-oic acid, in the presence of sodium methylate or ethylate - potassium methylate or ethylate, followed by neutralisation with an alkali metal hydroxide or ammon:

10. Process according to claim 1, characterized in that one prepares a surface-active cyclic polyethe of the formula (I) in which A denotes the group :

$$—CH_2—O—SO_3M \qquad (h)$$

in which M denotes a hydrogen atom, an ammonium group or an alkali metal, by sulphation of the intermediate of the formula (V) shown in claim 5, with chlorosulphonic acid, and neutralisation, if appropriate, with an alkali metal hydroxide or ammonia.

11. Process according to claim 1, characterized in that one prepares a surface-active cyclic polyether of the formula (I) in which A denotes the group :

$$—CH_2—S—[Y—O]_n—H \qquad (i)$$

in which $n$ denotes any number from 1 to 10 and Y denotes the ethylene or hydroxypropylene radical, by reacting mercaptoethanol or mercaptoglycerol with the mesylate or tosylate of the intermediate of the formula (V) which is shown in claim 1, or with a halogen compound of the intermediate of the formula (V), it being possible for the resulting compound to be condensed with 1 to 10 mols of ethylene oxide and/or of glycidol.

12. Process according to claim 1, characterized in that one prepares a surface-active cyclic polyether of the formula (I) in which A denotes the group :

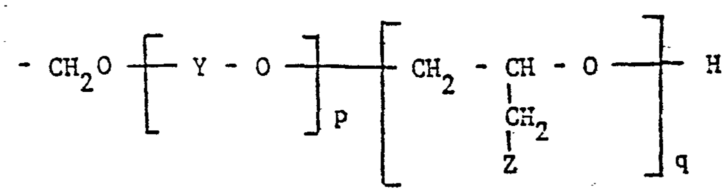
(j)

in which p and q, which are identical or different, denote any number from 0 to 10, p and q not simultaneously denoting zero, Y denotes the ethylene or hydroxypropylene radical and Z denotes one of the groups : —OH, $—S—CH_2—CH_2OH$, $—S—CH_2\text{-}CHOH\text{-}CH_2OH$,

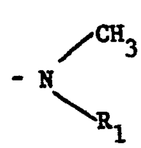 ; 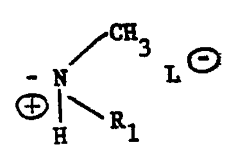 ; 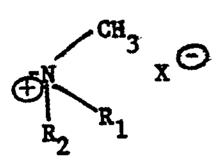 ; 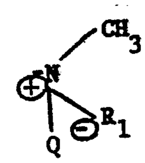

$—S—CH_2—COOM$ and $—SO_3M$,

and, if p = 0 or if Y denotes the ethylene group, Z also denotes one of the groups : $—OSO_3M$ or $—OCO—CH_2—SO_3M$, in which groups $R_1$ denotes $CH_3$ or $—[CH_2—CH_2—O]_n—H$, $n$ denotes any number from 1 to 10, $L^{\ominus}$ denotes an organic or mineral acid anion, $X^{\ominus}$ denotes a chloride, bromide, iodide, methylsulphate, mesylate or tosylate anion and M denotes a hydrogen atom, an ammonium group or an

alkali metal, by means of a polyaddition reaction, with the intermediate of the formula (V) shown in Claim 1, of reactants containing an epoxide group and chosen from amongst ethylene oxide, tert.-butyl glycidylether (TBGE), epichlorhydrin, epibromhydrin and mixtures thereof, the tert.-butoxy protective groups being replaced by hydroxyl groups, and the halogen atoms originating from an epihalohydrin being replaced by a hydroxyl group (by reaction with sodium acetate or potassium acetate, followed by saponification or alcoholysis of the acetic acid ester formed) or by a thio-hydroxyethyl, thiodihydroxypropyl or thioglycolate group by reaction with thioethanol, thioglycerol or methyl or ethyl thioglycolate, or by an amine group by reaction with dimethylamine or methylethanolamine ; if methylethanolamine is used, the compound obtained can be reacted, if appropriate, with ethylene oxide, it being possible, for the amine groups thus obtained to be salified and converted to ammonium, ammonioacetate, ammoniopropionate or ammoniopropane-sulphonate groups, and it being possible, if appropriate, for the hydroxyl groups obtained to be esterified and replaced by sulphate or sulphoacetate groups, respectively by reaction with chlorosulphonic acid or sulphoacetic acid.

13. Cosmetic composition for the care of the hair, in the form of a shampoo, a dyeing shampoo, a hair conditioning composition, a dyeing composition, in the form of an aqueous or aqueous-alcoholic solution, of a cream, of a jelly, of an emulsion or of an aerosol, which optionally may contain adjuvants and more particularly anionic, cationic, amphoteric, zwitterionic and non-ionic surface-active agents and their mixtures, perfumes, dyestuffs, preservatives, foam stabilisers, softeners, hair-structuring agents, anti-dandruff agents, cosmetic resins, sequestring agents, thickeners, foam synergists agents, electrolytes, characterized by the fact that it contains at least $0.5 \cdot 10^{-2}$ gram/liter of at least one compound of formula (I)

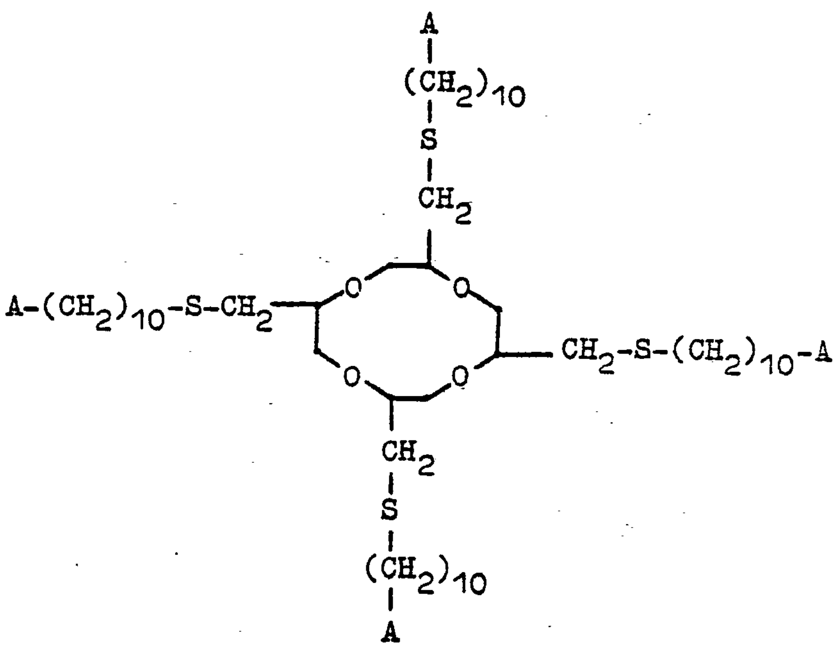

(I)

in which A is chosen from amongst the following :

a) the group

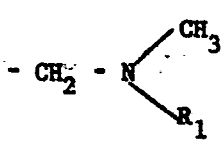

in which $R_1$ denotes $CH_3$ or $—[CH_2—CH_2—O]_n—H$, in which $n$ denotes any number from 1 to 10,

b) the group

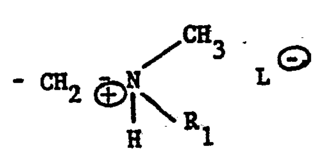

in which $L^{\ominus}$ denotes an organic or mineral acid anion and $R_1$ has the meaning indicated for (a),
c) the group

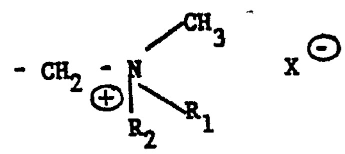

in which $R_2$ denotes an alkyl or hydroxyalkyl radical having from 1 to 3 carbon atoms, $X^{\ominus}$ denotes an anion and preferably a chloride, bromide, iodide, methyl-sulphate, mesylate or tosylate anion and $R_1$ has the meaning indicated under (a),
d) the group

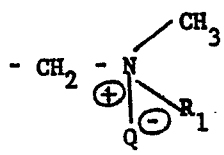

in which $Q^{\ominus}$ denotes one of the groups

$-CH_2COO^{\ominus}$, $CH_2-CH_2-COO^{\ominus}$ or $-CH_2-CH_2-CH_2-SO_3^{\ominus}$, and

$R_2$ has the meaning indicated for (a),
e) the group

$$-(CH_2-S-CH_2)_w-CONH-(CH_2)_m-B$$

in which *m* denotes 2 or 3, *w* denotes the number 0 or 1 and B denotes one of the groups

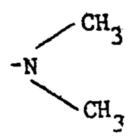 , 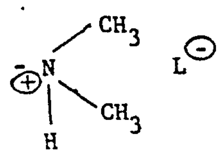 , 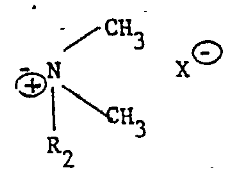 , 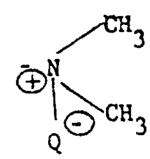

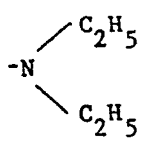 , 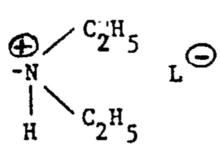 , 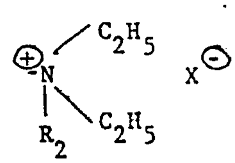 and 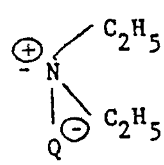

in which $L^{\ominus}$ has the meaning indicated for (b), $R_2$ and $X^{\ominus}$ have the meaning indicated for (c) and $Q^{\ominus}$ has the meaning indicated for (d),
f) the group

$$-(CH_2-S-CH_2)_w-\underset{\substack{\|\\ O}}{C}-(O-CH_2-CH_2)_n-B$$

in which *w* denotes the number 0 or 1, *n* denotes any number from 1 to 10 and B has the meaning indicated for (e),
g) the group

$$-[CH_2-S-CH_2]_w-COOM$$

in which *w* denotes the number 0 or 1 and $M_1$ denotes an ammonium group or an alkali metal,
h) the group

$$-CH_2-O-SO_3M$$

in which M denotes a hydrogen atom, an ammonium group, or an alkali metal ;
i) the group

$$—CH_2—S—[Y—O]_n—H$$

in which *n* denotes any number from 1 to 10,
Y denotes the ethylene or hydroxypropylene radical,
j) the group

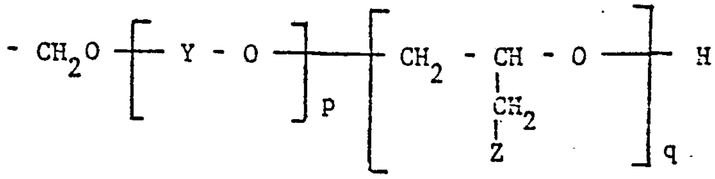

in which p and q, which are identical or different, denote any number from 0 to 10, but p and q do not simultaneously denote zero, Y has the meaning indicated for (i), Z denotes one of the following groups :

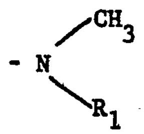 ; 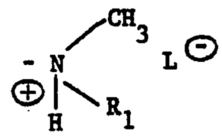 ; 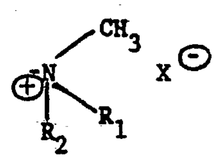 ; 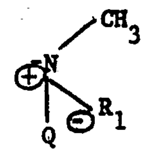

$—S—CH_2—COOM$ and $—SO_3M$,

and, if p denotes zero or if Y denotes ethylene, Z also denotes $—O—SO_3M$ or $—OCO—CH_2—SO_3M$ ; in these groups : $R_1$ denotes $CH_3$ or $[CH_2—CH_2—O]_n—H$, in which *n* denotes any number from 1 to 10, $L^{\ominus}$ denotes an organic or mineral acid anion, $R_2$ denotes a methyl, hydroxyethyl or dihydroxypropyl radical, $X^{\ominus}$ denotes an anion, $Q^{\ominus}$ denotes $—CH_2—COO^-$, $CH_2—CH_2—CH_2—COO^-$ or $CH_2—CH_2—CH_2—SO_3^-$, and M denotes a hydrogen atom, an ammonium group or an alkali metal.

14. Process for treating the hair, consisting in applying, to human hair, an effective amount of a composition according to claim 13, leaving said composition in contact with the hair during sufficient time, then rinsing, optionally shampooing and rinsing again.

15. Process for cleaning wet hair, consisting in applying to a head of hair previously wetted, a sufficient quantity of a composition according to claim 13, massaging the scalp in order to emulsionate and rinsing.

**Ansprüche** (für die Vertragsstaaten : BE, CH, DE, FR, GB, IT, LU, NL, SE)

1. Cyclische, grenzflächenaktive Polyäther der allgemeinen Formel I :

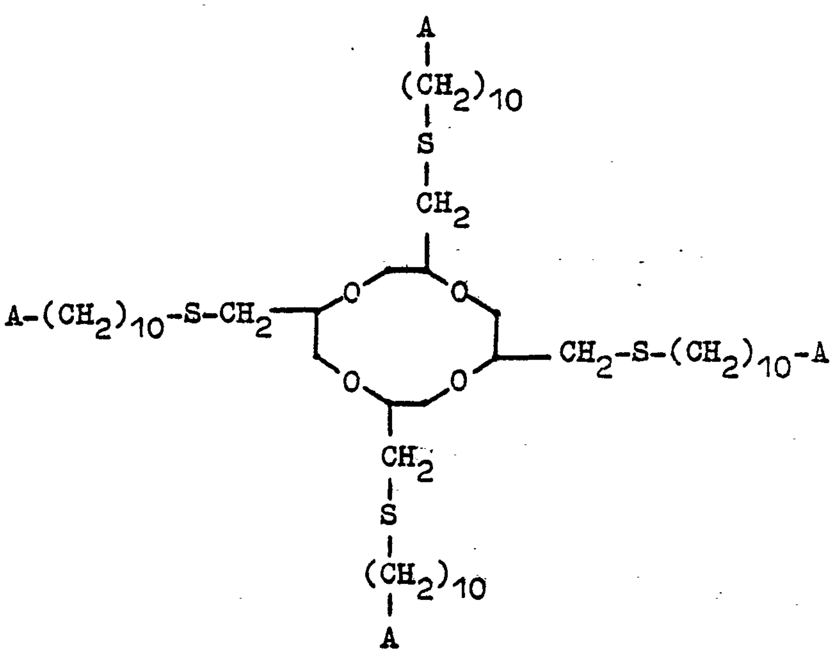

(I)

worin A darstellt ein hydrophiles, kationisches, anionisches, zwitterionisches oder nicht-ionisches Ganzes, welches mindestens eine Gruppe aufweist, ausgewählt unter Amin-, Ammonium-, Ammoniumalkylcarboxylat-, Ammoniumalkylsulfonatgruppen,

die Gruppe

$$-[CH_2-S-CH_2]_w-COOM_1 \qquad (g)$$

worin w für Null oder 1 steht, und
$M_1$ ein Alkalimetall bedeutet;
die Gruppe

$$-CH_2-O-SO_3M \qquad (h)$$

worin M für Wasserstoff- oder ein Alkalimetallatom steht;
die Gruppe

$$-CH_2-S-[Y-O-H]_n-H \qquad (i)$$

worin n eine Zahl zwischen 1 und 10 bedeutet; und Y für einen Äthylen- oder Hydroxypropylenrest steht;

die Gruppe

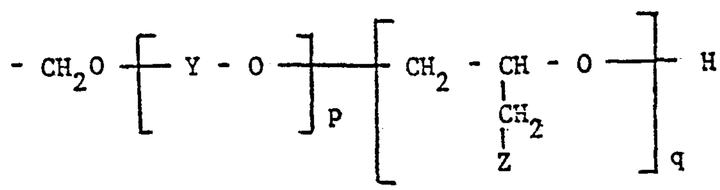

(j)

worin p und q, die gleich oder verschieden sein können, eine Zahl von 0 bis 10 bedeuten, wobei jedoch nicht beide gleichzeitig Null bedeuten können,
Y die unter (i) angegebene Bedeutung besitzt,
Z für eine der nachstehenden Gruppen steht:

$-OH$ ; $-S-CH_2-CH_2OH$ ; $-S-CH_2-CHOH-CH_2OH$, $-S-CH_2-COO\,M$ ; $-SO_3M$ ;

wobei außerdem, wenn p für Null steht oder wenn Y für Äthylen steht, Z auch $-O-SO_3-M$ oder $-OCO-CH_2-SO_3M$ bedeuten kann, und
M ein Wasserstoff- oder Alkalimetallatom bedeutet.

2. Cyclische, grenzflächenaktive Polyäther nach Anspruch 1, dadurch gekennzeichnet, daß die hydrophile Gruppe A, welche Amin-, Ammonium-, Ammoniumalkylcarboxylat- und/oder Ammoniumalkylsulfonatgruppen enthält, ausgewählt ist unter:

der Gruppe

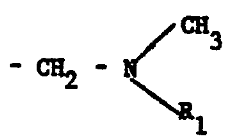

(a)

worin $R_1$ für $CH_3$ oder $-[CH_2-CH_2-O]_n-H$ steht,
worin n eine Zahl von 1 bis 10 bedeutet;
der Gruppe

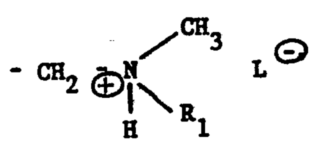

(b)

worin $L^{\ominus}$ für ein organisches oder anorganisches Säureanion steht, und
$R_1$ die für (a) angegebenen Bedeutungen besitzt;
der Gruppe

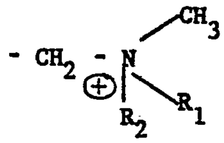 $X^{\ominus}$ (c)

worin $R_2$ für einen Alkyl- oder Hydroxyalkylrest mit 1 bis 3 Kohlenstoffatomen steht ;
$X^{\ominus}$ ein Anion bedeutet, vorzugsweise ein Chlorid-, Bromid-, Jodid-, Methylsulfat-, Mesylat- oder Tosylat-Anion, und
$R^1$ die unter (a) angegebenen Bedeutungen besitzt ;
der Gruppe

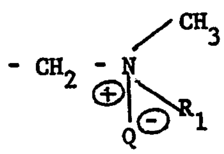 (d)

worin $Q^{\ominus}$ für eine der folgenden Gruppen steht :

$-CH_2-COO^{\ominus}$, $-CH_2-CH_2-COO^{\ominus}$ oder $-CH_2-CH_2-CH_2-SO_3^{\ominus}$ ; und

$R_1$ die unter (a) angegebenen Bedeutungen besitzt ;
der Gruppe

$$-(CH_2-S-CH_2)_w-CONH-(CH_2)_m-B \qquad (e)$$

worin m für 2 oder 3 steht,
w Null oder 1 bedeutet,
B für einerder nachfolgenden Gruppen steht :

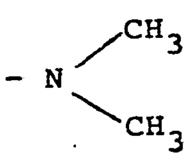 ; 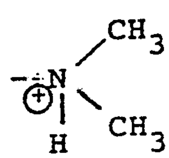 $L^{\ominus}$ ; 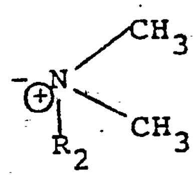 $X^{\ominus}$ ;

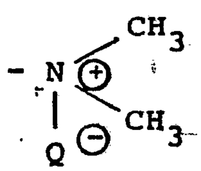 ; 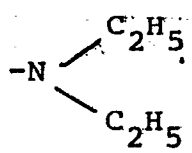 ; 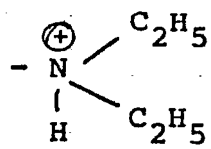 $L^{\ominus}$ ;

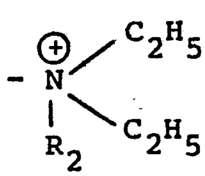 $X^{\ominus}$ ; 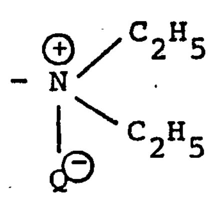

worin $L^{\ominus}$ die für (b) angegebene Bedeutung besitzt,
$R_2$ und $X^{\ominus}$ die für (c) angegebenen Bedeutungen haben, und
$Q^{\ominus}$ die für (d) angegebenen Bedeutungen hat ;
der Gruppe

$$-(CH_2-S-CH_2)_w-\underset{\underset{O}{\|}}{C}-(O-CH_2-CH_2)_n-B \qquad (f)$$

worin w für Null oder 1 steht,
n eine Zahl von 1 bis 10 ist und
B die für (e) angegebenen Bedeutungen besitzt ;
der Gruppe

$$—[CH_2—S—CH_2]_w—COOM_1 \quad (g')$$

worin w für Null oder 1 steht, und
$M_1$ eine Ammoniumgruppe bedeutet ;
der Gruppe

$$—CH_2—O—SO_3M \quad (h')$$

worin M eine Ammoniumgruppe bedeutet ;
der Gruppe

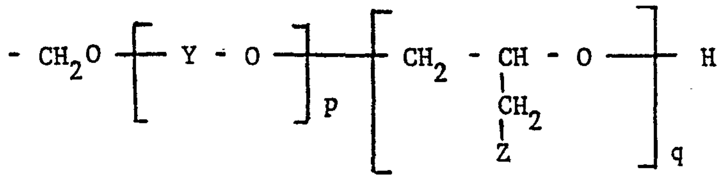

(j')

worin p und q, die gleich oder verschieden sein können, eine Zahl von 0 bis 10 bedeuten, jedoch p und q nicht gleichzeitig Null bedeuten,

Y für einen Äthylen- oder Hydroxypropylenrest steht ; und Z eine der nachfolgenden Gruppen bedeutet :

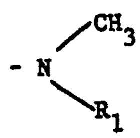 ; 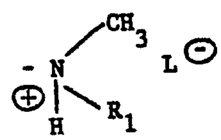 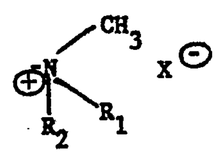 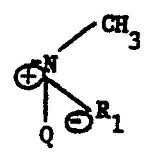

$—S—CH_2—COO\ M$ ; $—SO_3\ M$ ;

wobei, wenn p für Null steht oder wenn Y Äthylen bedeutet, Z ebenfalls $—O—SO_3—M$ oder $—O-CO—CH_2—SO_3\ M$ bedeuten kann,
wobei in diesen Gruppen
$R_1$ für $CH_3$ oder $[CH_2—CH_2—O]_n—H$ steht wobei
n eine Zahl von 1 bis 10 darstellt,
$L^{\ominus}$ für ein Anion einer organischen Säure oder einer Mineralsäure steht,
$R_2$ einen Methyl-, Hydroxyäthyl- oder Dihydroxy-propylrest bedeutet,
$X^{\ominus}$ für ein Anion steht,
$Q^{\ominus}$ für
$—CH_2—COO^{\ominus}$, $—CH_2—CH_2—CH_2—COO^{\ominus}$ oder $—CH_2—CH_2—CH_2—SO_3^{\ominus}$ steht, und
M eine Ammoniumgruppe bedeutet.

3. Zwischenprodukte zur Herstellung der Verbindungen der allgemeinen Formel I nach Anspruch 1, entsprechend der allgemeinen Formel :

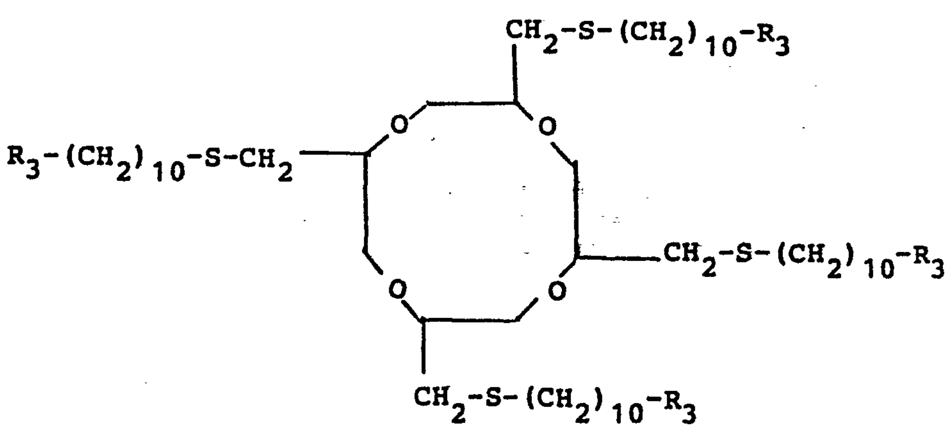

(VII)

worin $R_3$ für $-CH_2OH$ ; $-CH_2-O-SO_2-CH_3$ ; $-CH_2-O-SO_2-C_6H_4-CH_3$ ; $-COOH$ ; $-COOCH_3$ ; $-COOC_2H_5$ ; $-CH_2-S-CH_2-COOH$ ; $-CH_2-S-CH_2-COOCH_3$ oder $-CH_2-S-CH_2-COOC_2H_5$ steht.

4. Verfahren zur Herstellung der Zwischenprodukte der allgemeinen Formel (VII) nach Anspruch 3, dadurch gekennzeichnet, daß man

1) Thioessigsäure zu Undecenalkohol oder zu Undecensäure gibt ;

2) das in Stufe (1) hergestellte Reaktionsprodukt verseift, um entsprechend das 11-Mercaptoundecanol bzw. die 11-Mercapto-1-undecansäure zu erhalten ;

3) die 11-Mercapto-1-undecansäure gegebenenfalls in einen Methyl- oder Äthylester überführt ;

4) die aus Stufe (2) oder (3) erhaltene Verbindung mit dem Tetrameren von Epichlorhydrin oder Epibromhydrin kondensiert, oder das Methyl- oder Äthyl-thioglycolat mit der Halogenverbindung, dem Mesylat oder Tosylat der Verbindung, die durch Reaktion von 11-Mercapto-1-undecanol mit dem Tetrameren von Epichlorhydrin oder Epibromhydrin erhalten wurde, kondensiert.

5. Verfahren zur Herstellung eines cyclischen, polypoden, grenzflächenaktiven Polyäthers der allgemeinen Formel I, worin A für die Gruppe

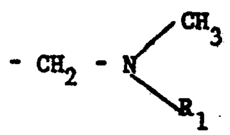

(a)

steht, worin

$R_1$ $CH_3$ oder $-[CH_2-CH_2-O]_n-H$ bedeutet und

n für eine Zahl von 1 bis 10 steht,

durch Umsetzung von Dimethylamin oder Methyläthanolamin mit dem Chlor- oder Bromderivat der Zwischenprodukte der Formel V :

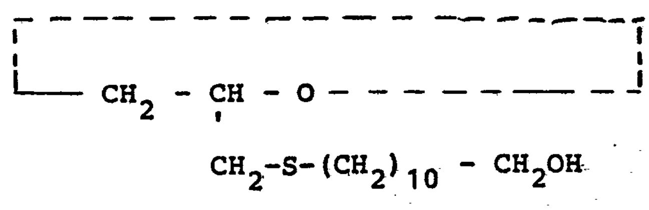

(V)

erhalten durch Umsetzung von Thionylchlorid oder Bromwasserstoffsäure mit dieser Zwischenverbindung der Formel (V), oder auch mit dem Mesylat oder Tosylat der Zwischenverbindung der Formel (V), bei einer Temperatur von 20 bis 160 °C, bei Normaldruck oder im Autoklaven ; ist das eingesetzte Amin Methyläthanolamin, so wird die erhaltene Verbindung gegebenenfalls mit 1 bis 10 Mol Äthylenoxyd oxyäthyleniert.

6. Verfahren zur Herstellung eines cyclischen, polypoden, grenzflächenaktiven Polyäthers der Formel (I), worin A für den Rest :

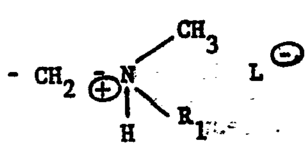

(b)

steht, worin

$R_1$ für $CH_3$ oder $-[CH_2-CH_2-O]_n-H$ steht,

n eine Zahl von 1 bis 10 bedeutet, und

$L^\ominus$ ein organisches oder anorganisches Anion und vorzugsweise ein Chlorid, Bromid, Sulfat, Phosphat, Acetat, Glycolat, Lactat, Tartrat, Methansulfonat oder p-Toluolsulfonat, ist ;

durch Überführen in ein Salz mit einer Säure der Formel HL, worin L die oben angegebenen Bedeutungen besitzt.

7. Verfahren zur Herstellung eines cyclischen, polypoden; grenzflächenaktiven Polyäthers der Formel (I), worin A für die Gruppe :

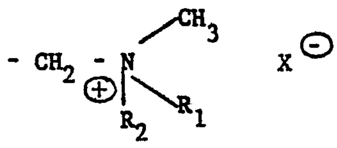

(c)

steht, worin

$R_2$ einen Methyl-, Hydroxyäthyl- oder Dihydroxy- propylrest bedeutet,

$X^{\ominus}$ für ein Anion, vorzugsweise für ein Chlorid-, Bromid-, Jodid-, Methylsulfat, Mesylat oder Tosylat, steht ; und

$R_1$ für $CH_3$ oder $-[CH_2-CH_2-O]_n-H$ steht, worin n eine Zahl von 1 bis 10 bedeutet, durch Alkylierung einer Verbindung I(a) nach Anspruch 2 mit einem Alkylierungsmittel, vorzugsweise ausgewählt unter Methylchlorid, -bromid, -jodid, -sulfat, -mesylat oder -tosylat, Glycolchlorhydrin oder Glycerinchlorhydrin.

8. Verfahren nach Anspruch 7, dadurch gekennzeichnet, daß $X^{\ominus}$ ein Mesylat- oder Tosylat-Anion ist und man die Mesylate oder Tosylate der Zwischenverbindung der Formel (V) :

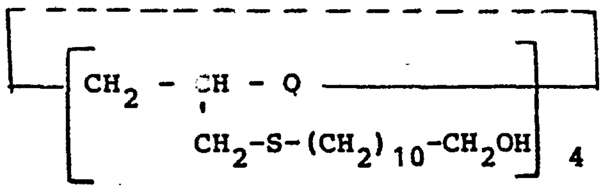

(V)

mit einem Amin der Formel :

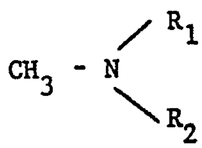

worin $R_1$ für $CH_3$ oder $-[CH_2-CH_2-O]_n-H$ steht,

n eine Zahl von 1 bis 10 bedeutet, und

$R_2$ für einem Methyl-, Hydroxyäthyl- oder Dihydroxypropyl-rest steht, bei einer Temperatur zwischen 20 und 100 °C umsetzt.

9. Verfahren zur Herstellung eines cyclischen, polypoden, grenzflächenaktiven Polyäthers der Formel (I), worin A für die Gruppe :

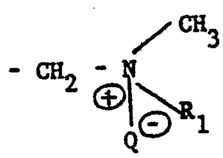

(d)

steht, worin

$Q^{\ominus}$ eine der Gruppen $-CH_2COO^{\ominus}$, $-CH_2-CH_2-COO^{\ominus}$, $-CH_2-CH_2-CH_2-SO_3^{\ominus}$ bedeutet,

$R_1$ für $CH_3$ oder $-[CH_2-CH_2-O]_n-H$ steht,

worin n eine Zahl von 1 bis 10 bedeutet,

durch Alkylierung der Verbindungen der Formel (I), worin A die Gruppe (a) bedeutet, mit Natriumchloracetat oder -chlorpropionat oder Propansulton.

10. Verfahren zur Herstellung eines cyclischen, polypoden, grenzflächenaktiven Polyäthers der Formel (I), worin A für die Gruppe

$$-(CH_2-S-CH_2)_w-CONH-(CH_2)_m-B \quad (e)$$

steht, worin

m für 2 oder 3 steht ;

w die Zahl Null oder 1 bedeutet,

B für eine der Gruppen steht :

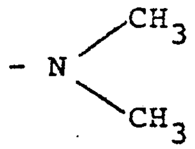 ; 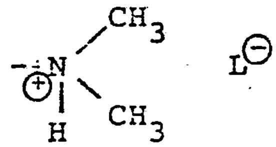 ; 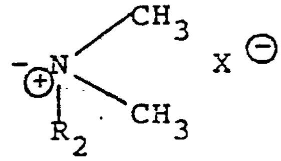 ;

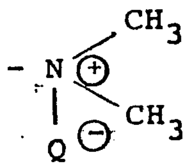 ; 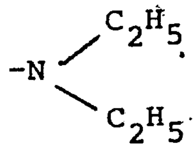 ; 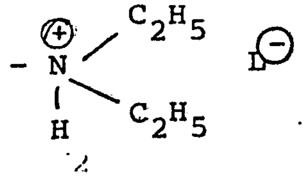

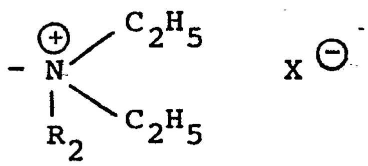 ; 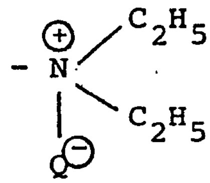

worin $R_2$ und $X^{\ominus}$ die in Anspruch 7 angegebenen Bedeutungen besitzen, $Q^{\ominus}$ die in Anspruch 9 angegebenen Bedeutungen hat und $L^{\ominus}$ die in Anspruch 6 angegebenen Bedeutungen hat ; durch Kondensation der Zwischenverbindung der Formel (VI) :

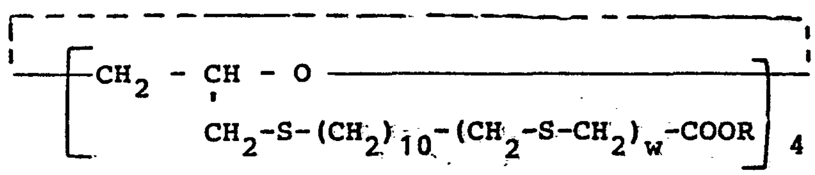 (VI)

worin w für Null oder 1 steht ; und R $CH_3$ oder $C_2H_5$ bedeutet, mit Dimethylaminoäthylamin, Dimethylaminopropylamin, Diäthylaminoäthylamin, Diäthylaminopropylamin, die so erhaltenen Aminfunktionen gegebenenfalls in ein Salz überführt, oder alkyliert und/oder in Ammonium-, Ammoniumacetat-, Ammoniumpropionat- oder Ammoniumpropansulfonatgruppen überführt.

11. Verfahren zur Herstellung cyclischer, polypoder, grenzflächenaktiver Polyäther der Formel (I), worin A für die Gruppe :

$$-(CH_2-S-CH_2)_w-\underset{\underset{O}{\|}}{C}-(O-CH_2-CH_2)_n-B \qquad (f)$$

steht, worin w für Null oder 1 steht, n eine Zahl von 1 bis 10 bedeutet, B die in Anspruch 10 angegebenen Bedeutungen besitzt, durch Kondensation des Zwischenprodukts der Formel (VI) nach Anspruch 10 mit einem oxyäthylierten Dimethylamin der Formel :

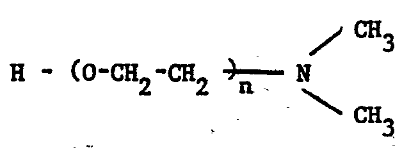

worin n für eine Zahl von 1 bis 10 steht ; bei einer Temperatur von 20 bis 160 °C ;

wobei die Amingruppe gegebenenfalls in ein Salz überführt, oder alkyliert und/oder in eine Ammonium-, Ammoniumacetat-, Ammoniumpropionat- oder Ammonium- propansulfonatgruppe überführt wird.

12. Verfahren zur Herstellung von cyclischen, polypoden, grenzflächenaktiven Polyäthern der allgemeinen Formel (I), worin A für die Gruppe :

$$-[CH_2-S-CH_2]_w-COO\ M_1 \qquad (g)$$

steht, worin

w die Zahl Null oder 1 bedeutet,

$M_1$ eine Ammoniumgruppe oder ein Alkalimetall, vorzugsweise Lithium, Natrium, oder Kalium bedeutet,

indem man den Methyl- oder Äthylester der Zwischenverbindung der Formel (VI) entsprechend Anspruch 10 verseift und anschließend mit einem Alkalimetallhydroxid oder Ammoniak neutralisiert, oder wenn

w für Null steht, das Tetramere von Epichlorhydrin oder Epibromhydrin direkt mit der 11-Mercapto-1-undecansäure in Gegenwart von Natrium- oder Kalium-methylat oder -äthylat umsetzt und anschließend mit einem Alkalihydroxyd oder Ammoniak neutralisiert.

13. Verfahren zur Herstellung von cyclischen, polypoden, grenzflächenaktiven Polyäthern der Formel (I) worin A für die Gruppe :

$$-CH_2-O-SO_3\ M \qquad (h)$$

steht, worin

M für ein Wasserstoffatom, eine Ammoniumgruppe oder ein Alkalimetall steht,

durch Sulfatieren der Zwischenverbindung der Formel (V) aus Anspruch 5, mit Chlorsulfonsäure und gegebenenfalls Neutralisation mit einem Alkalihydroxid oder Ammoniak.

14. Verfahren zur Herstellung cyclischer, polypoder, grenzflächenaktiver Polyäther der Formel (I), worin A für die Gruppe :

$$-CH_2-S-[Y-O]_n-H \qquad (i)$$

steht, worin

n eine Zahl von 1 bis 10 bedeutet,

Y für den Äthylen- oder Hydroxypropylenrest steht,

durch Umsetzung von Mercapto-äthanol oder Mercaptoglycerin mit dem Mesylat oder Tosylat der Zwischenverbindung der Formel (V) oder mit einer Halogenverbindung der Zwischenverbindung der Formel (V) aus Anspruch 5, wobei die erhaltene Verbindung mit 1 bis 10 Mol Äthylenoxyd und/oder Glycidol kondensiert werden kann.

15. Verfahren zur Herstellung cyclischer, polypoder, grenzflächenaktiver Polyäther der Formel I, worin A für die Gruppe :

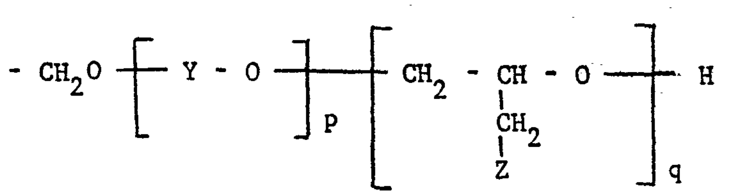

(j)

steht, worin

p und q, die gleich oder verschieden sein können, eine Zahl von Null bis 10 bedeuten, wobei p und q nicht gleichzeitig Null bedeuten können,

Y für einen Äthylen- oder Hydroxypropylenrest steht,

Z für eine der Gruppen :

$-OH$ ; $-S-CH_2-CH_2OH$ ; $-S-CH_2-CHOH-CH_2OH$ ;

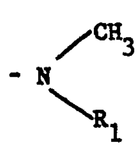 ; 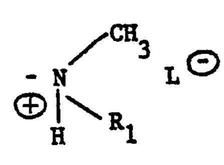 ; 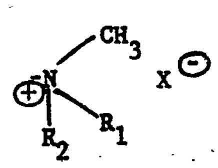 ; 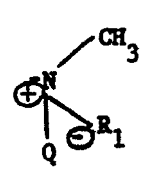

$—S—CH_2—COO\ M$, $—SO_3\ M$ ;

steht, wobei, wenn $p = 0$ oder Y für die Äthylengruppe steht,
Z außerden für einen der folgenden Reste stehen kann :
$—OSO_3\ M$ oder $—OCO—CH_2—SO_3M$
worin $R_1$ für $CH_3$ oder $—[CH_2—CH_2—O]_n—H$ steht,
n eine Zahl von 1 bis 10 darstellt,
$L^{\ominus}$ ein Anion einer organischen oder organischen Säure bedeutet,
$X^{\ominus}$ für ein Chlorid-, Bromid-, Jodid-, Methylsulfat-, Mesylat- oder Tosylat-Anion steht,
M ein Wasserstoffatom, eine Ammoniumgruppe oder ein Alkalimetall bedeutet,
durch Polyaddition der Zwischenverbindung der Formel (V) aus Anspruch 5 mit Epoxydverbindungen ausgewählt unter Äthylenoxid, Tert.-Butylglycidyläther (TBGE), Epichlorhydrin, Epibromhydrin und deren Mischungen, wobei die tert.-Butoxyschutzgruppen durch Hydroxylgruppen ersetzt werden, und die von einem Epihalogenhydrin stammenden Halogenatome durch eine Hydroxygruppe (durch Umsetzung mit Natrium- oder Kaliumacetat und anschließende Verseifung oder Alkoholyse des gebildeten Essigsäureesters), oder durch eine Thiohydroxyäthyl-, Thiohydroxypropyl- oder Thioglycolat-gruppe, durch Umsetzung mit Thioäthanol, Thioglycerin oder Methyl- oder Äthyl-thioglycolat, oder durch eine Aminogruppe, durch Umsetzung mit Dimethylamin oder Methyläthanolamin, ersetzt werden ; wobei, wenn man Methyläthanolamin verwendet, die erhaltene Verbindung gegebenenfalls mit Äthylenoxyd umgesetzt werden kann, und die so erhaltenen Aminfunktionen anschließend in ein Salz verwandelt und in Ammonium-, Ammoniumacetat-, Ammoniumpropionat- oder Ammonium-propan-sulfonat-Gruppen überführt werden können ; die erhaltenen Hydroxygruppen können gegebenenfalls verestert und durch Sulfat- oder Sulfoacetat-gruppen ersetzt werden, jeweils durch Umsetzung mit Chlorsulfonsäure oder Sulfoessigsäure.

16. Mittel, enthaltend mindestens $0{,}5.10^{-2}$ g/Liter einer cyclischen, grenzflächenaktiven Verbindung der Formel (I).

17. Kosmetiches oder pharmazeutisches Mittel, dadurch gekennzeichnet, daß es mindestens $0{,}5.10^{-2}$ g/Liter einer Verbindung der Formel (I) in Gegenwart eines wäßrigen oder wäßrig-alkoholischen Trägers enthält.

18. Mittel nach Anspruch 17, dadurch gekennzeichnet, daß die wäßrig-alkoholische Lösung einen Alkohol oder ein Polyol mit 1 bis 4 Kohlenstoffatomen enthält.

19. Mittel nach den Ansprüchen 16 und 17, dadurch gekennzeichnet, daß es in Form einer Lösung, einer Creme, eines Gels, einer Emulsion oder eines Aerosols vorliegt.

20. Kosmetisches Mittel zur Pflege der Haare, das in einer Lösungsmittellösung, ausgewählt unter Wasser und einer wäßrig-alkoholischen Lösung, eine wirksame Menge eines kosmetischen Mittels aus einer oder mehreren Verbindungen der Formel (I) enthält.

21. Shampoo zur Pflege der Haare, welches in Lösung in einem Lösungsmittel, ausgewählt unter Wasser und einer wäßrig-alkoholischen Lösung, eine kosmetisch wirksame Menge einer oder mehrerer Verbindungen der Formel (I) enthält.

22. Haarshampoo, gelöst in einem Lösungsmittel, ausgewählt unter Wasser und einem wäßrig-alkoholischen Lösungsmittel, eine kosmetisch wirksame Menge einer oder mehrerer Verbindungen der Formel (I) zusammen mit einem oder mehreren Adjuvantien, ausgewählt unter anionischen, kationischen, amphoteren, zwitterionischen, nicht-ionischen grenzflächenaktiven Mitteln, Parfums, Farbstoffen, Konservierungsmitteln, Verdickungsmitteln, schaumstabilisierenden Mittelns, weichmachenden Mitteln, die Haare restrukturierenden Mitteln, Anti-Schuppen-mitteln, kosmetischen Harzen, synergistischen Schäummitteln und Elektrolyten, enthält.

23. Verfahren zur Behandlung von Haaren, bei dem man auf menschliches Haar eine ausreichende Menge eines Mittels aufträgt, das gelöst in einem Lösungsmittel, ausgewählt unter Wasser und einer wäßrig-alkoholischen Lösung, eine kosmetisch wirksame Menge eines oder mehrerer cyclischer, grenzflächenaktiver Polyäther der Formel (I) enthält.

24. Färbemittel für Haare, welches gelöst in einem Lösungsmittel, ausgewählt unter Wasser und einer wäßrigalkoholischen Lösung, eine kosmetisch wirksame Menge einer oder mehrerer Verbindungen der Formel (I) sowie einen Haarfarbstoff und gegebenenfalls ein oder mehrere Adjuvantien, ausgewählt unter anionischen, kationischen, nicht-ionischen, zwitterionischen, amphoteren grenzflächenaktiven Mitteln und deren Mischungen, Parfums, Konservierungsmitteln, Verdickungsmitteln, weichmachenden Mitteln, kosmetischen Harzen und Sequestriermitteln, enthält.

25. Mittel nach Anspruch 24, dadurch gekennzeichnet, daß die Farbstoffe ausgewählt sind unter Anthrachinonfarbstoffen, Azofarbstoffen, Nitrofarbstoffen der Benzolreihe, Indophenolen, Indoanilinen und Indaminen.

**Ansprüche** (für den Vertragsstaat AT)

1. Verfahren zur Herstellung von oberflächenaktiven cyclischen Polyäthern der allgemeinen Formel

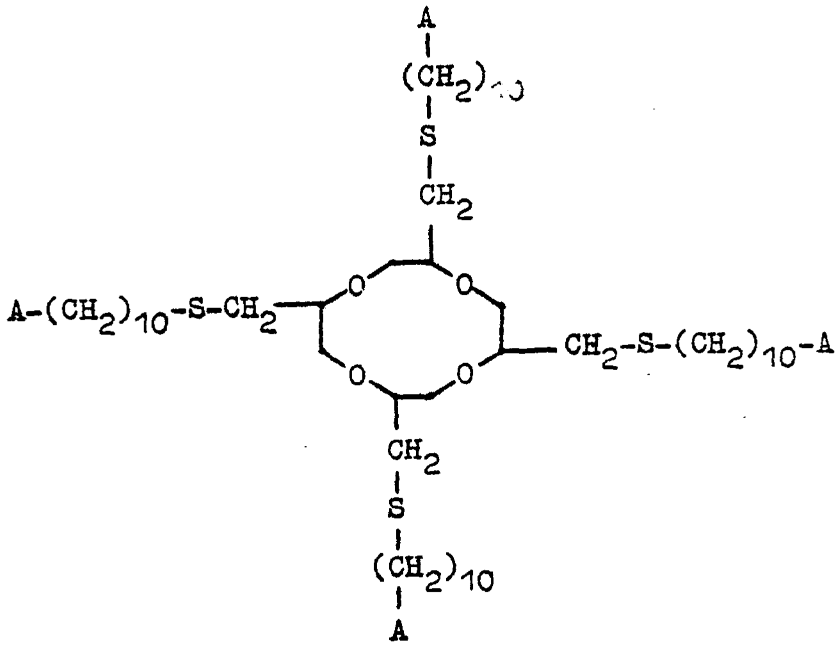

,(I)

worin A eine anionische, kationische, zwitterionische oder nicht-ionische Gruppierung ausgewählt unter den folgenden darstellt:

a) eine Gruppierung

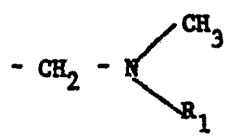

worin $R_1CH_3$ oder $-[CH_2-CH_2-O]_n-H$ und n eine beliebige Zahl von 1 bis 10 bedeutet,

b) eine Gruppierung

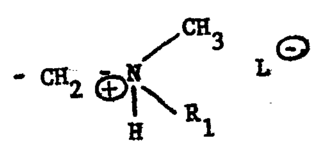

worin $L^{\ominus}$ das Anion einer organischen Säure oder einer Mineralsäure darstellt und $R_1$ die bei a) angegebene Bedeutung hat,

c) eine Gruppierung

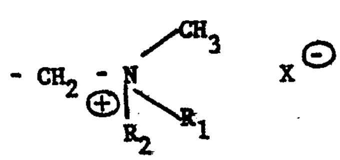

worin $R_2$ ein Alkyl- oder Hydroxyalkylrest mit 1 bis 3 C-Atomen und $X^{\ominus}$ ein Anion, vorzugsweise Chlorid, Bromid, Jodid, Methylsulfat, Mesylat oder Tosylat bedeutet und $R_1$ die bei a) angegebene Bedeutung hat,

d) eine Gruppierung

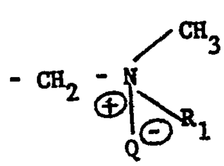

worin $Q^{\ominus}$ $-CH_2-COO^{\ominus}$, $-CH_2-CH_2-COO^{\ominus}$ oder $CH_2-CH_2-CH_2-SO_3^{\ominus}$ bedeutet und $R_1$ die bei a) angegebene Bedeutung hat,

e) eine Gruppierung $-(CH_2-S-CH_2)_w-CONH-(CH_2)_m-B$, worin m 2 oder 3 und w O oder 1 ist, B eine der folgenden Gruppen

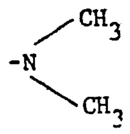 , 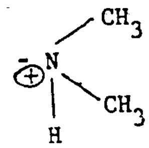 $L^{\ominus}$ , 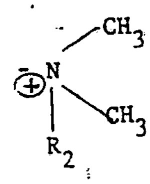 , 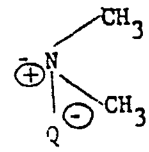

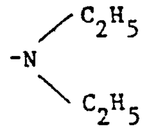 , 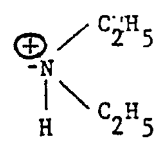 $L^{\ominus}$ , 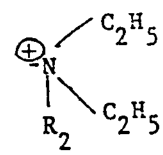 $X^{\ominus}$ und 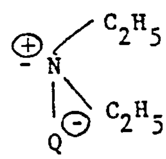

in welchen $L^{\ominus}$ die bei b), $R_2$ und $X^{\ominus}$ die bei c) und $Q^{\ominus}$ die bei d) angegebene Bedeutung haben,

f) eine Gruppierung

$$-(CH_2-S-CH_2)_w-\underset{\underset{O}{\|}}{C}-(O-CH_2-CH_2)_n-B$$

in welcher w 0 oder 1 und n eine beliebige Zahl von 1 bis 10 ist und B die bei e) angegebene Bedeutung hat,

g) eine Gruppierung $-[CH_2-S-CH_2]_w-COOM_1$,

in welcher w 0 oder 1 ist und $M_1$ eine Ammoniumgruppe oder ein Alkalimetall bedeutet,

h) eine Gruppierung $-CH_2-O-SO_3M$, worin M Wasserstoff, eine Ammoniumgruppe oder ein Alkalimetall bedeutet,

i) eine Gruppierung $-CH_2-S-[Y-O]_n-H$, in welcher n eine beliebige Zahl von 1 bis 10 ist und Y eine Äthylen- oder Hydroxypropylenrest bedeutet,

j) eine Gruppierung

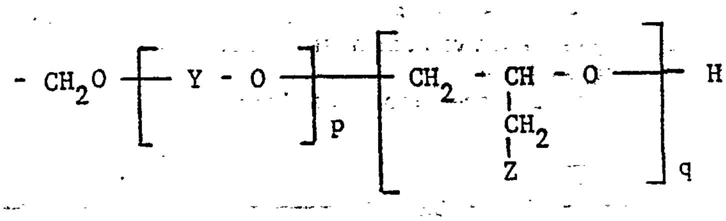

worin p und q, die gleich oder verschieden sind, eine beliebige Zahl von 0 bis 10 bedeuten, wobei p und q nicht gleichzeitig 0 sein können, Y die bei i) angegebene Bedeutung hat und Z eine der folgenden Gruppen darstellt:

$-OH$, $-S-CH_2-CH_2OH$, $-S-CH_2-CHOH-CH_2OH$,

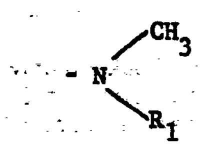 ; 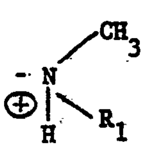 $L^{\ominus}$ ; 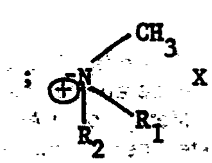 $X^{\ominus}$ ; 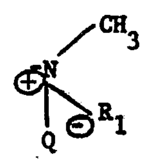

$-S-CH_2-COOM$, $-SO_3M$,

und außerdem für den Fall, daß p = 0 ist oder Y Äthylen bedeutet, Z auch $-O-SO_3-M$ oder $-OCO-CH_2-SO_3M$ bedeuten kann.

In den oben angegebenen Gruppen bedeutet $R_1CH_3$ oder $-[CH_2-CH_2-O]_n-H$, worin n eine beliebige Zahl von 1 bis 10 ist; $L^{\ominus}$ ein Anion einer organischen Säure oder einer Mineralsäure, $R_2$ eine Methyl-, Hydroxyäthyl- oder Dohydroxypropylrest, $X^{\ominus}$ ein Anion, $Q^{\ominus}$ $-CH_2-COO^{\ominus}$, $-CH_2-CH_2-COO^{\ominus}$ oder $CH_2-CH_2-CH_2-SO_3^{\ominus}$, und M Wasserstoff, eine Ammoniumgruppe oder ein Alkalimetall, dadurch gekennzeichnet, daß in einer ersten Stufe 11-Mercapto-1-undecanol der Formel

$$HS—(CH_2)_{10}—CH_2OH \quad (II)$$

oder 11-Mercapto-1-undecansäure der Formel

$$HS—(CH_2)_{10}—COOH \quad (III)$$

hergestellt wird, und in einer zweiten Stufe ein Zwischenprodukt der Formel

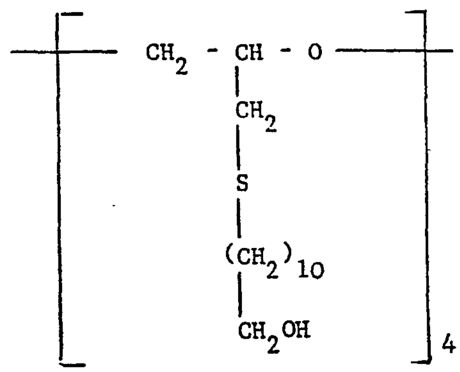

(V)

durch Reaktion von 11-Mercapto-1-undecanol mit dem Tetrameren von Epichlorhydrin oder Epibromhydrin der Formel

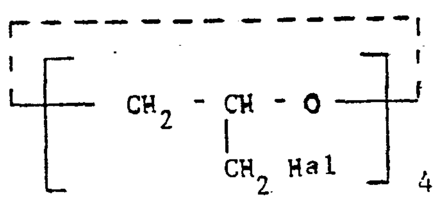

(IVa)

hergestellt wird, in welcher Hal Chlor oder Brom bedeutet (wobei das Tetramere von Epichlorhydrin oder Epibromhydrin durch Polymerisation von Epichlorhydrin oder Epibromhydrin in Gegenwart einer Lewis-Säure als Katalysator hergestellt wird),
und in einer dritten Stufe ein Zwischenprodukt der Formel

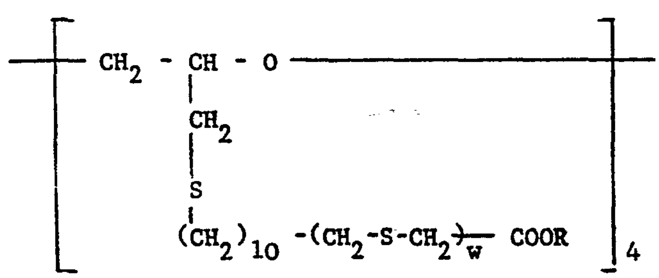

(VI)

in welcher R Wasserstoff, $CH_3$ oder $C_2H_5$ und w 0 oder 1 bedeuten durch Reaktion entweder mit 11-Mercapto-1-undecansäure oder deren Methyl- oder Äthylester mit dem Tetrameren von Epichlorhydrin oder Epibromhydrin oder von Methyl- oder Äthylthioglykolat mit einem Halogenderivat, dem Mesylat oder Tosylat des Zwischenproduktes der Formel (V) hergestellt wird, worauf anschließend folgende Verbindungen hergestellt werden :

a) Verbindingen Ia, entsprechend der Formel (I), in welcher A die Gruppierung (a) bedeutet, durch Reaktion von Dimethylamin oder Methyläthanolamin mit dem Chlor- oder Bromderivat oder dem Mesylat oder Tosylat des Zwischenproduktes der Formel (V) ;

b) Verbindungen Ib, entsprechend der Formel (I), in welcher A die Gruppierung (b) bedeutet, durch Salzbildung der entsprechenden Verbindungen Ia mit einer organischen Säure oder einer Mineralsäure ;

c) Verbindungen Ic, entsprechend der Formel (I), in welcher A die Gruppierung (c) bedeutet, durch Alkylierung der entsprechenden Verbindungen Ia oder durch direkte Reaktion des Mesylates oder Tosylates des Zwischenproduktes der Formel (V) mit einem Amin der Formel

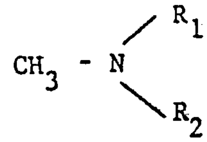

d) Verbindungen Id, entsprechend der Formel (I), in welcher A die Gruppierung (d) bedeutet, durch Alkylierung der Verbindungen Ia mit Na-Chloracetat oder -Chlorpropionat oder Propansulton ;

e) Verbindungen Ie, entsprechend der Formel (I), in welcher A die Gruppierung (e) bedeutet, durch Kondensation des Zwischenproduktes der Formel (VI) mit Dimethylaminoäthylamin, Dimethylaminopropylamin, Diäthylaminoäthylamin oder Diäthylaminopropylamin ;

f) Verbindungen If, entsprechend der Formel (I), in welcher A die Gruppierung (f) bedeutet, durch Kondensation des Zwischenproduktes der Formel (VI) mit einem oxäthylenierten Dimethylamin der Formel

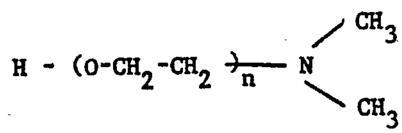

worin n eine beliebige Zahl von 1 bis 10 bedeutet, wobei die Aminogruppen anschließend mit einer Mineralsäure oder einer organischen Säure in ein Salz übergeführt oder mit einem Alkylierungsmittel alkyliert werden können ;

g) Verbindungen Ig, entsprechend der Formel (I), in welcher A die Gruppierung (g) bedeutet, durch Verseifung des Methyl- oder Äthylesters des Zwischenproduktes der Formel (VI) und anschließende Neutralisation mit einem Alkalihydroxyd oder Ammoniak oder auch für der Fall, daß w 0 bedeutet, durch direkte Reaktion des Tetrameren von Epichlorhydrin oder Epibromhydrin mit 11-Mercapto-1-undecansäure in Gegenwart von Na- oder K-Methylat oder -Äthylat und anschließende Neutralisation mit einem Alkalihydroxyd oder Ammoniak ;

h) Verbindungen Ih, entsprechend der allgemeinen Formel (I), in welcher A die Gruppierung (h) bedeutet, durch Sulfatation des Zwischenproduktes der Formel (V) mit Chlorsulfonsäure und gegebenenfalls Neutralisation mit einem Alkalihydroxyd oder Ammoniak ;

i) Verbindungen Ii, entsprechend der Formel (I), in welcher A die Gruppierung (i) bedeutet, durch Reaktion von Mercaptoäthanol oder Mercaptoglycerin mit dem Mesylat oder Tosylat des Zwischenproduktes der Formel (V) oder einem halogenierten Derivat dieser Verbindung und gegebenenfalls anschließender Polyaddition von Äthylenoxyd und/oder Glycid in Gegenwart eines alkalischen Katalysators ;

j) Verbindungen Ij, entsprechend der Formel (I), in welcher A die Gruppierung (j) bedeutet, durch Polyadditionsreaktionen des Zwischenproduktes der Formel (V) mit eine Epoxyfunktion aufweisenden Reaktanten ausgewählt aus : Äthylenoxyd, Tertiobutylglycidyläther (TBGE), Epichlorhydrin, Epibromhydrin und deren Mischungen, wobei die Tertiobutoxy-Schutzgruppen durch Hydroxylgruppen und die Halogenatome der Epihalogenhydrine durch Hydroxylgruppen (durch Reaktion mit Na- oder K-acétat und anschließende Verseifung oder Alkoholyse des gebildeten Essigsäureesters) durch eine Thiohydroxyäthyl-, Thiodihydroxypropyl- oder Thioglykolatgruppe durch Reaktion mit Thioäthanol, Thioglycerin oder Methyl-, oder Äthyl-Thioglykolat) oder durch eine Aminogruppe (durch Reaktion mit Dimethylamin oder Methyläthanolamin) ersetzt sein können, wobei für den Fall der Verwendung von Methyläthanolamin die erhaltene Verbindung gegebenenfalls einer Reaktion mit Äthylenoxyd unterworfen werden kann und die erhaltenen Aminfunktionen in ein Salz übergeführt und in Ammoniumfunktionen, Ammoniumacetat, Ammoniumpropionat oder Ammoniumpropansulfonat umgewandelt werden können und die erhaltenen Hydroxylgruppen gegebenenfalls verestert bzw. durch Reaktion mit Chlorsulfonsäure oder Sulfoessigsäure durch Sulfat- oder Sulfoacetatgruppen ersetzt werden können.

2. Verfahren nach Anspruch 1, dadurch gekennzeichnet, daß ein oberflächenaktiver cyclischer Polyäther der Formel (I), in welcher A eine Gruppierung

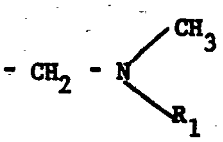 (a)

darstellt, worin $R_1CH_3$ oder $-[CH_2-CH_2-O]_n-H$ und n eine beliebige Zahl von 1 bis 10 ist, durch Reaktion von Dimethylamin oder Methyläthanolamin mit einem Chlor- oder Bromderivat des Zwischenproduktes der in Anspruch 1 angegebenen Formel (V) oder auch mit einem Mesylat oder Tosylat des

Zwischenproduktes der Formel (V) bei einer Temperatur von 20 bis 160 °C, unter Normaldruck oder im Autoklaven hergestellt wird, wobei für den Fa daß das verwendete Amin Methyläthanolamin ist, die erhaltene Verbindung gegebenenfalls mit 1 bis 0 Mol Äthylenoxyd oxäthyleniert wird.

3. Verfahren nach Anspruch 1, dadurch gekennzeichnet, daß ein oberflächenaktiver cyclischer Polyäther der Formel (I), in welcher A eine Gruppierung

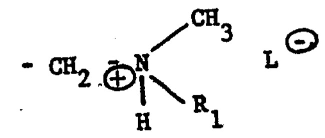 (b)

darstellt, worin $R_1$ die in Anspruch 2 angegebene Bedeutung hat und $L^{\ominus}$ das Anion einer organischen Säure oder einer Mineralsäure, vorzugsweise Chlorid, Bromid, Sulfat, Phosphat, Acetat, Glykolat, Lactat, Tartrat, Methansulfonat, p-Toluolsulfonat darstellt, durch Salzbildung mittels einer Säure der Formel HL, worin L die oben angegebene Bedeutung hat, hergestellt wird.

4. Verfahren nach Anspruch 1, dadurch gekennzeichnet, daß ein oberflächenaktiver cyclischer Polyäther der Formel (I), in welcher A eine Gruppierung

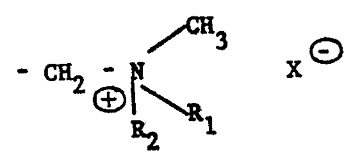 (c)

darstellt, worin $R_2$ einen Methyl-, Hydroxyäthyl- oder Dihydroxypropylrest und $X^{\ominus}$ ein Anion, vorzugsweise Chlorid, Bromid, Jodid, Methylsulfat, Mesylat oder Tosylat darstellt und $R_1$ die in Anspruch 2 angegebene Bedeutung hat durch Alkylierung einer in Anspruch 2 angegebenen Verbindung Ia mit einem Alkylierungsmittel, vorzugsweise ausgewählt aus Methylchlorid, -bromid, -jodid, -sulfat, -mesylat oder -tosylat, Glykol-Chlorhydrin oder Glycerin-Chlorhydrin hergestellt wird.

5. Verfahren nach Anspruch 4, dadurch gekennzeichnet, daß $X^{\ominus}$ ein Mesylat- oder Tosylat-Anion darstellt und daß das Mesylat oder Tosylat des Zwischenproduktes der Formel

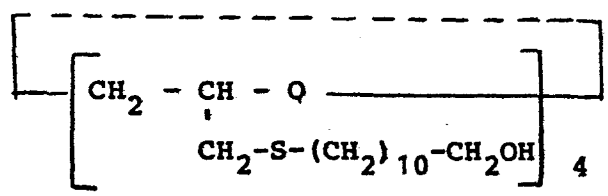 (V)

mit einem Amin der Formel

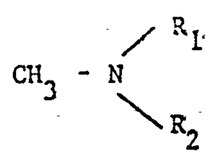

in welcher $R_1CH_3$ oder $—[CH_2—CH_2—O]_n—H$, n eine beliebige Zahl von 1 bis 10 und $R_1$ einen Methyl-, Hydroxyäthyl- oder Dihydroxypropylrest bedeutet, bei einer Temperatur zwischen 20 und 100 °C zur Reaktion gebracht wird.

6. Verfahren nach Anspruch 1, dadurch gekennzeichnet, daß ein oberflächenaktiver cyclischer Polyäther der Formel (I), in welcher A eine Gruppierung

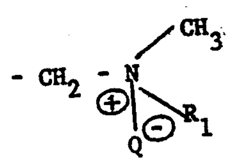 (d)

darstellt, in welcher $Q^{\ominus}$ eine der Gruppen $—CH_2COO^{\ominus}$, $—CH_2—CH_2—COO^{\ominus}$, $—CH_2—CH_2—CH_2—SO_3^{\ominus}$ bedeutet und $R_1$ die in Anspruch 2 angegebene Bedeutung hat, durch Alkylierung der Verbindungen der Formel (I), in welchen A eine Gruppierung (a) bedeutet, mit Na-Chloracetat oder -Chlorpropionat oder Propansulton hergestellt wird.

7. Verfahren nach Anspruch 1, dadurch gekennzeichnet, daß ein oberflächenaktiver cyclischer Polyäther der Formel (I), in welcher A

$$-(CH_2-S-CH_2)_w-CONH-(CH_2)_m-B \qquad (e)$$

darstellt, worin m 2 oder 3 und w 0 oder 1 ist und B eine der Gruppen

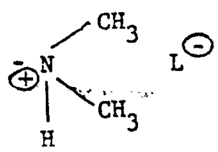 , 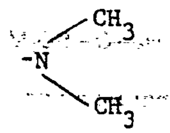 , 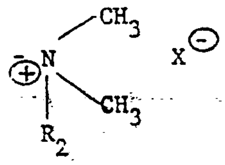 , 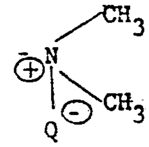

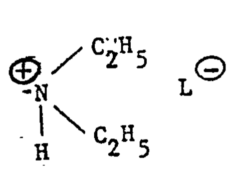 , 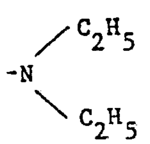 , 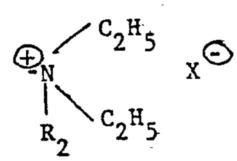 ; 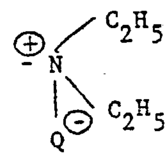

bedeutet, worin $R_2$ und $X^{\ominus}$ die in Anspruch 4 angegebene Bedeutung, $Q^{\ominus}$ die in Anspruch 6 angegebene Bedeutung und $L^{\ominus}$ die in Anspruch 3 angegebene Bedeutung haben durch Kondensation des Zwischenproduktes der Formel

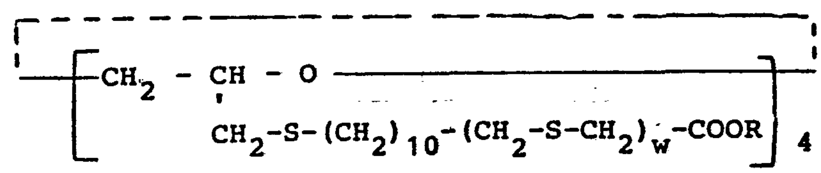 (VI)

in welcher w 0 oder 1 und R $CH_3$ oder $C_2H_5$ darstellt, mit einem primär-tertiären Diamin wie Dimethylaminoäthylamin, Dimethylaminopropylamin, Diäthylaminoäthylamin, Diäthylaminopropylamin, wobei die erhaltenen Aminfunktionen gegebenenfalls in Salze übergeführt, alkyliert und/oder in Ammoniumgruppen, Ammoniumacetat, Ammoniumpropionat oder Ammoniumpropansulfonat übergeführt werden können, hergestellt wird.

8. Verfahren nach Anspruch 1, dadurch gekennzeichnet, daß ein oberflächenaktiver cyclischer Polyäther der Formel (I), in welcher A eine Gruppierung

$$-(CH_2-S-CH_2)_w-\underset{\overset{\|}{O}}{C}-(O-CH_2-CH_2)_n-B \qquad (f)$$

darstellt, worin w 0 oder 1 und n eine beliebige Zahl von 1 bis 10 ist und B die in Anspruch 7 angegebene Bedeutung hat durch Kondensation des Zwischenproduktes der in Anspruch 1 angegebenen Formel (VI) mit einem oxäthylenierten Diamin der Formel

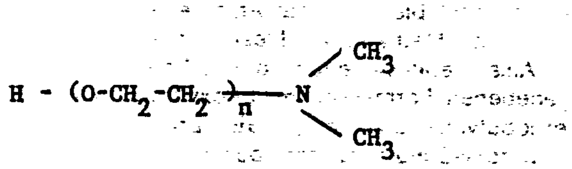

in welcher n eine beliebige Zahl von 1 bis 10 ist, hergestellt wird, wobei die Kondensation bei einer Temperatur zwischen 20 und 160 °C durchgeführt wird und die so erhaltenen Aminfunktionen gegebenenfalls in Salze übergeführt, alkyliert und/oder in Ammoniumgruppen, Ammoniumacetat, Ammoniumpropionat oder Ammoniumpropansulfonat umgewandelt werden können.

9. Verfahren nach Anspruch 1, dadurch gekennzeichnet, daß ein oberflächenaktiver cyclischer Polyäther der Formel (I), in welcher A eine Gruppierung

$$-[CH_2-S-CH_2]_w-COOM_1 \qquad (g)$$

darstellt, worin w 0 oder 1 und $M_1$ eine Ammoniumgruppe oder ein Alkalimetall, vorzugsweise Lithium,

Natrium oder Kalium bedeutet, durch Verseifung eines Methyl- oder Äthylesters des Zwischenproduktes der in Anspruch 1 angegebenen Formel (VI) und anschliessender Neutralisation mit einem Alkalihydroxyd oder Ammoniak, oder für den Fall daß $w = 0$ ist, durch direkte Reaktion des Tetrameren von Epichlorhydrin oder Epibromhydrin mit 11-Mercapto-1-undecansäure in Gegenwart von Na- oder K-Methylat oder Äthylat und anschließender Neutralisation mit einem Alkalihydroxyd oder Ammoniak hergestellt wird.

10. Verfahren nach Anspruch 1, dadurch gekennzeichnet, daß ein oberflächenaktiver cyclischer Polyäther der Formel (I), in welcher A eine Gruppierung

$$—CH_2—O—SO_3M \qquad (h)$$

darstellt, worin M Wasserstoff, eine Ammoniumgruppe oder ein Alkalimetall bedeutet, durch Sulfatation des Zwischenproduktes der in Anspruch 1 angegebenen Formel (V) mit Chlorsulfonsäure und gegebenenfalls Neutralisation mit einem Alkalihydroxyd oder Ammoniak hergestellt wird.

11. Verfahren nach Anspruch 1, dadurch gekennzeichnet, daß ein oberflächenaktiver cyclischer Polyäther der Formel (I), in welcher A eine Gruppierung

$$—CH_2—S—[Y—O]_n—H \qquad (i)$$

darstellt, worin n eine beliebige Zahl von 1 bis 10 und Y einen Äthylen- oder Hydroxypropylenrest bedeutet, durch Reaktion von Mercaptoäthanol oder Mercaptoglycerin mit dem Mesylat oder Tosylat des Zwischenproduktes der in Anspruch 1 angegebenen Formel (V) oder eines Halogenderivates des Zwischenproduktes der Formel (V) hergestellt wird, wobei die erhaltene Verbindung mit 1 bis 10 Mol Äthylenoxyd und/oder Glycidoxyd kondensiert werden kann.

12. Verfahren nach Anspruch 1, dadurch gekennzeichnet, daß ein oberflächenaktiver cyclischer Polyäther der Formel (I), in welcher A eine Gruppierung

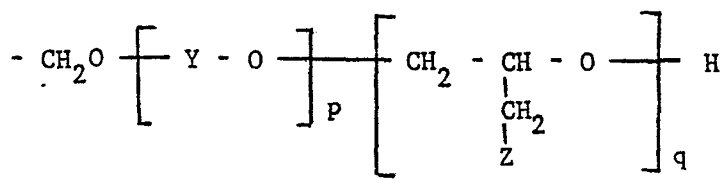 (j)

darstellt, worin p und q, die gleich oder verschieden sind, eine beliebige Zahl von 0 bis 10 darstellen, wobei p und q nicht gleichzeitig 0 sein können, Y einen Äthylen- oder Hydroxypropylenrest und Z eine der Gruppen $—OH$, $—S—CH_2—CH_2OH$, $—S—CH_2—CHOH—CH_2OH$,

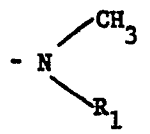 ; 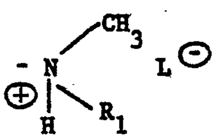 ; 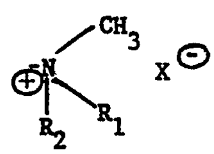 ; 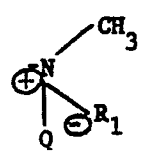

$—S—CH_2—COOM$, $—SO_3M$ bedeutet, wobei für den Fall, daß $p = 0$ ist oder wenn Y den Äthylenrest darstellt, Z auch $—OSO_3M$ oder $—OCOCH_2—SO_3M$ sein kann, und $R_1$ $CH_3$ oder $—[CH_2—CH_2—O]_n—H$, n eine beliebige Zahl von 1 bis 10, $L^{\ominus}$ das Anion einer organischen Säure oder einer Mineralsäure, $X^{\ominus}$ ein Chlorid-, Bromid-, Jodid-, Methylsulfat-, Mesylat- oder Tosylat-Anion und M Wasserstoff, eine Ammoniumgruppe oder ein Alkalimetall bedeuten, durch Polyadditionsreaktionen des Zwischenproduktes der in Anspruch 1 angegebenen Formel (V) mit Epoxyfunktionen aufweisenden Reagentien ausgewählt aus: Äthylenoxyd, Tertiobutylglycidyläther (TBGE), Epichlorhydrin, Epibromhydrin oder deren Mischungen, wobei die Tertiobutoxy-Schutzgruppen durch Hydroxylgruppen und die in dem Epihalogenhydrin vorandenen Halogenatome durch eine Hydroxylgruppe (durch Reaktion mit Na- oder K-Acetat und an- ießende Verseifung oder Alkoholyse des gebildeten Essigsäureesters), durch eine Thiohydroxyäth- hiodihydroxypropyl- oder Thioglykolatgruppe (durch Reaktion mit Thioäthanol, Thioglycerin oder Me oder Äthyl-Thioglykolat) oder durch eine Aminogruppe (durch Reaktion mit Dimethylamin oder Me hanolamin) ersetzt sein können, wobei für den Fall, daß Methyläthanolamin verwendet wird, die erh en Verbindung gegebenenfalls einer Reaktion mit Äthylenoxyd unterworfen werden kann und die so itenen Aminfunktionen in ein Salz übergeführt und in Ammoniumfunktionen, Ammoniumacetat, Ammoniumpropionat oder Ammoniumpropansulfonat umgewandelt werden können und die erhaltenen Hydroxylgruppen gegebenenfalls verestert bzw. durch Reaktion mit Chlorsulfonsäure oder Sulfoessigsäure durch Sulfat- oder Sulfoacetatgruppen ersetzt werden können, hergestellt wird.

13. Kosmetische Zubereitung zur Pflege der Haare in Form eines Shampoos, eines färbenden

Shampoos, eines Konditionierungsmittels für die Haare oder eines Färbemittels, welche in Form einer wässerigen oder wässerig-alkoholischen Lösung oder einer Creme, eines Gels, einer Emulsion oder eines Aerosols vorliegt und außerdem Adjuvantien, insbesondere anionische, kationische, amphotere, zwitterionische oder nicht-ionische oberflächenaktive Stoffe oder deren Mischungen, Riechstoffe, Farbstoffe, Konservierungsmittel, Schaumstabilisatoren, Weichmacher, Mittel zur Wiederherstellung der Haarstruktur, Antischuppenmittel, kosmetische Harze, Sequestriermittel, Verdickungsmittel, Schaumverstärkungsmittel, Elektrolyte enthalten kann dadurch gekennzeichnet, daß sie mindestens $0{,}5.10^{-2}$ g/l mindestens einer Verbindung der Formel

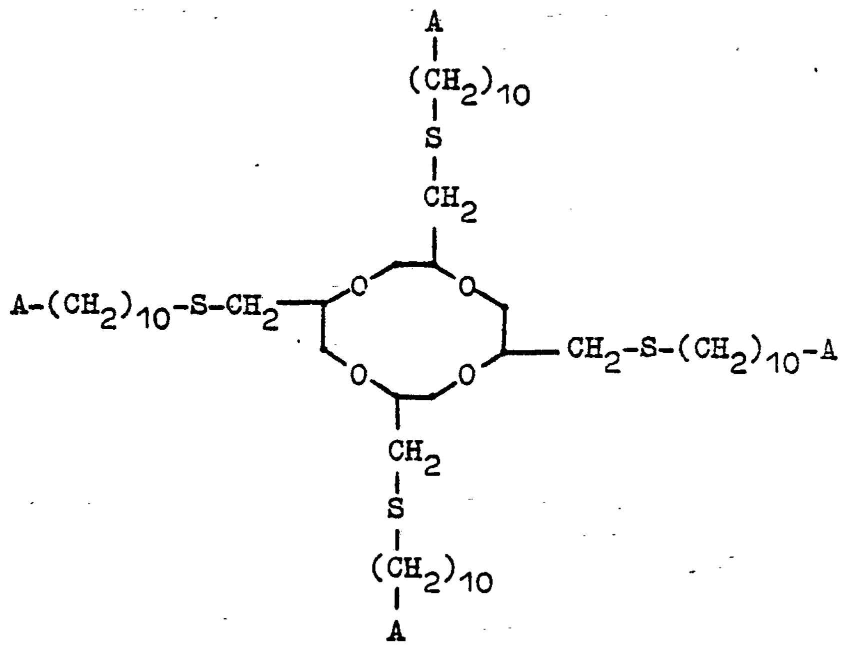
(I)

in welcher A aus den folgenden Gruppierungen ausgewählt ist:

a) eine Gruppierung

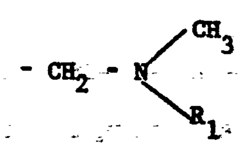

worin $R_1$ $CH_3$ oder $-[CH_2-CH_2-O]_n-H$, wobei n eine beliebige Zahl von 1 bis 10 ist, darstellt,

b) eine Gruppierung

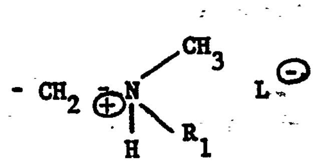

worin $L^\ominus$ ein Anion einer organischen Säure oder einer Mineralsäure ist und $R_1$ die bei a) angegebene Bedeutung hat;

c) eine Gruppierung

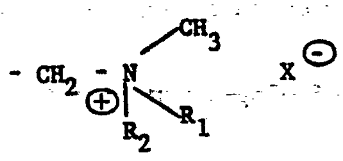

worin $R_2$ eine Alkyl- oder Hydroxyalkylrest mit 1 bis 3 C-Atomen und $X^\ominus$ ein Anion, vorzugsweise Chlorid, Bromid, Jodid, Methylsulfat, Mesylat oder Tosylat bedeutet und $R_1$ die bei a) angegebene Bedeutung hat;

d) eine Gruppierung

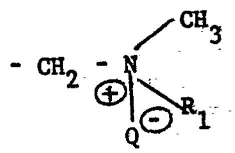

worin $Q^{\ominus}$ eine der Gruppen $-CH_2-COO^{\ominus}$, $-CH_2-CH_2-COO^{\ominus}$ oder $CH_2-CH_2-CH_2-SO_3^{\ominus}$ darstellt und $R_1$ die bei a) angegebene Bedeutung hat;

e) eine Gruppierung $-(CH_2-S-CH_2)_w-CONH-(CH_2)_m-B$, worin m 2 oder 3 und w 0 oder 1 ist und B eine der folgenden Gruppen

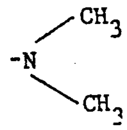 , 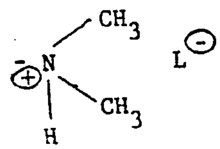 , 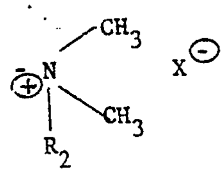 , 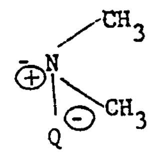

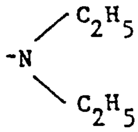 , 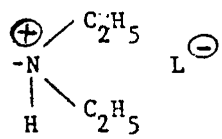 , 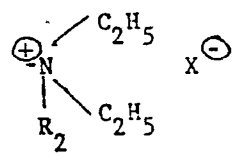 und 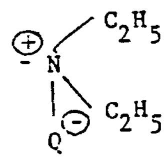

in welchen $L^{\ominus}$ die bei b) angegebene Bedeutung, $R_2$ und $X^{\ominus}$ die bei c) angegebene Bedeutung und $Q^{\ominus}$ die bei d) angegebene Bedeutung haben, darstellt;

f) eine Gruppierung

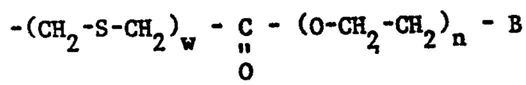

worin w 0 oder 1 und n eine beliebige Zahl von 1 bis 10 ist und B die bei e) angegebene Bedeutung hat;

g) eine Gruppierung

$$-[CH_2-S-CH_2]_w-COOM_1,$$

worin w 0 oder 1 ist und M eine Ammoniumgruppe oder ein Alkalimetall darstellt;

h) eine Gruppierung $-CH_2-O-SO_3M$, worin M Wasserstoff, eine Ammoniumgruppe oder ein Alkalimetall darstellt;

i) eine Gruppierung $-CH_2-S-[Y-O]_n-H$, worin n eine beliebige Zahl von 1 bis 10 ist und Y einen Äthylen- oder Hydroxypropylenrest bedeutet;

j) eine Gruppierung

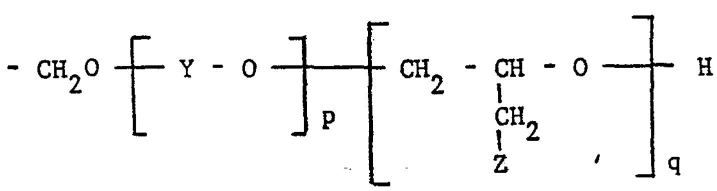

worin p und q, die gleich oder verschieden sind, eine beliebige Zahl von 0 bis 10 bedeuten, wobei p und q nicht zugleich 0 sein können, Y die bei i) angegebene Bedeutung hat und Z eine der folgenden Gruppen bedeutet:

$-OH$, $-S-CH_2-CH_2OH$, $-S-CH_2-CHOH-CH_2OH$,

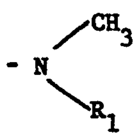 ; 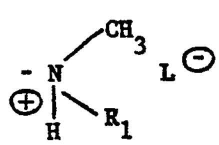 ; 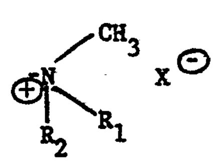 ; 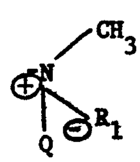

$—S—CH_2—COOM$, $—SO_3M$,

wobei für den Fall, daß $p = 0$ oder Y Äthylen ist, Z außerdem auch $—O—SO_3—M$ oder $—O-CO—CH_2—SO_3M$ bedeutet, in welchen Gruppen $R_1$ $CH_3$ oder $—[CH_2—CH_2—O]_n—H$, worin n eine beliebige Zahl von 1 bis 10 ist, $L^{\ominus}$ ein Anion einer organischen Säure oder einer Mineralsäure, $R_2$ einen Methyl-, Hydroxyäthyl- oder Dihydroxypropylrest, $X^{\ominus}$ ein Anion, $Q^{\ominus}$ $—CH_2—COO^{\ominus}$, $—CH_2—CH_2—COO^{\ominus}$ oder $CH_2—CH_2—CH_2SO_3^{\ominus}$ und M Wasserstoff, eine Ammoniumgruppe oder ein Alkalimetall bedeuten, enthält.

14. Verfahren zur Behandlung der Haare, dadurch gekennzeichnet, daß auf die menschlichen Haare eine wirksame Menge einer Zubereitung nach Anspruch 13 aufgetragen und eine ausreichende Zeit einwirken gelassen wird, worauf die Haare gespült, und gegebenenfalls shampooniert und erneut gespült werden.

15. Verfahren zur Reinigung nasser Haare, dadurch gekennzeichnet, daß auf die vorher befeuchteten Haare eine ausreichende Menge der Zubereitung gemäß Anspruch 13 aufgebracht wird, sodann die Haare zur Emultionierung des Schmutzes massiert und anschließend gespült werden.